# EUROPÄISCHE PATENTSCHRIFT

(11) **EP 0 456 118 B1**
(45) Veröffentlichungstag und Bekanntmachung des Hinweises auf die Patenterteilung: **12.08.1998**
(21) Anmeldenummer: 91107140.5
(22) Anmeldetag: 02.05.1991
(51) Int. Cl.: C07D 409/12, C07D 407/12, C07D 405/12, C07D 409/08, C07D 407/08, C07D 405/08, C07D 333/16, C07D 333/28, C07D 307/42, A01N 43/08, A01N 43/10, A01N 43/40, A01N 43/28, A01N 43/36, A01N 43/16, A01N 43/18

(54) **Cyclohexenonoximether, Verfahren und Zwischenprodukte zu ihrer Herstellung und ihre Verwendung als Herbizide**
Cyclohexenonoximethers, process and intermediates for their preparation and their use as herbicides
Ethers de cyclohexènonoxime, procédé et intermédiaires pour leur préparation et leur utilisation comme herbicides

(30) Priorität: 09.05.1990 DE 4014987
(43) Veröffentlichungstag der Anmeldung: 13.11.1991
(73) Patentinhaber: BASF Aktiengesellschaft, 67063 Ludwigshafen (DE)
(72) Erfinder: Misslitz, Ulf, Dr., W-6730 Neustadt (DE); Meyer, Norbert, Dr., W-6802 Ladenburg (DE); Kast, Juergen, Dr., W-6737 Boehl-Iggelheim (DE); Goetz, Norbert, Dr., W-6520 Worms 1 (DE); Harreus, Albrecht, Dr., W-6700 Ludwigshafen (DE); Kuekenhoehner, Thomas, Dr., W-6710 Frankenthal (DE); Wuerzer, Bruno, Dr., W-6701 Otterstadt (DE); Walter, Helmut, Dr., W-6719 Obrigheim (DE); Westphalen, Karl-Otto, Dr., W-6720 Speyer (DE); Gerber, Matthias, Dr., W-6704 Mutterstadt (DE)

(56) Entgegenhaltungen:
- EP-A- 177 913
- EP-A- 238 021
- EP-A- 309 271
- EP-A- 368 227
- GB-A- 2 125 042
- US-A- 4 432 786

## Beschreibung

Die Erfindung betrifft neue herbizid wirksame Cyclohexenonoximether der Formel I 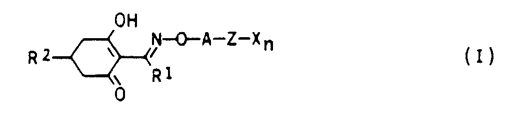 in der die Substituenten folgende Bedeutung haben:
- R¹: eine C₁-C₆-Alkylgruppe;
- A: C₂-C₆-Alkylen oder C₃-C₅-Alkenylen, wobei diese Gruppen ein bis drei C₁-C₃-Alkylgruppen, Halogenatome tragen können;
- Z: ein 5-gliedriger Heteroaromat mit ein bis drei Heteroatomen, ausgewählt aus einer Gruppe bestehend aus drei Stickstoffatomen und einem Sauerstoff- oder Schwefelatom,
ein 6-gliedriger Heteroaromat mit ein bis vier Stickstoffatomen;
- X: eine Aminogruppe -NR^{a}R^{b}, worin
R^{a} Wasserstoff, eine C₁-C₄-Alkylgruppe, eine C₃-C₆-Alkenylgruppe oder eine C₃-C₆-Alkinylgruppe und
R^{b} Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei der folgenden Substituenten tragen kann: Nitro, Cyano, Halogen, C₁-C₄-Alkyl, C₁-C₄-Alkoxy, C₁-C₄-Alkylthio und C₁-C₄-Halogenalkyl bedeuten oder Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkoxy, Carboxyl, C₁-C₄-Alkoxycarbonyl, Benzyloxycarbonyl, Phenyl, wobei die aromatischen Reste noch einen bis drei der folgenden Substituenten tragen können: Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl, Benzyloxycarbonyl,
- n: 0 bis 3 oder 1 bis 4 für den Fall, daß Z einen halogensubstituierten Pyridylrest bedeutet;
- R²: eine C₁-C₄-Alkoxy-C₁-C₆-alkyl- oder C₁-C₄-Alkylthio-C₁-C₆-alkylgruppe;
eine C₃-C₇-Cycloalkylgruppe oder eine C₅-C₇-Cycloalkenylgruppe, wobei diese Gruppen noch einen bis drei Reste tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel und wobei der Heterocyclus noch einen bis drei Reste tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein 6- oder 7-gliedriger gesättigter oder ein- oder zweifach ungesättigter Heterocyclus, enthaltend ein oder zwei Heteroatome, ausgewählt aus einer Gruppe bestehend aus Sauerstoff und Schwefel, wobei dieser Ring noch einen bis drei Reste tragen kann, ausgewählt aus einer Gruppe bestehend aus Hydroxy, Halogen, C₁-C₄-Alkyl, C₁-C₄-Alkoxy, C₁-C₄-Alkylthio und C₁-C₄-Halogenalkyl;
die Phenylgruppe oder die Pyridylgruppe, wobei diese Gruppen noch einen bis drei Reste tragen können, ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy, C₁-C₄-Alkoxy-C₁-C₄-alkyl und -NR^{a}R^{b}, worin R^{a} und R^{b} die oben genannte Bedeutung haben,
bedeuten,
sowie ihre landwirtschaftlich nutzbaren Salze und die Ester von I mit C₁-C₁₀-Carbonsäuren oder anorganischen Säuren.

Außerdem betrifft die Erfindung ein Verfahren und Zwischenprodukte zu ihrer Herstellung sowie ihre Anwendung als Pflanzenschutzmittel.

Die erfindungsgemäßen Cyclohexenone I haben offensichtlich sauren Charakter, d.h. sie können einfache Umsetzungsprodukte wie Salze von Alkali- oder Erdalkaliverbindungen oder Enolester bilden.

Die Verbindungen der Formel I können in mehreren tautomeren Formen auftreten, die alle vom Anspruch erfaßt werden.

In der Literatur sind Cyclohexenone der allgemeinen Formel I' 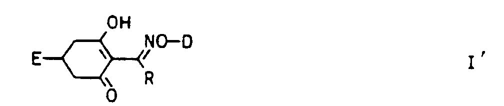 in der u.a.
- D Benzyl und E 2-Ethylthiopropyl (US-A 4 440 566);
- D Benzyl, But-2-enyl und E einen substituierten 5-gliedrigen Heteroarylrest (EP-A 238 021, EP-A 125 094);
- D Benzyl, But-2-enyl und E ein substituiertes Phenyl (EP-A 80 301);
- D But-2-enyl und E einen 5- bis 7-gliedrigen heterocyclischen Ring mit bis zu zwei O-, S-Atomen und mit bis zu zwei Doppelbindungen (EP-A 218 233)
bedeutet, als Herbizide beschrieben.

Es werden jedoch Verbindungen gesucht, die bei geringer Aufwandmenge hohe Selektivität aufweisen, d.h. unerwünschte Pflanzen bekämpfen, ohne dabei die Kulturpflanzen zu schädigen.

Entsprechend dieser Aufgabe wurden die neuen Cyclohexenonoximether der Formel I gefunden, die sich durch eine gute herbizide Wirkung gegen unerwünschte Gräser auszeichnen. Die Verbindungen sind mit breitblättrigen Kulturpflanzen sowie einige mit Gramineenkulturen wie Reis verträglich.

Die Cyclohexenone der Formel I können in an sich bekannter Weise aus schon bekannten Derivaten der Formel II (EP-A 80 301, EP-A-125 094, EP-A 142 741, US-A 4 249 937, EP-A 137 174 und EP-A 177 913) und den entsprechenden Hydroxylaminen der Formel III (Houben-Weyl, 10/1 S. 1181 ff) hergestellt werden (EP-A 169 521).

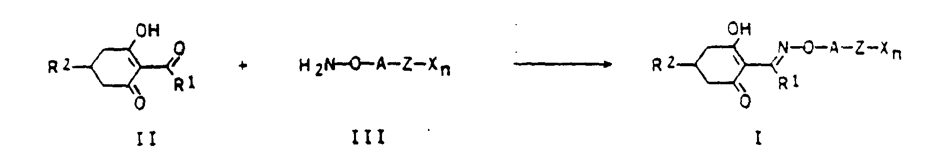

Zweckmäßig führt man die Umsetzung in heterogener Phase in einem Lösungsmittel, bei einer ausreichenden Temperatur unterhalb von etwa 80°C, in Gegenwart einer Base durch und verwendet das Hydroxylamin III in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen I können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel IV 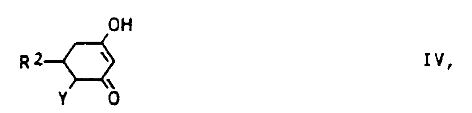 in der
- Y: Wasserstoff oder Methoxycarbonyl bedeutet
nach bekannten Methoden (Tetrahedron Lett., 2491 (1975)) hergestellt werden.

Es ist auch möglich, die Verbindungen der Formel II über die Zwischenstufe der Enolester herzustellen, die bei der Umsetzung von Verbindungen der Formel IV mit Säurechloriden in Gegenwart von Basen anfallen und anschließend mit bestimmten Imidazol- oder Pyridinderivaten umgelagert werden (JP-OS 79/063 052).

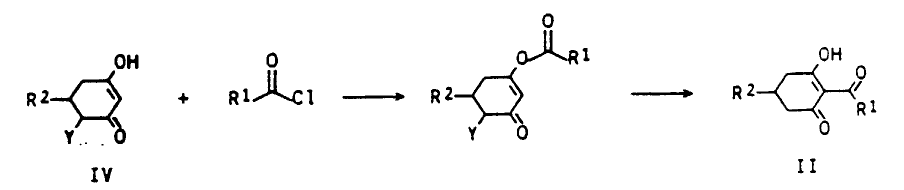

Zu den Verbindungen der Formel IV gelangt man über eine Reihe bekannter Verfahrensschritte ausgehend von bekannten Vorprodukten.

Die Synthese der Hydroxylamine III, in denen A eine unsubstituierte But-3-enylenbrücke bedeutet, erfolgt gemäß dem nachstehenden Reaktionsschema: 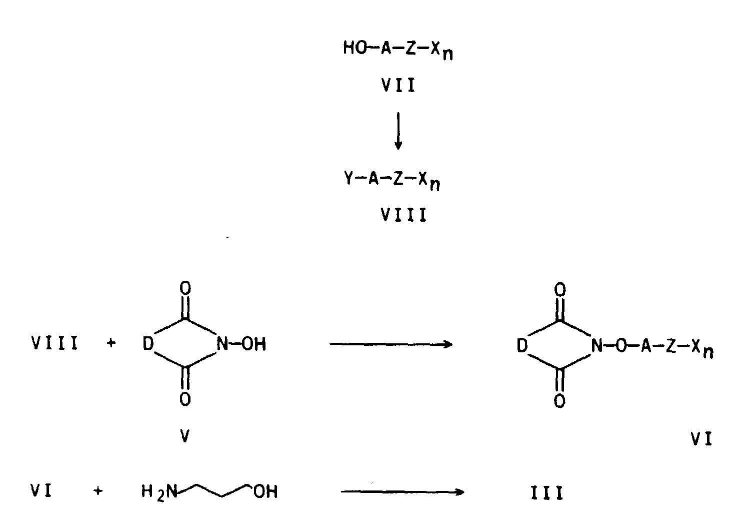 Y = Abgangsgruppe, z.B. Halogen wie Chlor, Brom und Jod oder CH₃SO₂-O-.

Die Alkylierungsmittel VIII lassen sich nach an sich bekannten Methoden herstellen [vgl. "Umlagerung von Cyclopropylhetarylcarbinolen": J. Heterocycl. Chem. 14, 525 (1976), JP 55 051 004, JP 55 047 601; Houben-Weyl: Methoden der Organischen Chemie, Band 4/3, S. 424 ff.; "Kupplung metallierter Heterocyclen mit 1,w-Dibromalkanen": DE-A 28 21 409 und Chem. Ber. 114, 3667 und 3674 (1981)].

Gewünschtenfalls können die Alkylierungsmittel VIII auf an sich bekannte Weise aus den Carbinolen VII [Kupplung von Hetaryliodiden, -bromiden mit 1,w-Alkenolen in Gegenwart von Palladiumkatalysatoren: Tetrahedron 35, 329 (1979); Chem Lett., 423 (1977); Houben-Weyl: Methoden der Organischen Chemie, Band 13/9B, s. 964 ff] erhalten werden [vgl. Houben-Weyl: Methoden der Organischen Chemie, Band 5/3. S. 862 ff und S. 899 ff und dto., Band 5/4, S. 361 ff.] Vorzugsweise koppelt man das Alkylierungsmittel mit einem cyclischen Hydroxyimid V und spaltet das hierbei erhaltene geschützte Hydroxylaminderivat VI zum freien Hydroxylamin III, z.B. mit 2-Aminoethanol.

In den cyclischen Hydroxyimiden V steht D z.B. für C₂-C₃-Alkylen, C₂-Alkenylen oder einen mit bis zu 3 Doppelbindungen und gegebenenfalls 1 Stickstoffatom enthaltenden 5- oder 6-Ring, z.B. für Phenylen, Pyridinylen, Cyclopentylen, Cyclohexylen oder Cyclohexenylen. Beispielsweise kommen folgende Substanzen in Betracht:

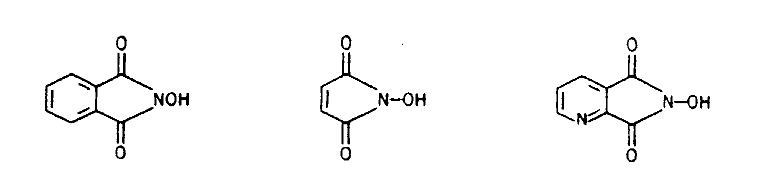

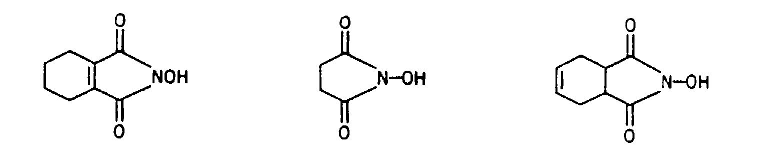

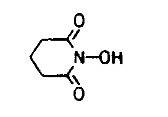

Die Umsetzung der Verbindungen VIII mit den Hydroxyimiden V wird zweckmäßigerweise in Gegenwart einer Base ausgeführt. Geeignet sind prinzipiell alle Basen, die in der Lage sind, die Hydroxyimide V zu deprotonieren ohne das Imidsystem anzugreifen. Dies sind insbesondere die sogenannten nicht-nucleophilen Basen. Beispielsweise genannt seien Mineralbasen wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazabicyclooctan, Diazabicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid V eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids V einzusetzen, um einem nucleophilen Angriff der Hydroxylionen auf die Carbonylfunktion der Imidgruppierung vorzubeugen.

Zweckmäßigerweise setzt man die Ausgangsverbindungen VIII mit den Hydroxyimiden V in einem Lösungsmittel um, das sich unter den Reaktionsbedingungen inert verhält. Vorteilhafte Lösungsmittel sind z.B. polareaprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen VIII mit den Hydroxyimiden V kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37 - 45 und S. 86 - 93, Verlag Chemie, Weinheim 1980, beschrieben sind. Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen VIII mit den Hydroxyimiden V wird im allgemeinen im Temperaturbereich zwischen 0 und 140°C, bevorzugt zwischen 20 und 100°C, insbesondere zwischen 40 und 80°C, durchgeführt. Zweckmäßigerweise wird dabei so vorgegangen, daß man das Hydroxyimid V zusammen mit der Base im Lösungsmittel vorlegt und das Ausgangsmaterial VIII zu dieser Lösung dosiert. Dabei kann es sich als günstig erweisen, wenn das Hydroxyimid bei einer tieferen Temperatur, bespielsweise bei 0 bis 50°C, zugegeben und die Reaktionsmischung erst nach dieser Zugabe auf die eigentliche Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate VI als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate VI können zwischengelagert werden oder sogleich in die Hydroxylaminderivate III mit freier Aminogruppe umgewandelt werden.

Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nach dem die Hydroxylaminderivate III mittels Ethanolamin freigesetzt werden. Die Freisetzung der Hydroxylaminderivate III mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate III mittels üblicher Aufarbeitungsmethoden isoliert werden, beispielsweise durch Extraktion oder durch Kristallisation. Zur Erhöhung der Kristallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in äquivalenten Mengen. Die erhaltenen Hydroxylammoniumsalze können wie die Hydroxylaminderivate mit freier Aminogruppe direkt zu den Herbiziden der Formel I weiterverarbeitet werden oder auch, falls gewünscht, gelagert werden.

Im Hinblick auf die biologische Wirksamkeit werden Cyclohexenone der Formel I bevorzugt, in denen die Substituenten folgende Bedeutung haben:
- R¹: Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Ethyl und Propyl;
- A: Alkylen oder Alkenylen wie Ethylen, Propylen, Prop-2-enylen, Butylen, But-2-enylen, But-3-enylen, Pentylen, Pent-4-enylen, Hexylen, Hex-5-enylen, wobei die genannten Gruppen ein- bis dreifach durch insbesondere Methyl bzw. Ethyl und/ oder Fluor bzw. Chlor substituiert sein können; bei den ungesättigten Ketten können sowohl die cis- als auch die trans-Form auftreten. Besonders bevorzugt sind But-2-enylen und But-3-enylen.
- Z: 5-gliedriges Heteroaryl wie Furanyl, Pyrrolyl, Thienyl, Imidazolyl, Pyrazolyl, Isoxazolyl, Oxazolyl, Isothiazolyl, Thiazolyl, Oxadiazolyl, Thiadiazolyl, Triazolyl, insbesondere Furanyl und Thienyl,
6-gliedriges Heteroaryl wie Pyridyl, Pyridazyl, Pyrimidyl, Pyrazyl, Triazyl, Tetrazyl, insbesondere Pyridyl und Pyrimidyl;
- X: Nitro, Cyano
Halogen wie Fluor, Chlor, Brom und Iod, insbesondere Fluor und Chlor;
Alkyl wie Methyl, Ethyl, Propyl, 1-Methylethyl, Butyl, 1-Methylpropyl, 2-Methylpropyl und 1,1-Dimethylethyl, insbesondere Methyl und 1,1-Dimethylethyl,
Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio,
Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl,
Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,
Carboxyl,
Alkoxycarbonyl wie Methoxycarbonyl, Ethoxycarbonyl, Propyloxycarbonyl, 1-Methylethoxycarbonyl, Butyloxycarbonyl und 1,1-Dimethylethoxycarbonyl, insbesondere Methoxycarbonyl, Ethoxycarbonyl und 1,1-Dimethylethoxycarbonyl, insbesondere Methoxycarbonyl.

Benzyloxycarbonyl und Phenyl, wobei die aromatischen Reste ihrerseits ein bis drei der folgenden Reste tragen können: Nitro, Cyano, Carboxyl, Benzyloxycarbonyl, Halogen wie im allgemeinen und im besonderen bei X genannt; Alkyl wie bei R1 genannt, insbesondere Methyl, Ethyl und 1-Methylethyl; Alkoxy wie vorstehend genannt, insbesondere Methoxy und Ethoxy; Alkylthio wie vorstehend genannt, insbesondere Methylthio; Halogenalkyl wie vorstehend genannt, insbesondere Trifluormethyl; Halogenalkoxy wie vorstehend genannt, insbesondere Difluormethoxy und Trifluormethoxy und/oder Alkoxycarbonyl wie vorstehend genannt, insbesondere Methoxycarbonyl und Ethoxycarbonyl.

Eine Aminogruppe -NR^{a}R^{b}:
- R^{a}: Wasserstoff;
Alkyl wie Methyl, Ethyl, Propyl, 1-Methylethyl, Butyl, 1-Methylpropyl, 2-Methylpropyl und 1,1-Dimethylethyl, insbesondere Methyl und Ethyl;
Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,3-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl und 2-Butenyl;
- R^{b}: Wasserstoff;
Alkyl wie vorstehend im allgemeinen und im besonderen genannt;
Alkenyl wie vorstehend im allgemeinen und im besonderen genannt;

Alkinyl wie 2-Propinyl, 2-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-1,1-Dimethyl-3-butinyl, 1,2-Dimethyl-3-butinyl, 1-Ethyl-3-butinyl, 2-Ethyl-3-butinyl und l-Ethyl-l-methyl-2-propinyl, insbesondere 2-Propinyl und 2-Butinyl;
Acylgruppen wie Acetyl, Propionyl, Butyryl, 2-Methylpropionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutyryl, insbesondere Acetyl und Propionyl;
oder ein Benzoylrest, wobei der aromatische Rest ein bis drei der folgenden Reste tragen kann: Nitro, Cyano, Carboxyl, Benzyloxycarbonyl, Halogen wie im allgemeinen und im besonderen bei X genannt; Alkyl wie bei R¹ genannt, insbesondere Methyl, Ethyl und 1-Methylethyl; Alkoxy wie vorstehend genannt, insbesondere Methoxy und Ethoxy; Alkylthio wie vorstehend genannt, insbesondere Methylthio; Halogenalkyl wie vorstehend genannt, insbesondere Trifluormethyl; Halogenalkoxy wie vorstehend genannt, insbesondere Difluormethoxy und Trifluormethoxy und/oder Alkoxycarbonyl wie vorstehend genannt, insbesondere Methoxycarbonyl und Ethoxycarbonyl;
- n: 0, 1, 2 oder 3, insbesondere 0, 1 und 2, im Fall daß Z einen halogensubstituierten Pyridylrest bedeutet auch 1 bis 4. Bevorzugt steht n für 0, 1 oder 2. Bei mehreren Resten X können die Substituenten gleich oder verschieden sein;
- R²: Alkyl wie unter X genannt, sowie Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl und 1-Ethyl-2-methylpropyl, welche mit einer der unter X genannten Alkoxy-oder Alkylthiogruppe bevorzugt in 1-, 2- oder 3-Position substituiert sind, insbesondere 2-Ethylthiopropyl;
5-gliedriges Heterocycloalkyl wie Tetrahydrofuranyl, Tetrahydrothienyl, Dioxolanyl, Dithiolanyl und Oxathiolanyl, insbesondere Tetrahydrofuranyl Tetrahydrothienyl und Dioxolanyl, wobei diese Ringe ein bis drei der bereits unter X genannten C₁-C₄-Alkylgruppen, C₁-C₄-Alkoxygruppen, C₁-C₄-Alkylthiogruppen und/oder C₁-C₄-Halogenalkylgruppen tragen können,
5-gliedriges Heteroaryl wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl,
ein 6- oder 7-gliedriger Heterocyclus wie Tetrahydropyran-3-yl, -3-yl, Dihydropyran-3-yl, Tetrahydropyran-4-yl, Dihydropyran-4-yl, Tetrahydrothiopyran-3-yl, Dihydrothiopyran-3-yl, Tetrahydrothiopyran-4-yl, Dihydrothiopyran-4-yl und Dioxepan-5-yl, insbesondere Tetrahydropyran-3-yl, Tetrahydropyran-4-yl und Tetrahydrothiopyran-3-yl,
ein Phenyl- oder ein Pyridylrest,
wobei die cyclischen Reste ein bis drei der unter X genannten Alkylgruppen, Alkoxygruppen, Alkylthiogruppen und/oder Halogenalkylgruppen tragen können.

Die 5-gliedrigen Heteroaromaten in der Bedeutung R² können als Substituenten folgende Reste tragen:
Halogenatom wie unter X genannt, insbesondere Fluor und Chlor,
Alkoxyalkyl wie Methoxymethyl, 2-Methoxyethyl, 2-Methoxypropyl, 3-Methoxypropyl, 2-Methoxy-l-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl,
Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-2-Methyl-2-pentenyl, 3-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-l-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3, 3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Tri-methyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl bzw. entsprechendes Alkenyloxy und/oder Halogenalkenyl.

Die 6- und 7-gliedrigen Heterocyclen können neben den vorstehend genannten Substituenten auch Hydroxygruppen tragen.

Bei den Phenyl- und Pyridylresten kommen als Substituenten neben den obengenannten Gruppen auch folgende Reste in Betracht:
Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyl, -butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyl, und 1-Ethyl-2-methyl-2-propenyloxy, insbesondere 2-Propenyloxy und 2-Butenyloxy;
Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-l-methyl-2-propinyloxy, insbesondere 2-Propinyloxy und 2-Butinyloxy;
Alkoxyalkyl wie im allgemeinen und im besonderen vorstehend genannt; -NR^{a}R^{b} wie bei X im allgemeinen und im besonderen genannt.

Insbesondere bevorzugte Cyclohexenonoximether der Formel I sind in den folgenden Tabellen zusammengefaßt.

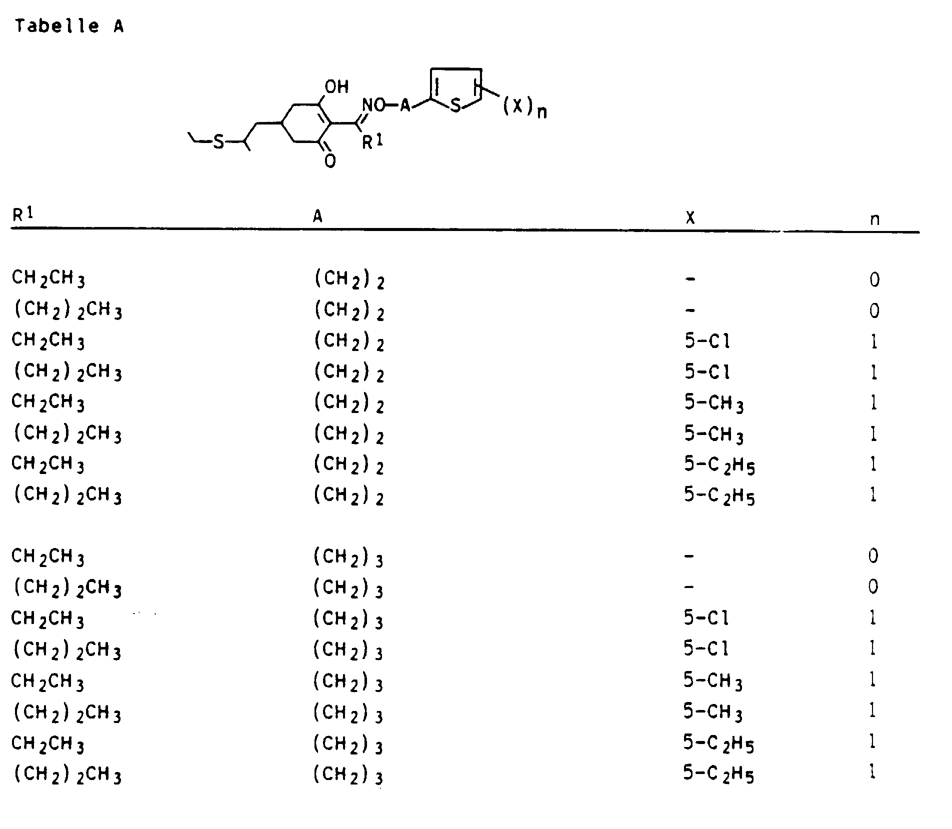

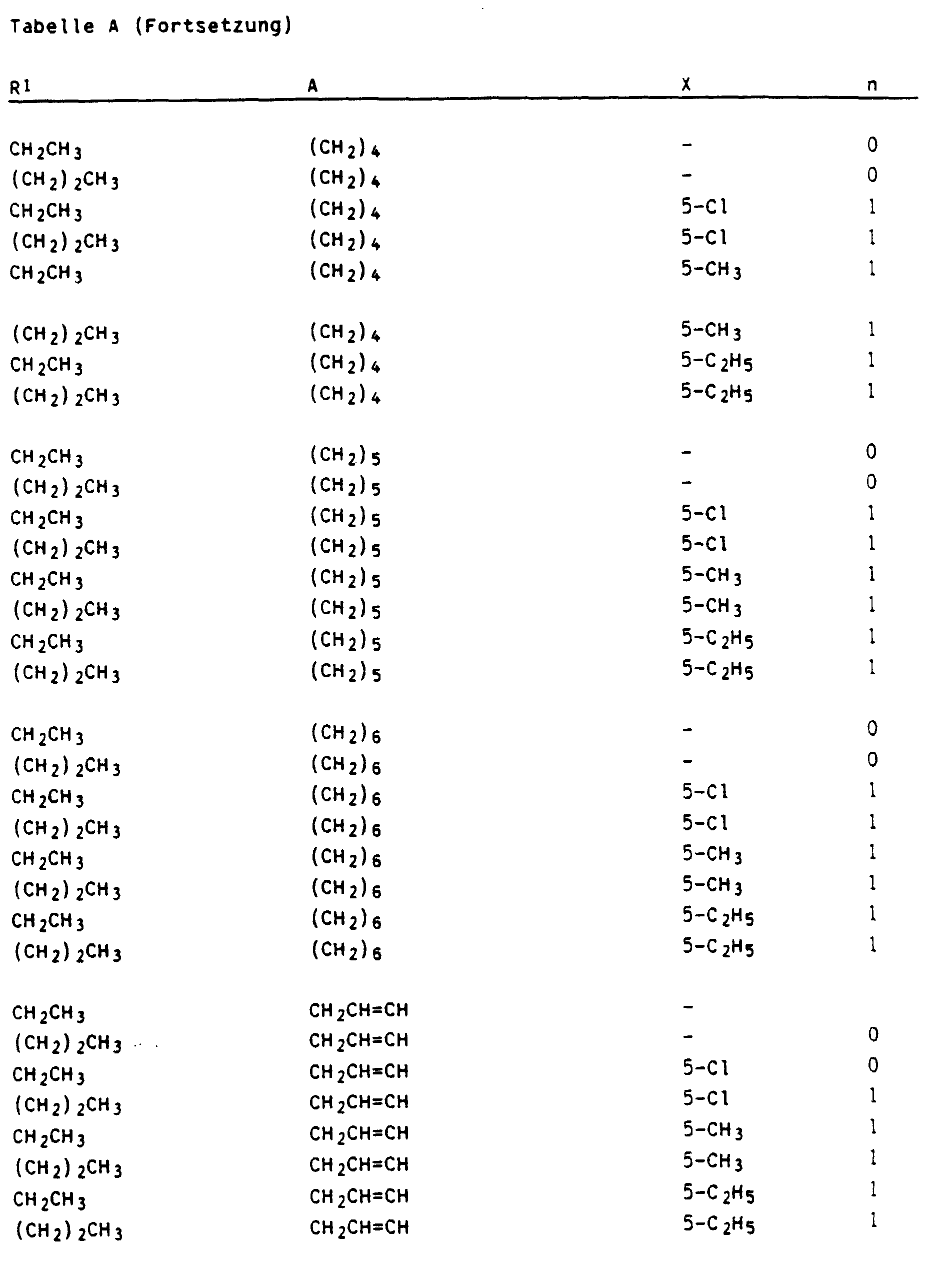

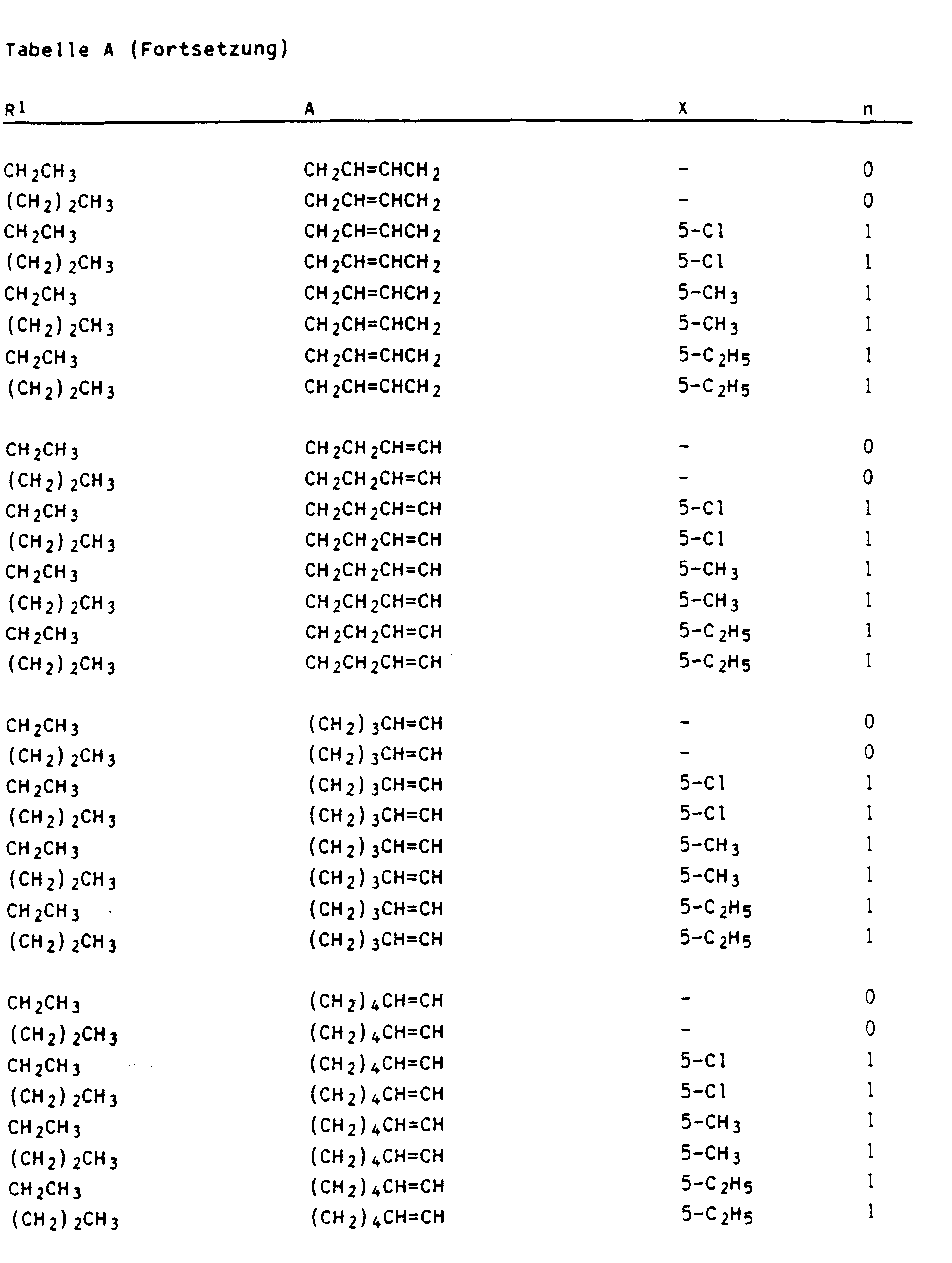

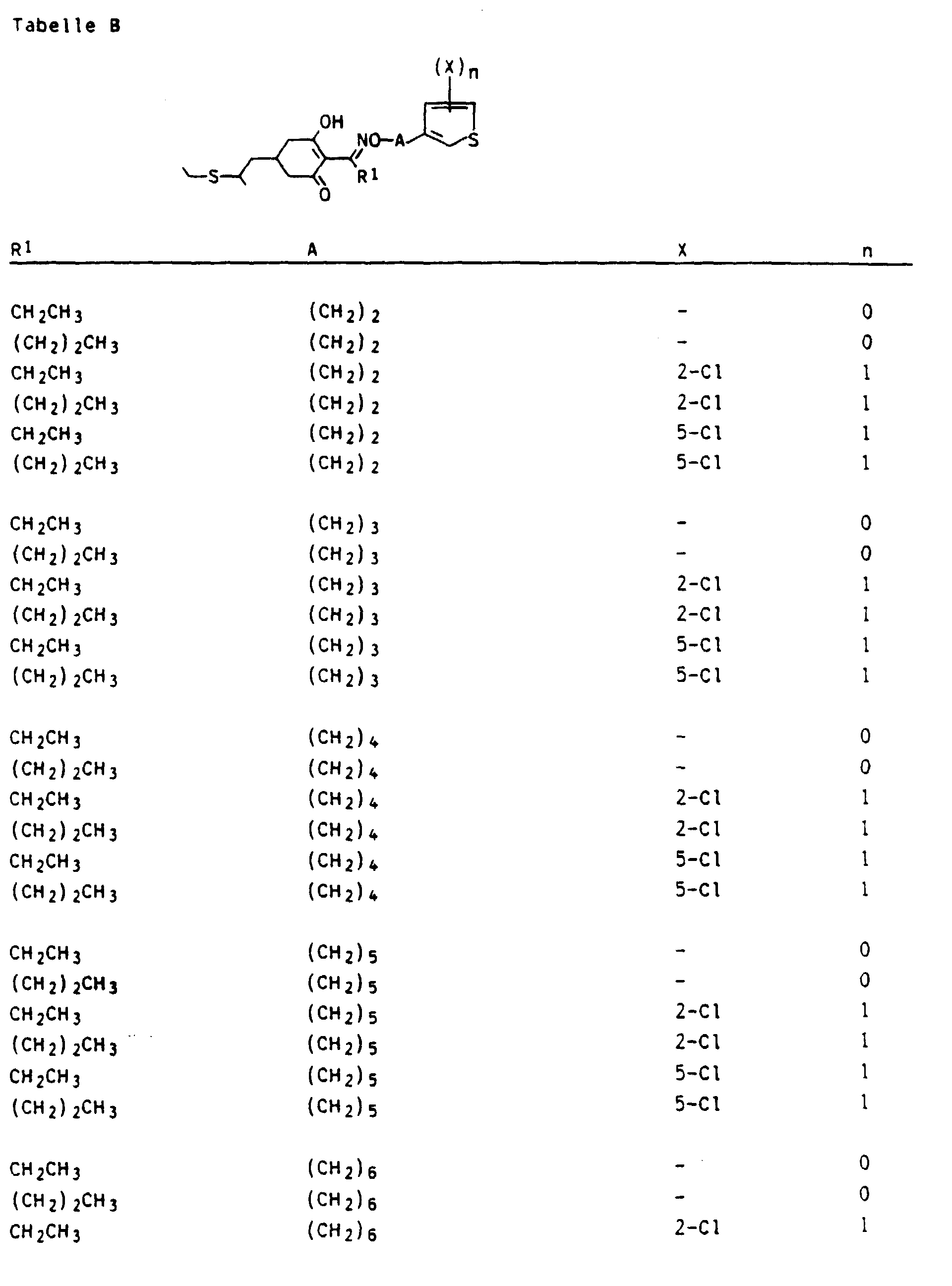

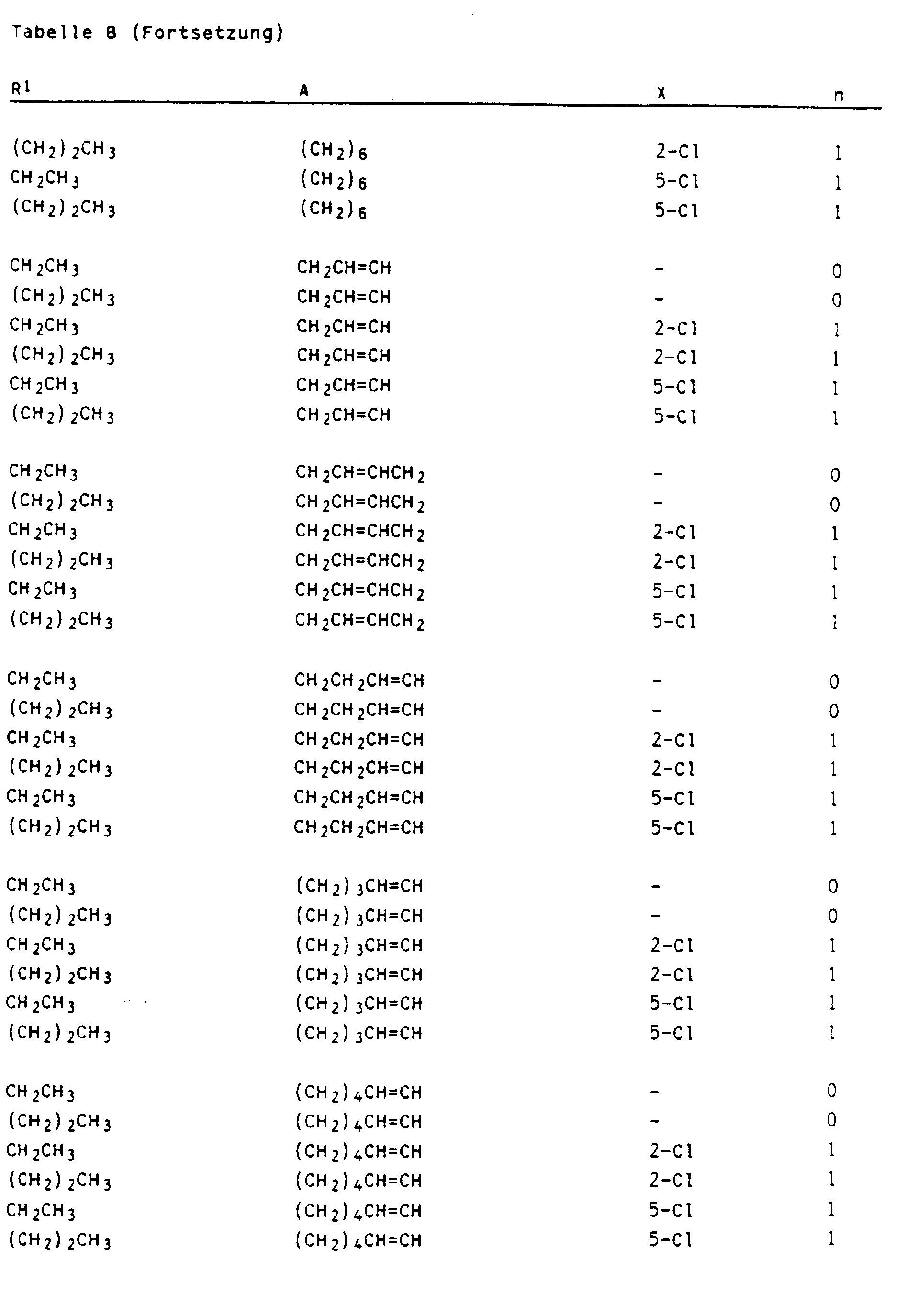

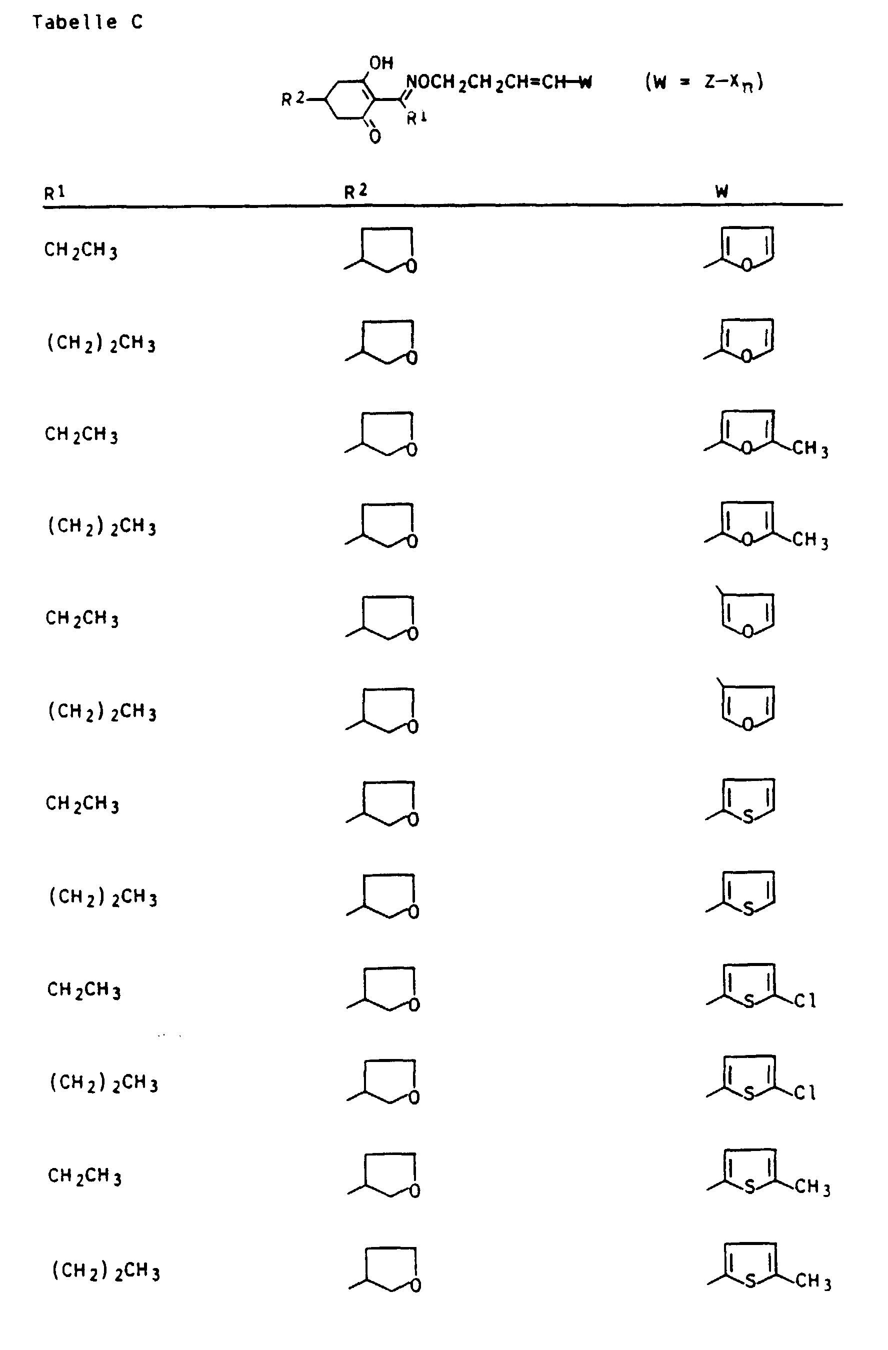

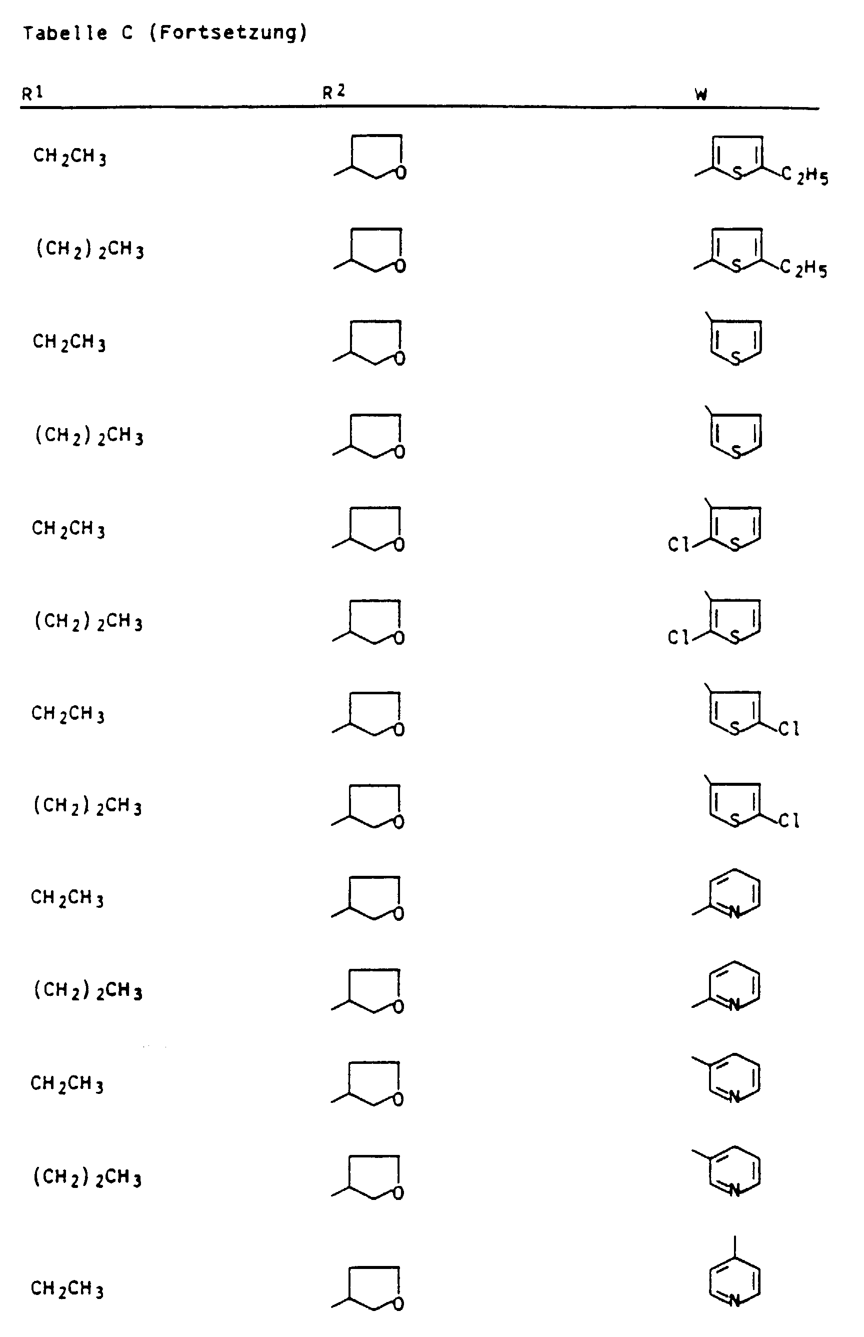

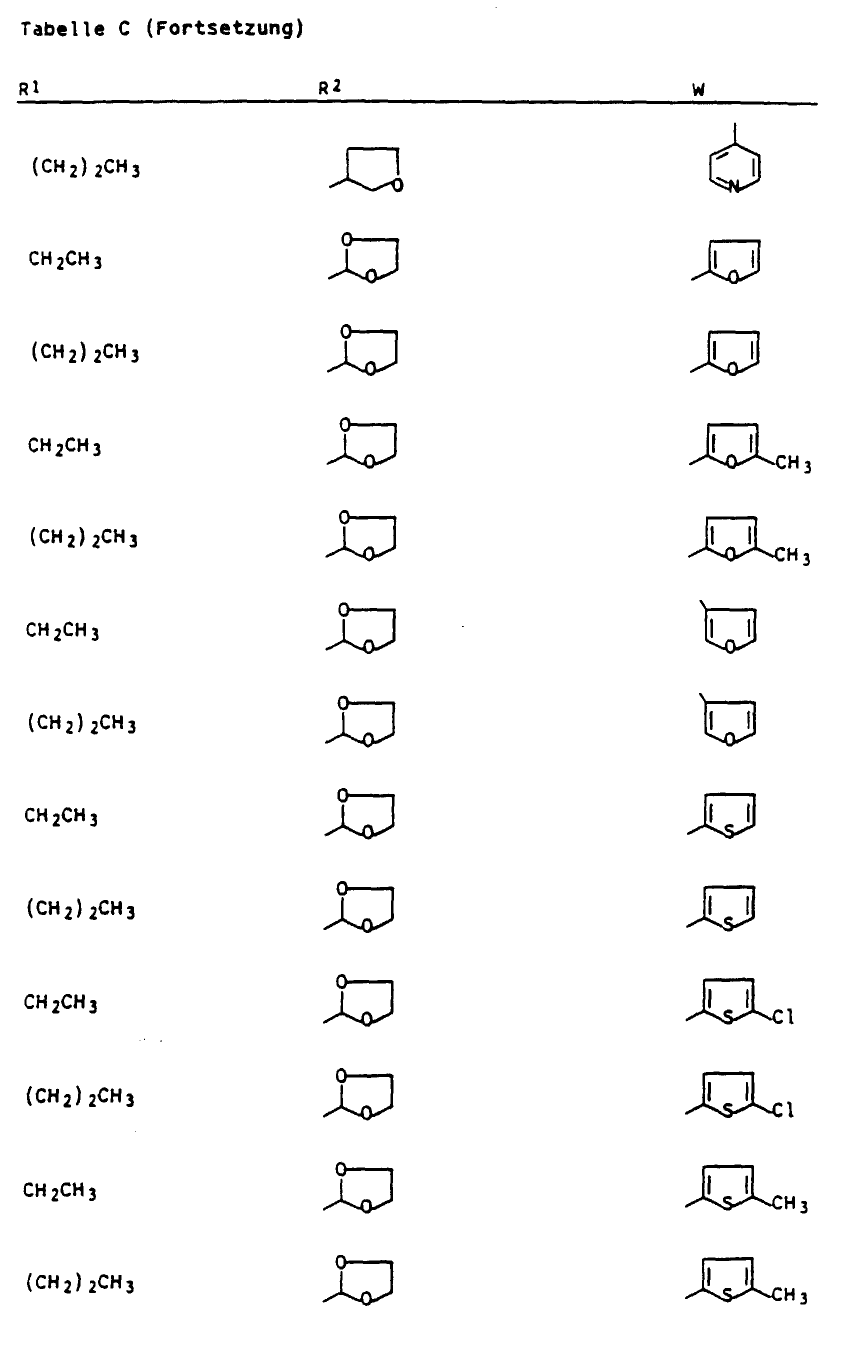

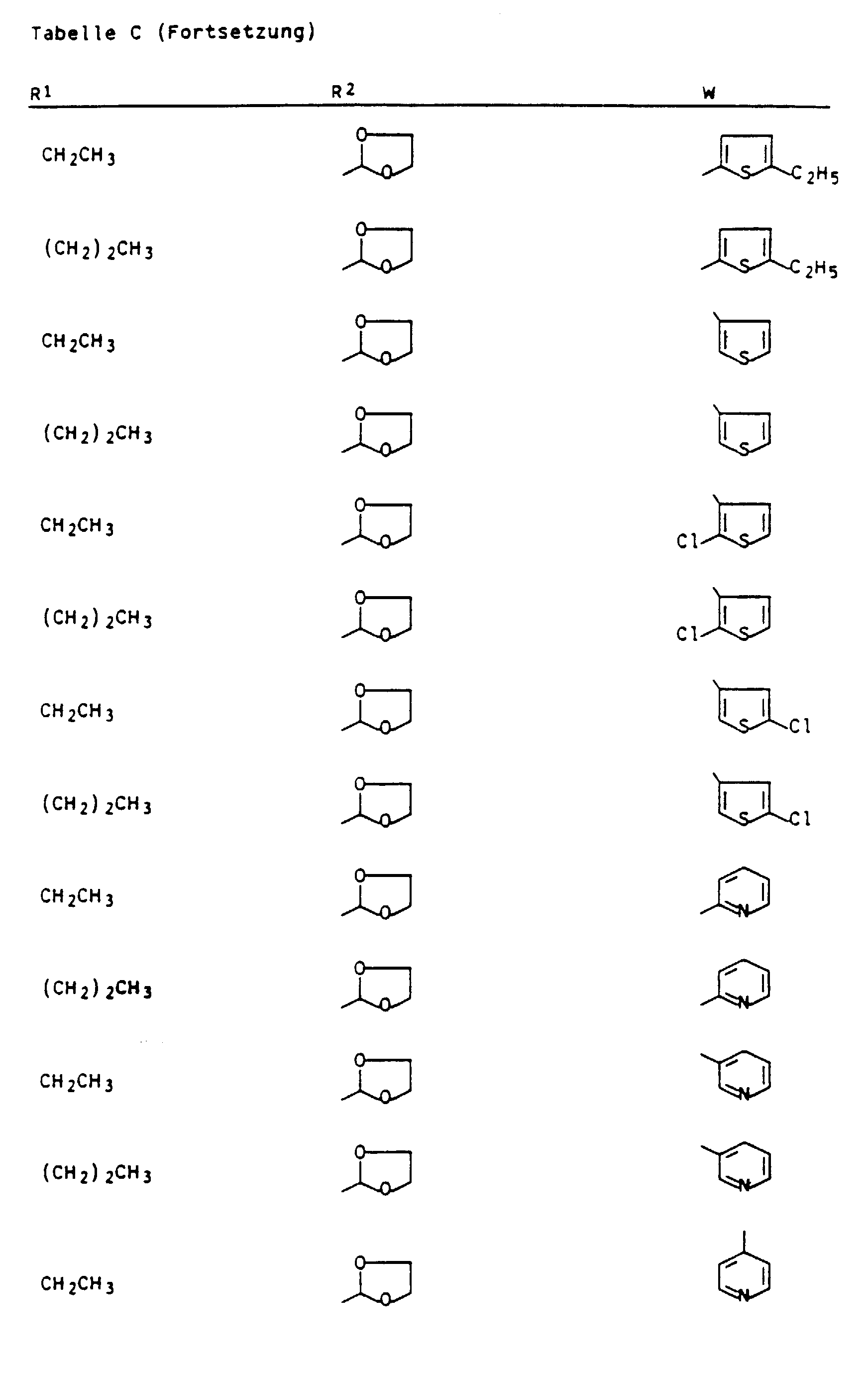

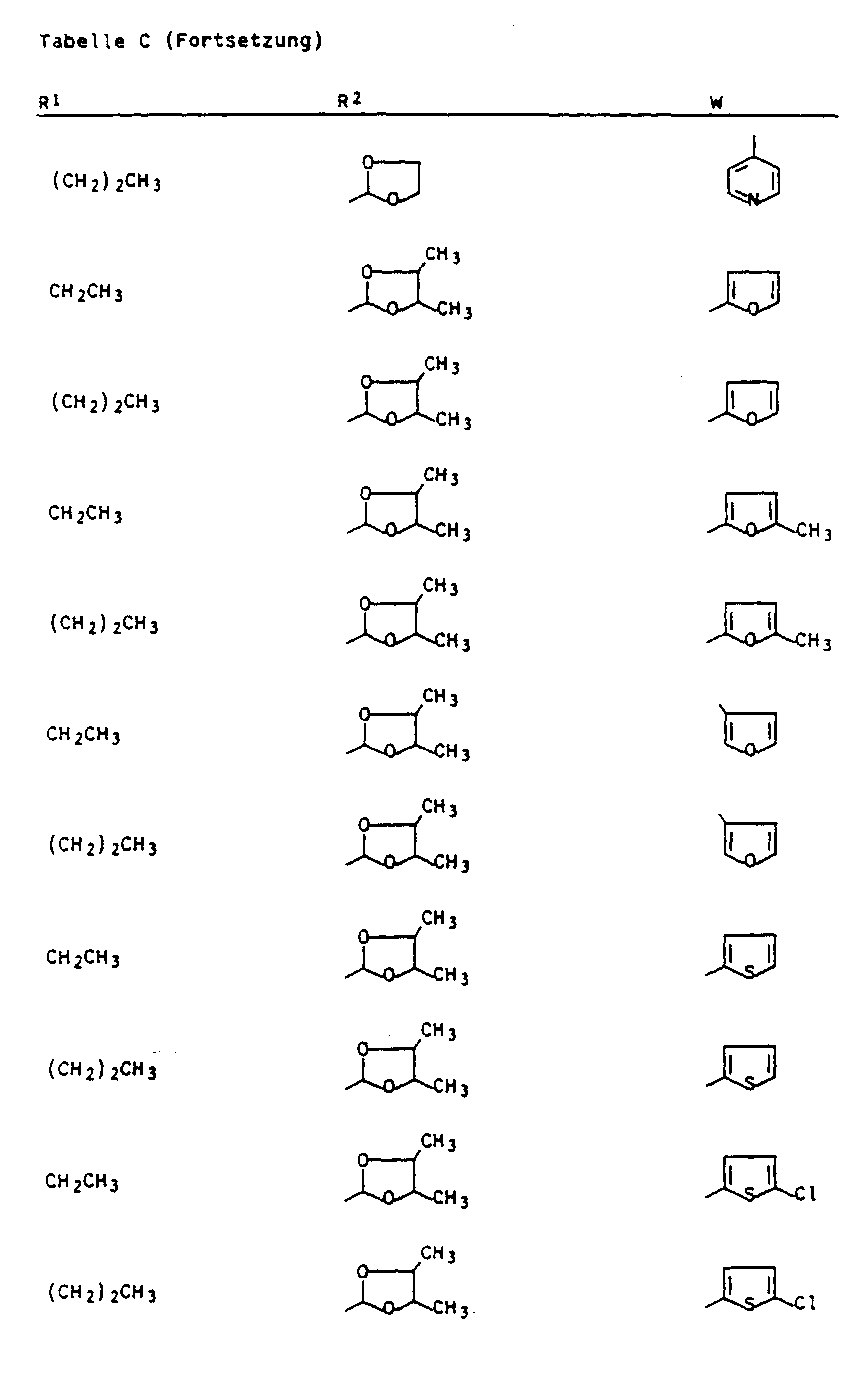

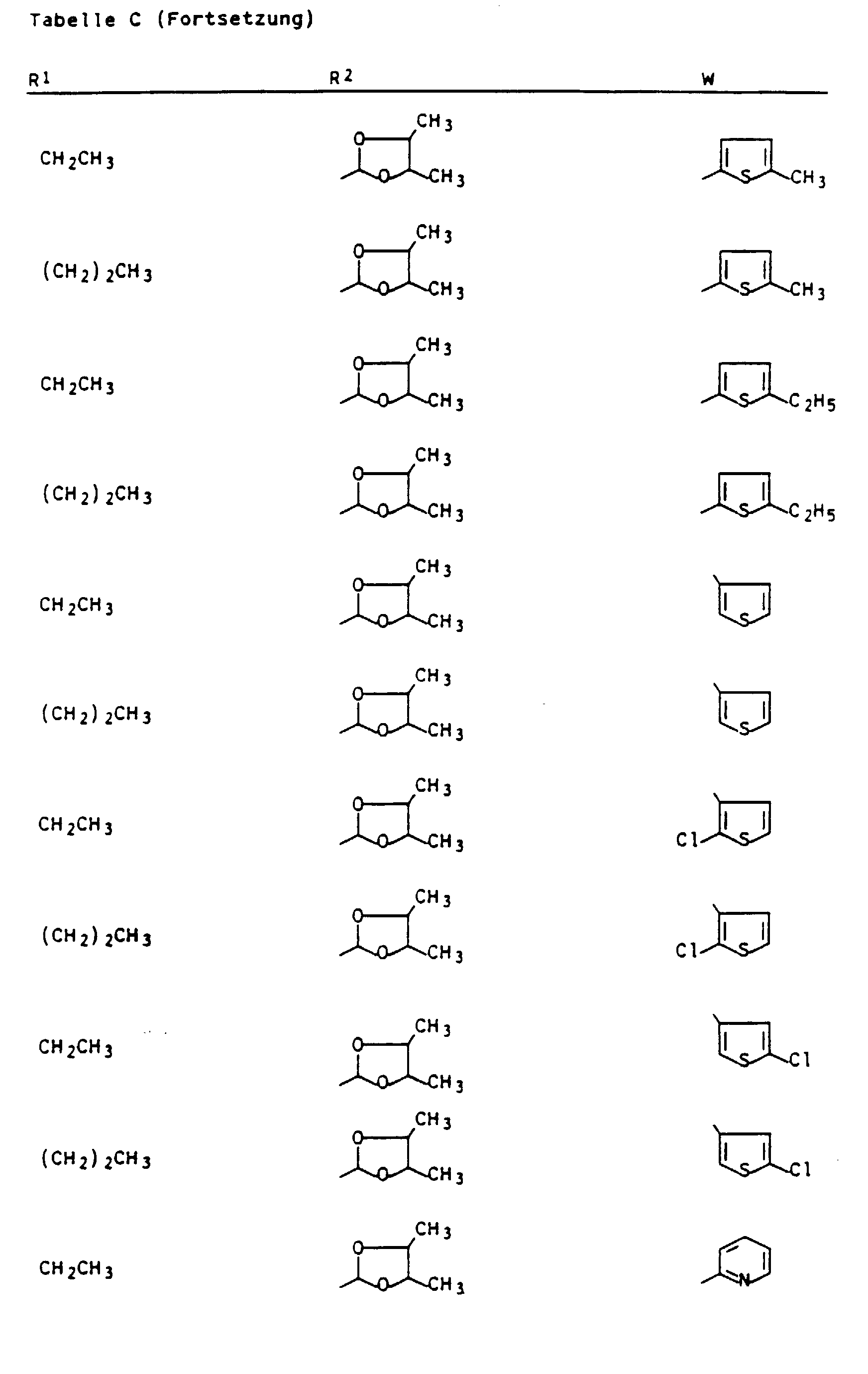

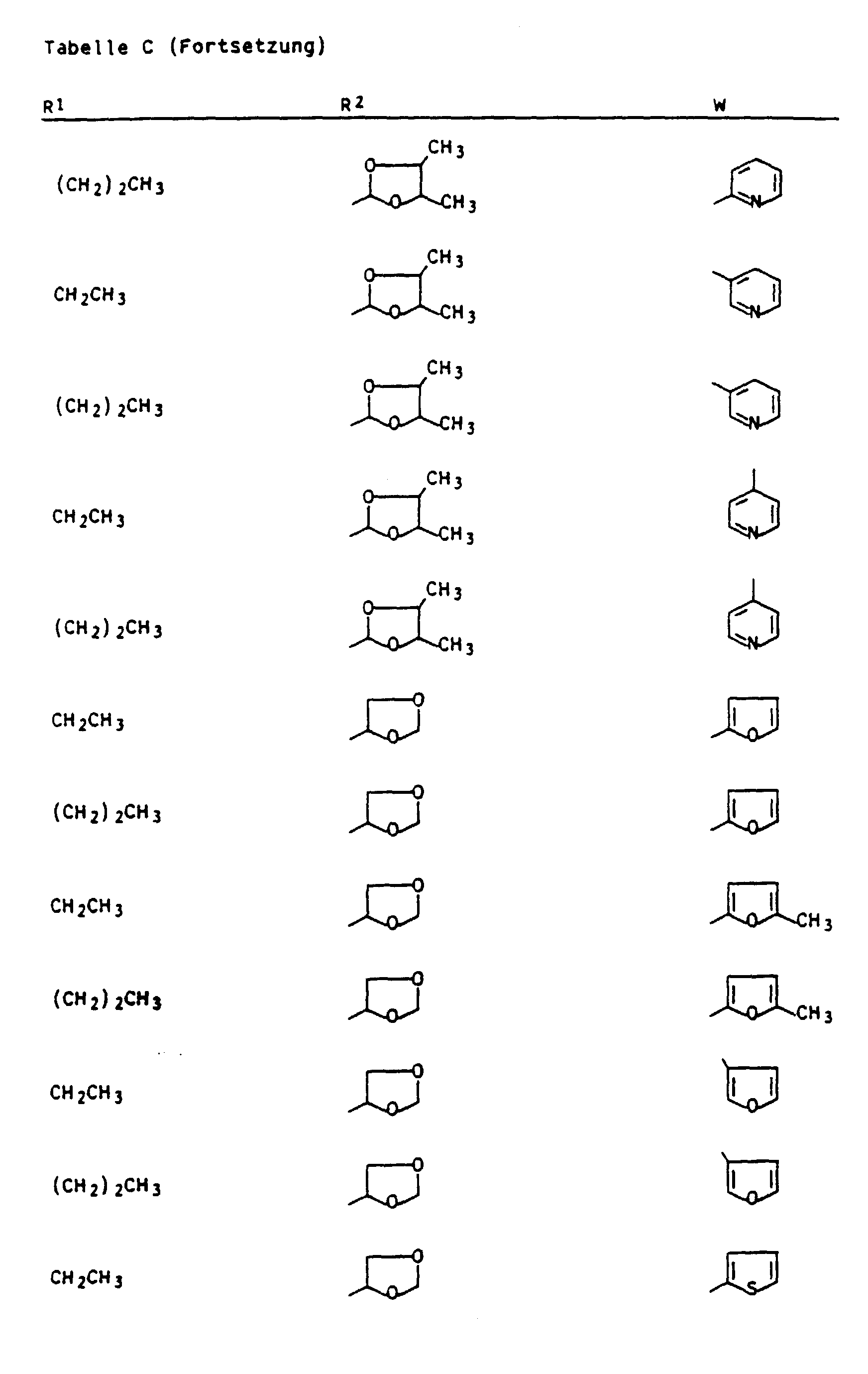

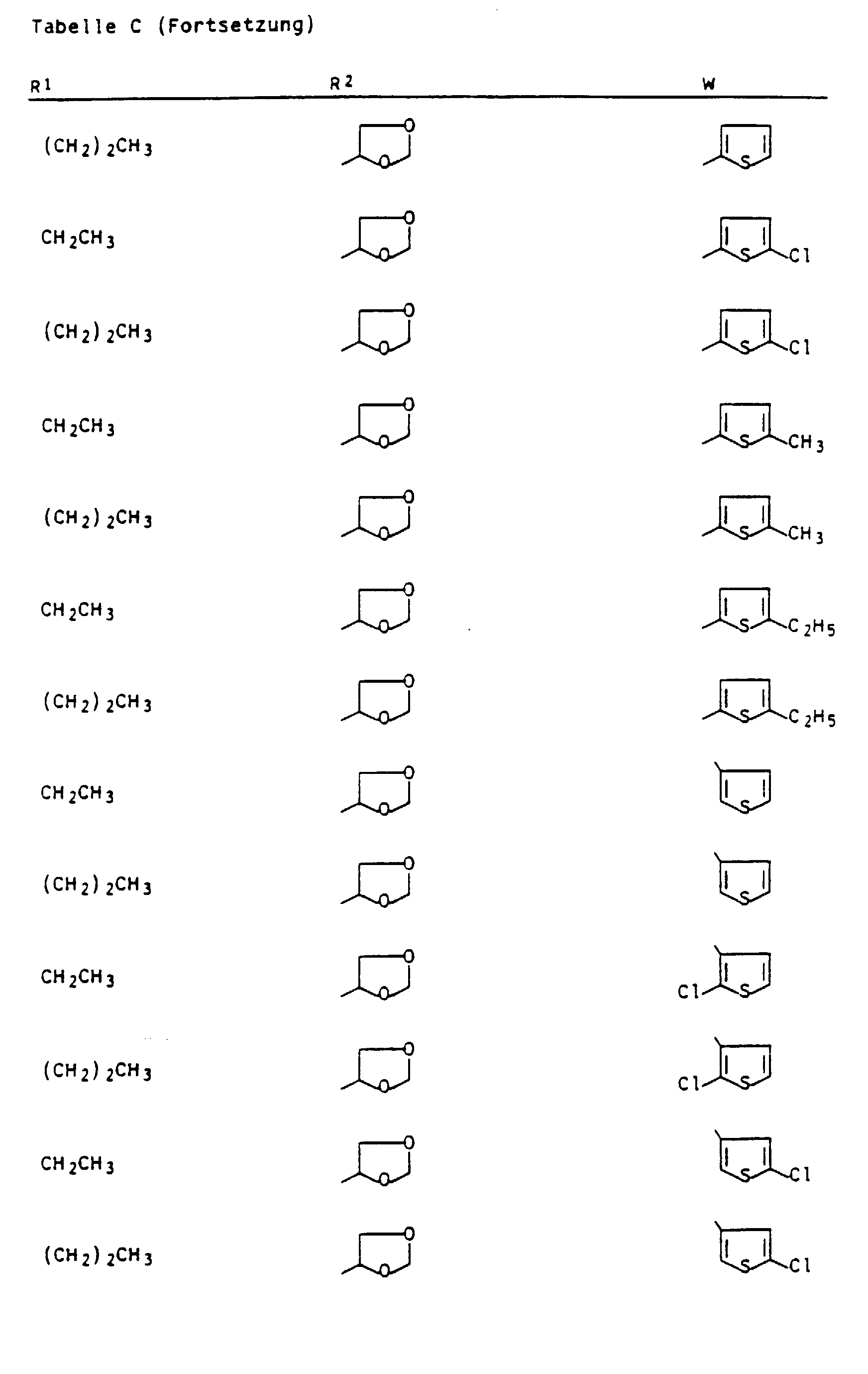

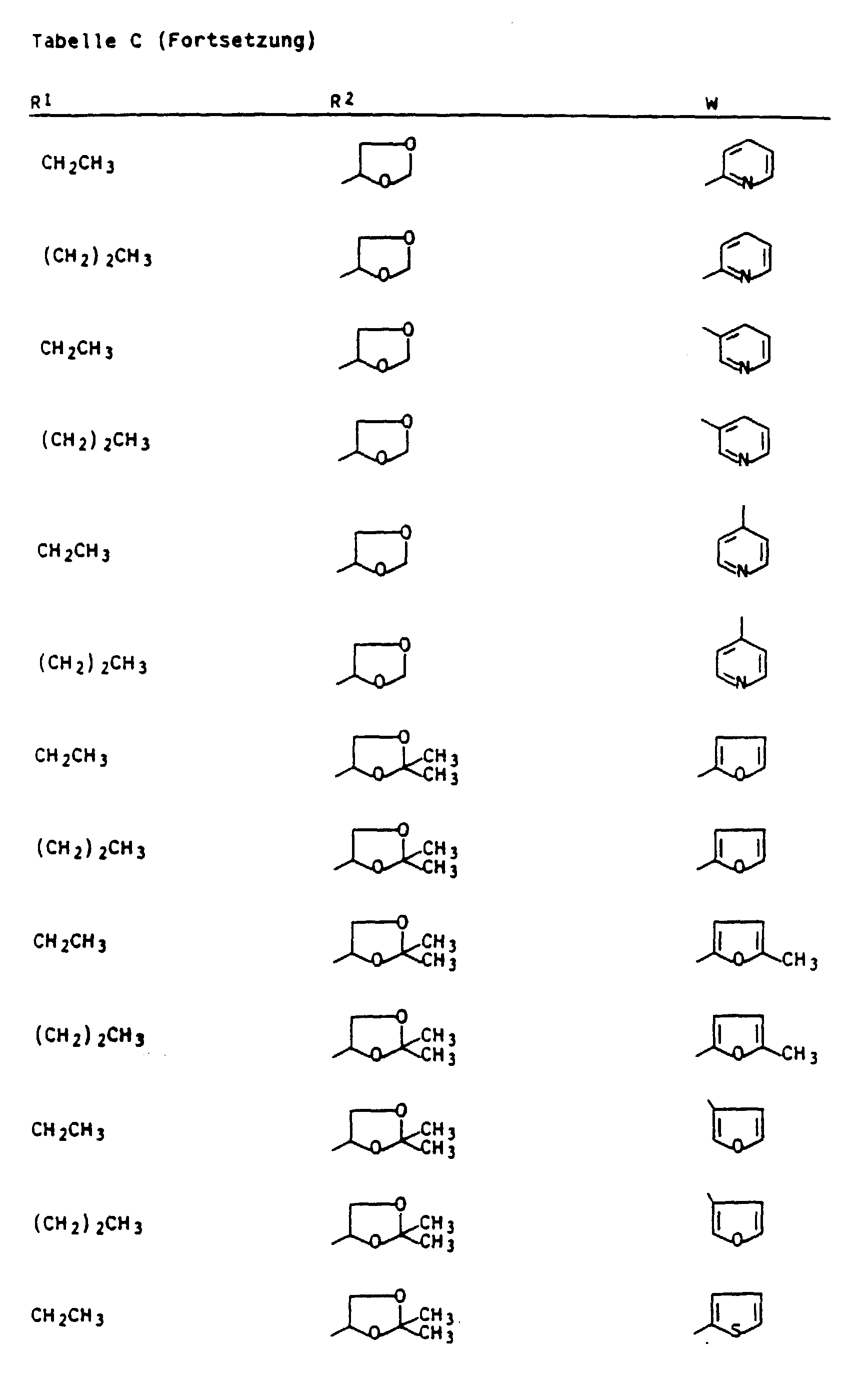

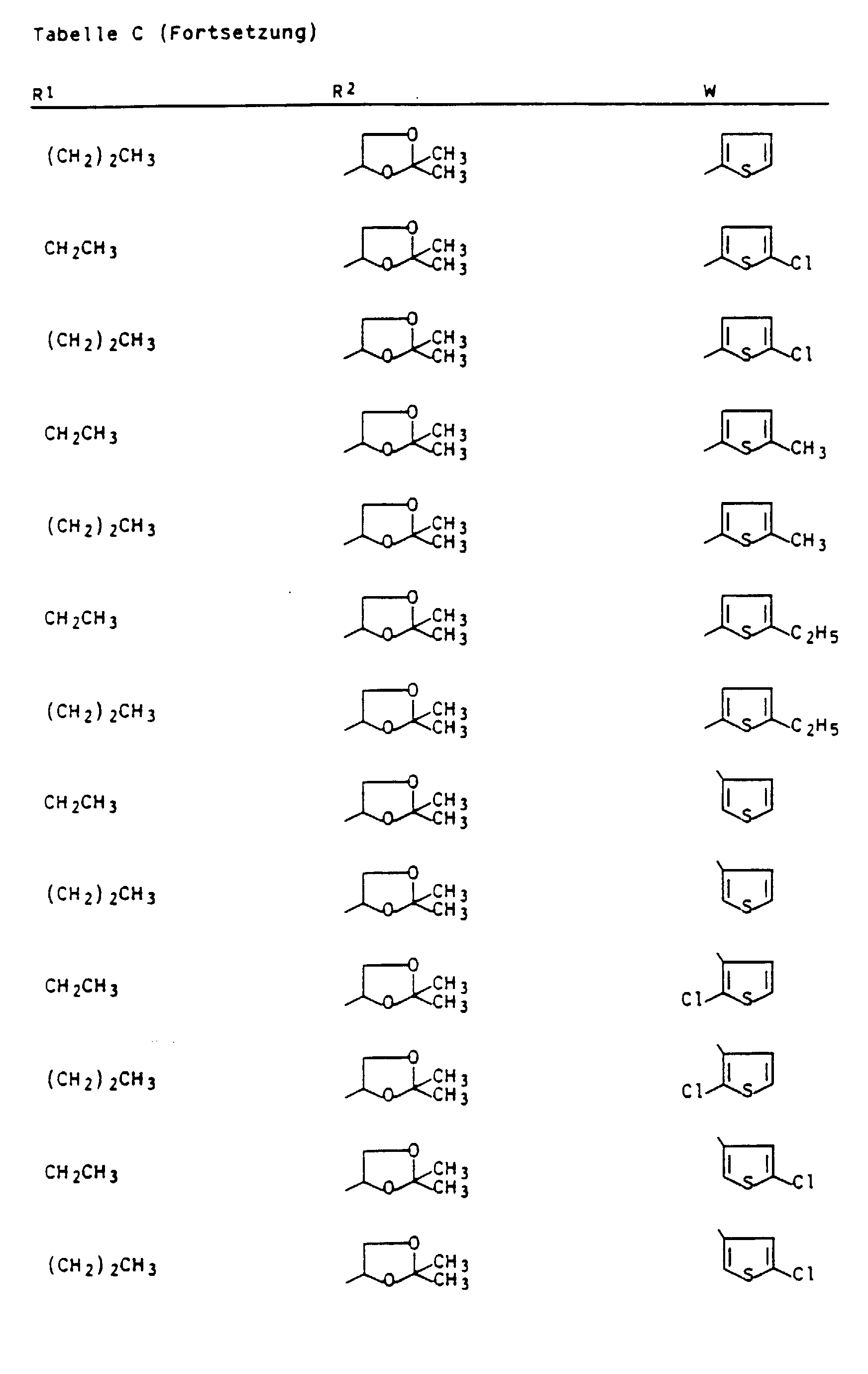

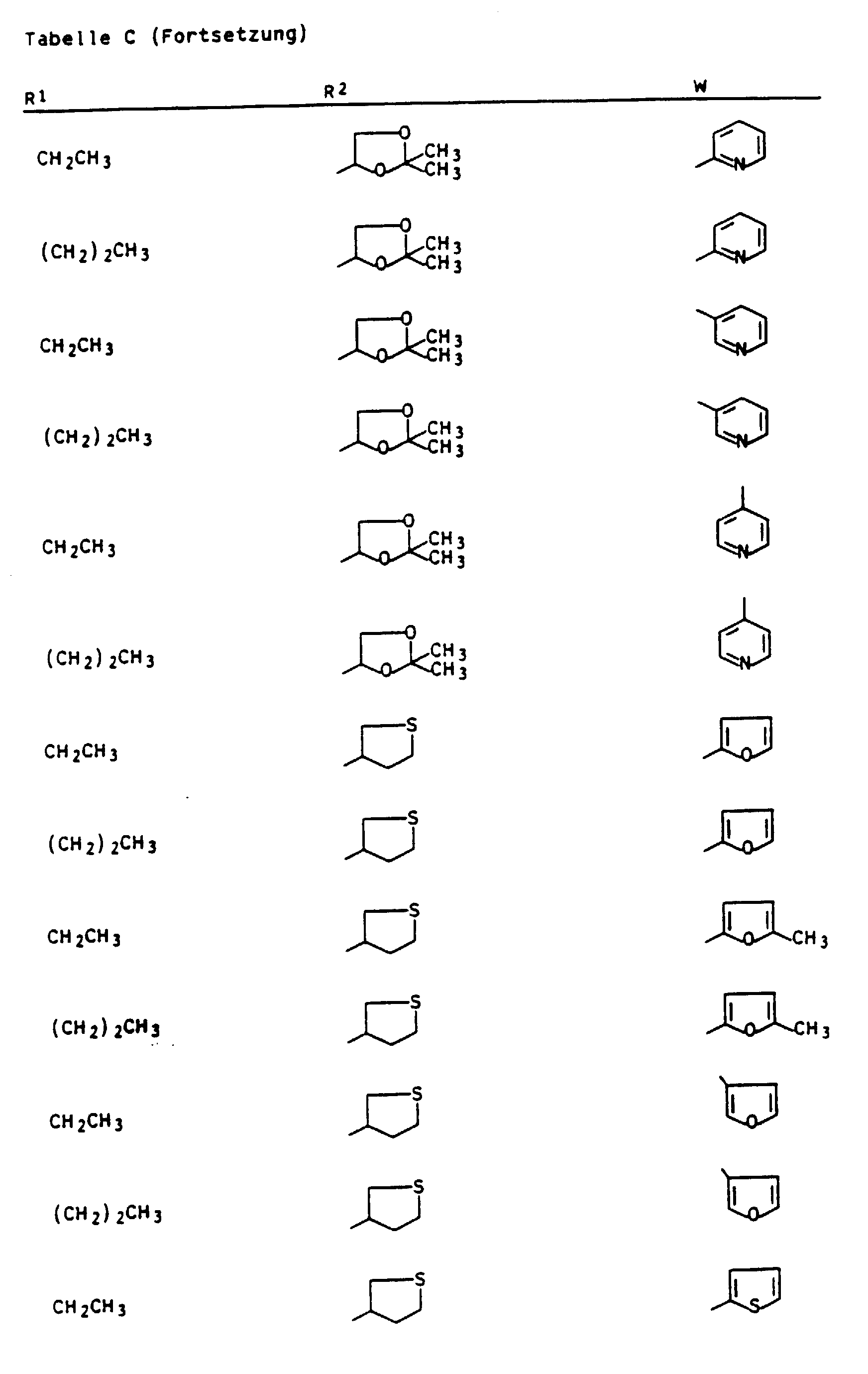

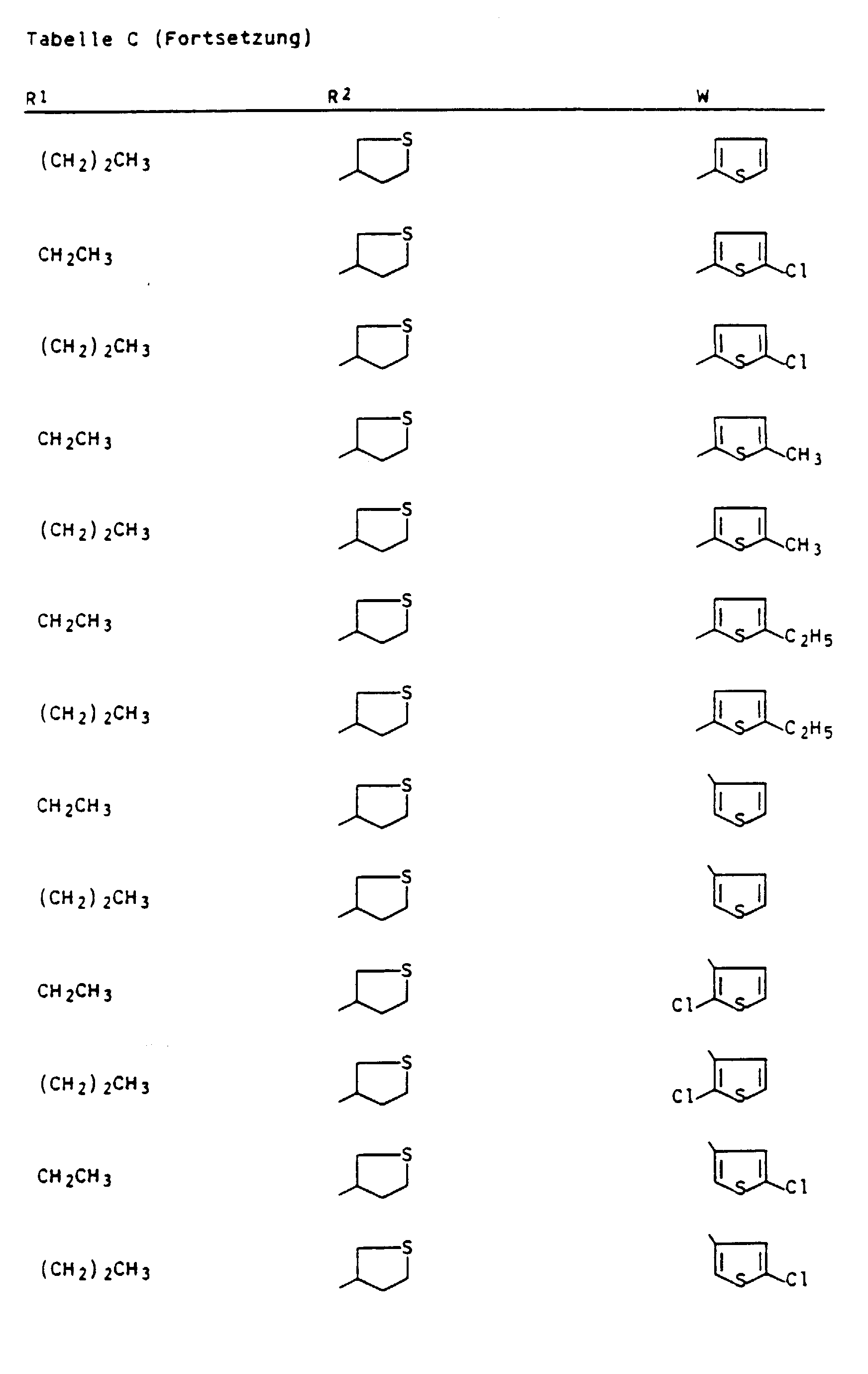

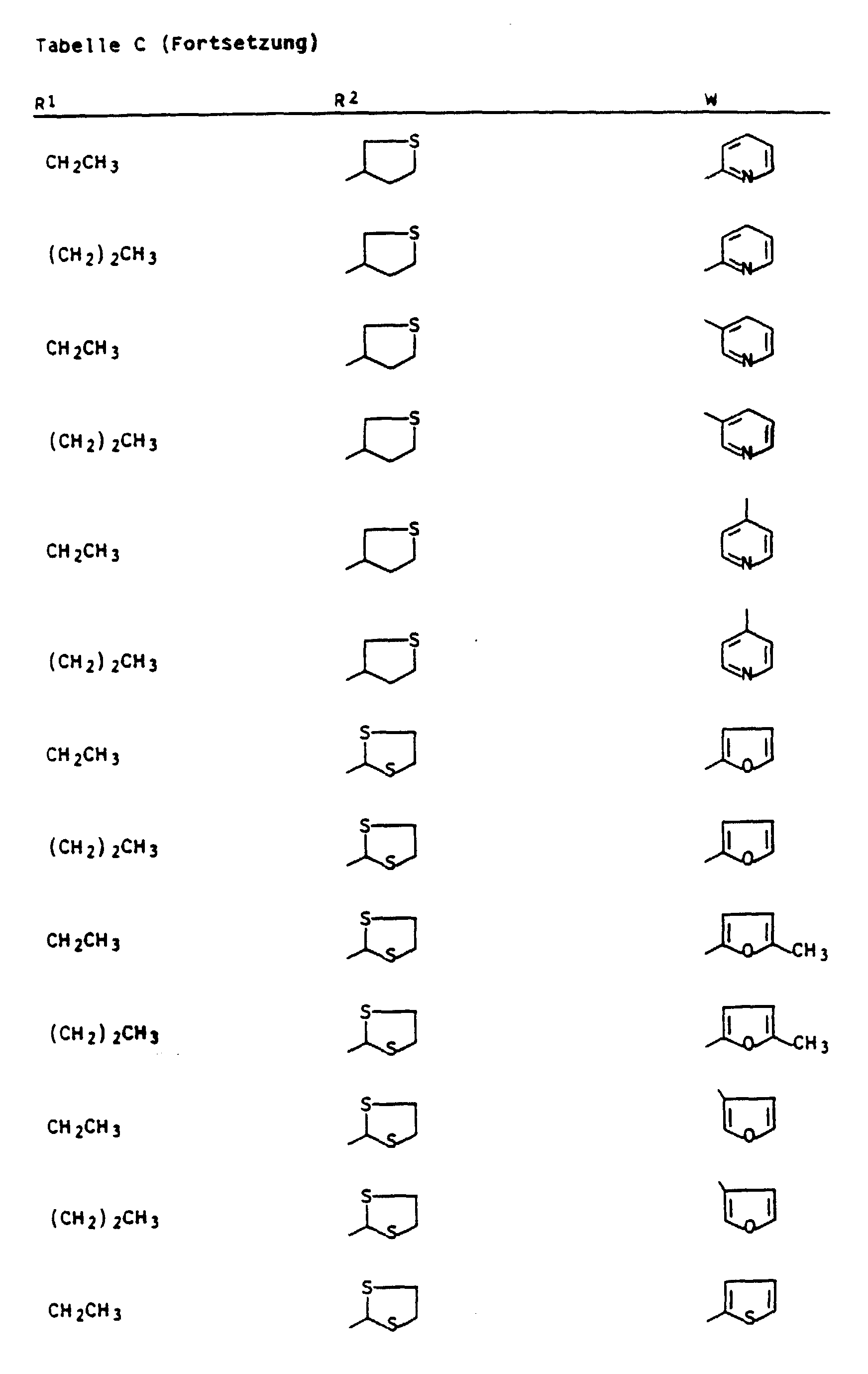

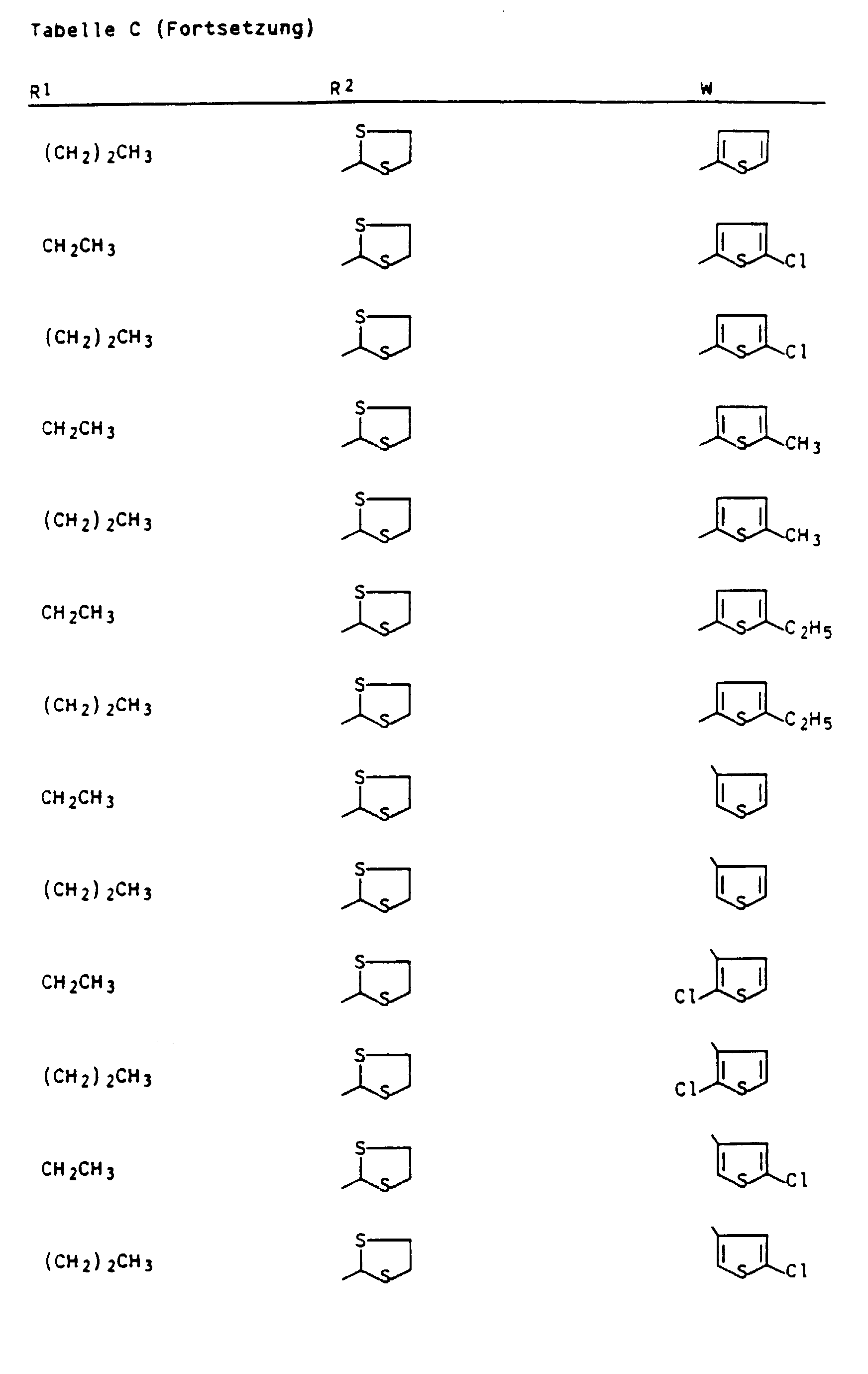

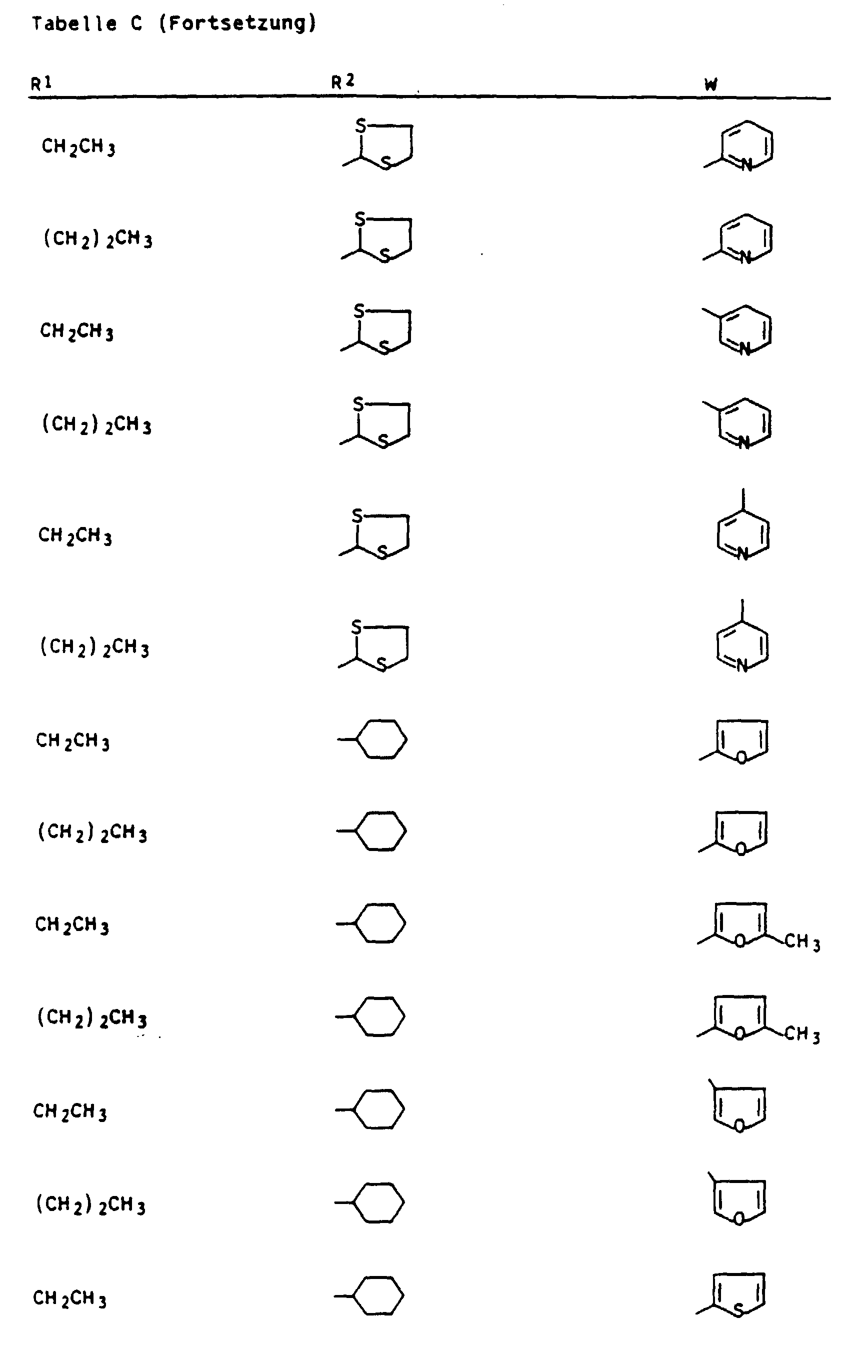

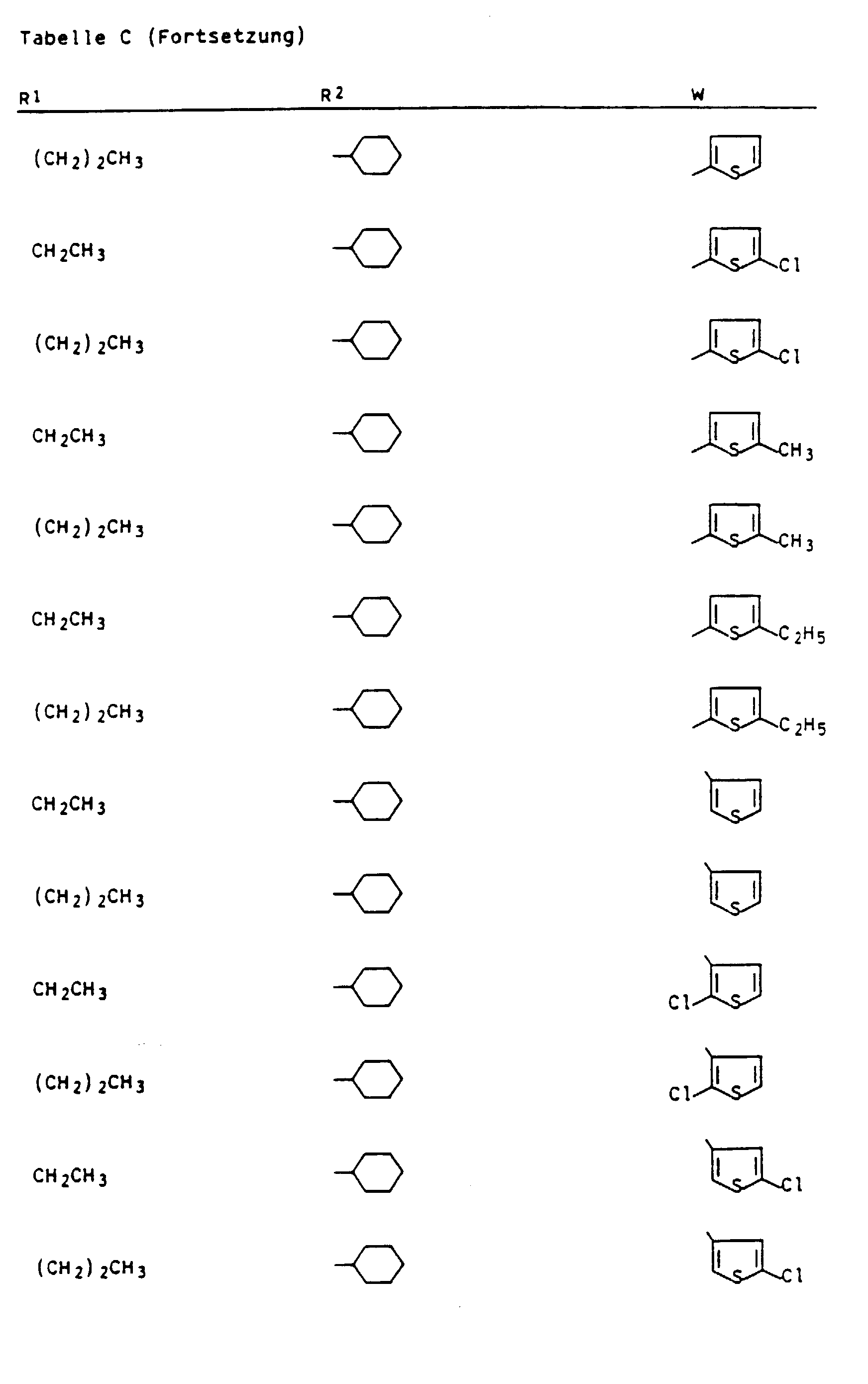

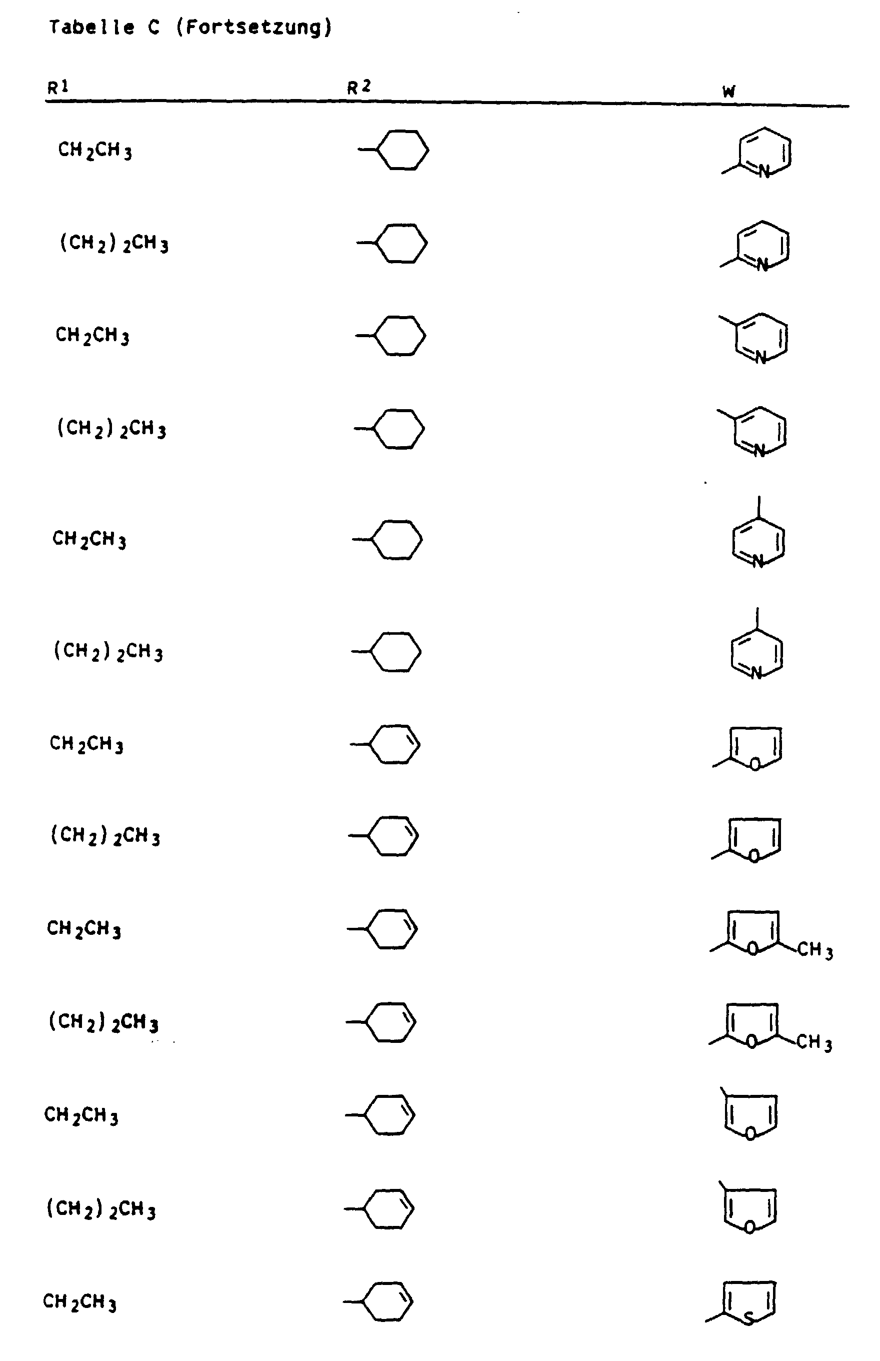

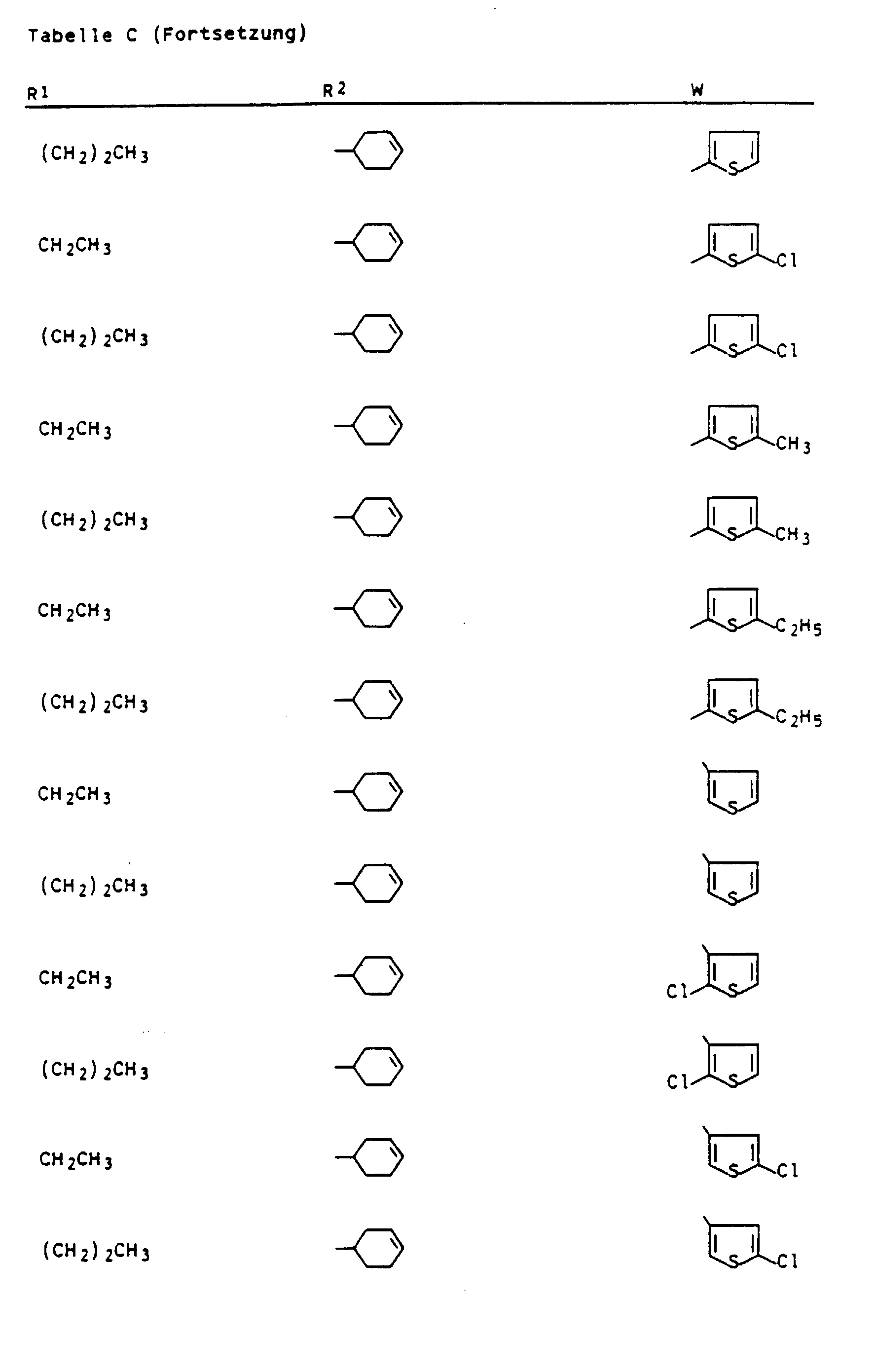

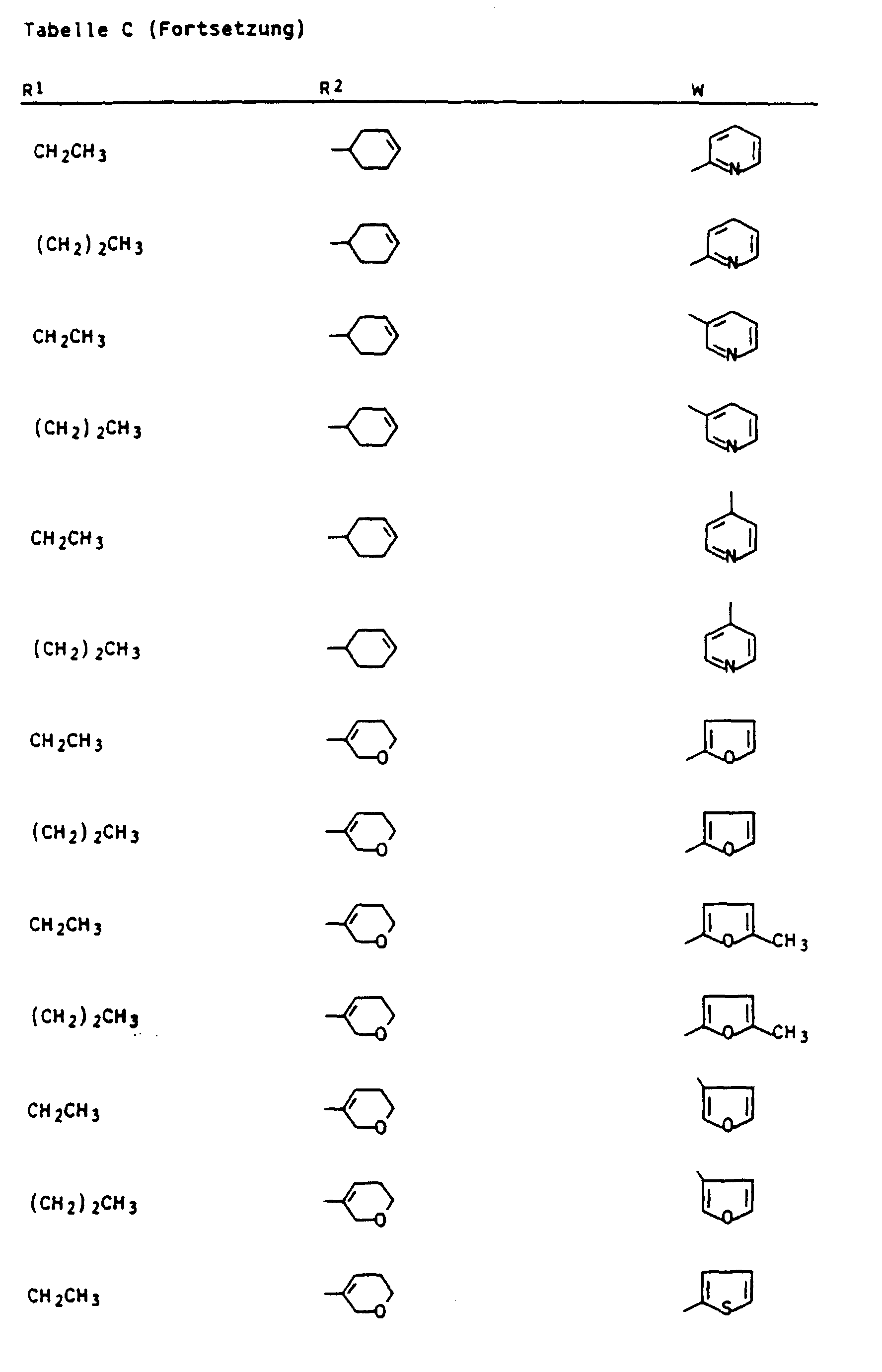

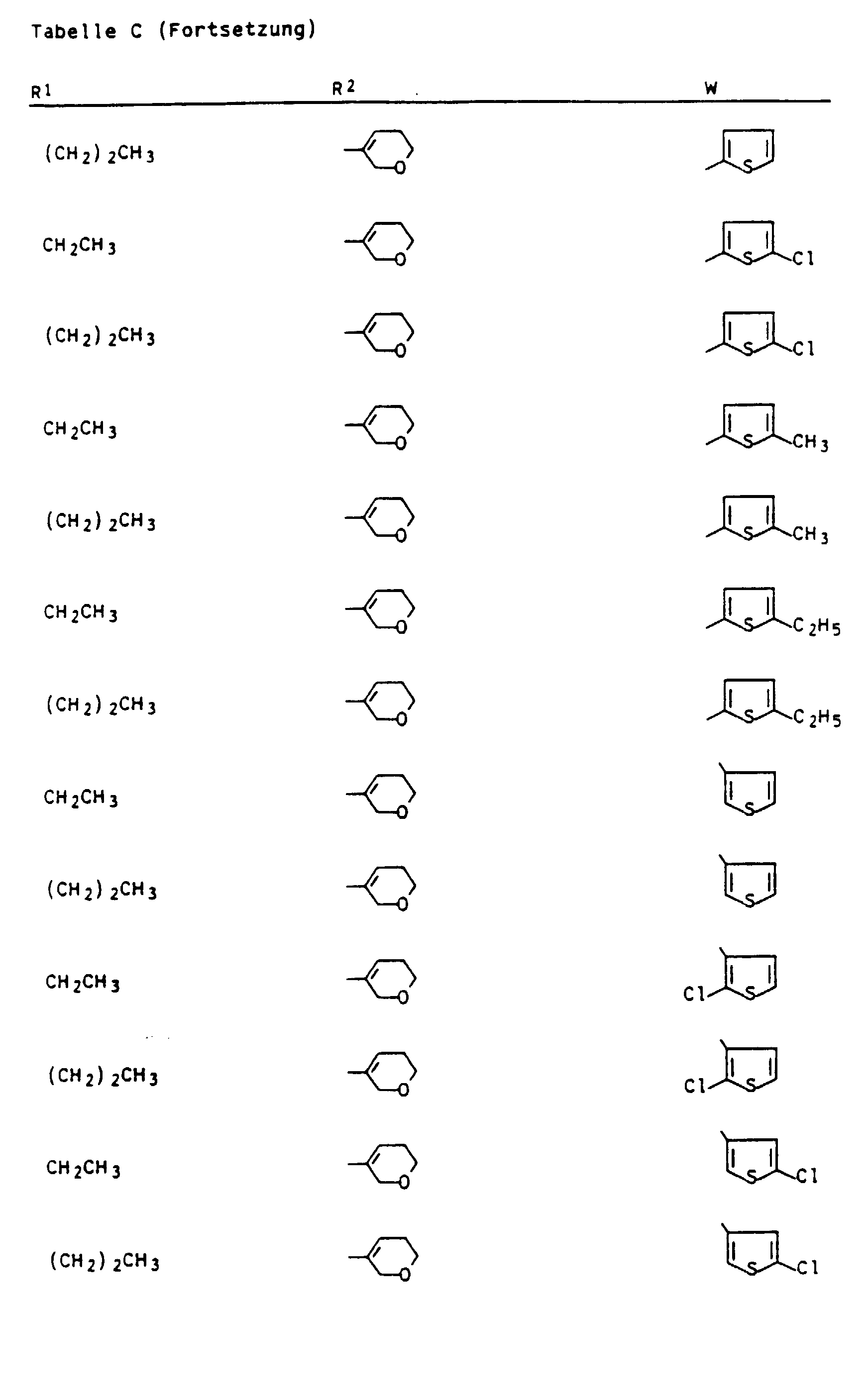

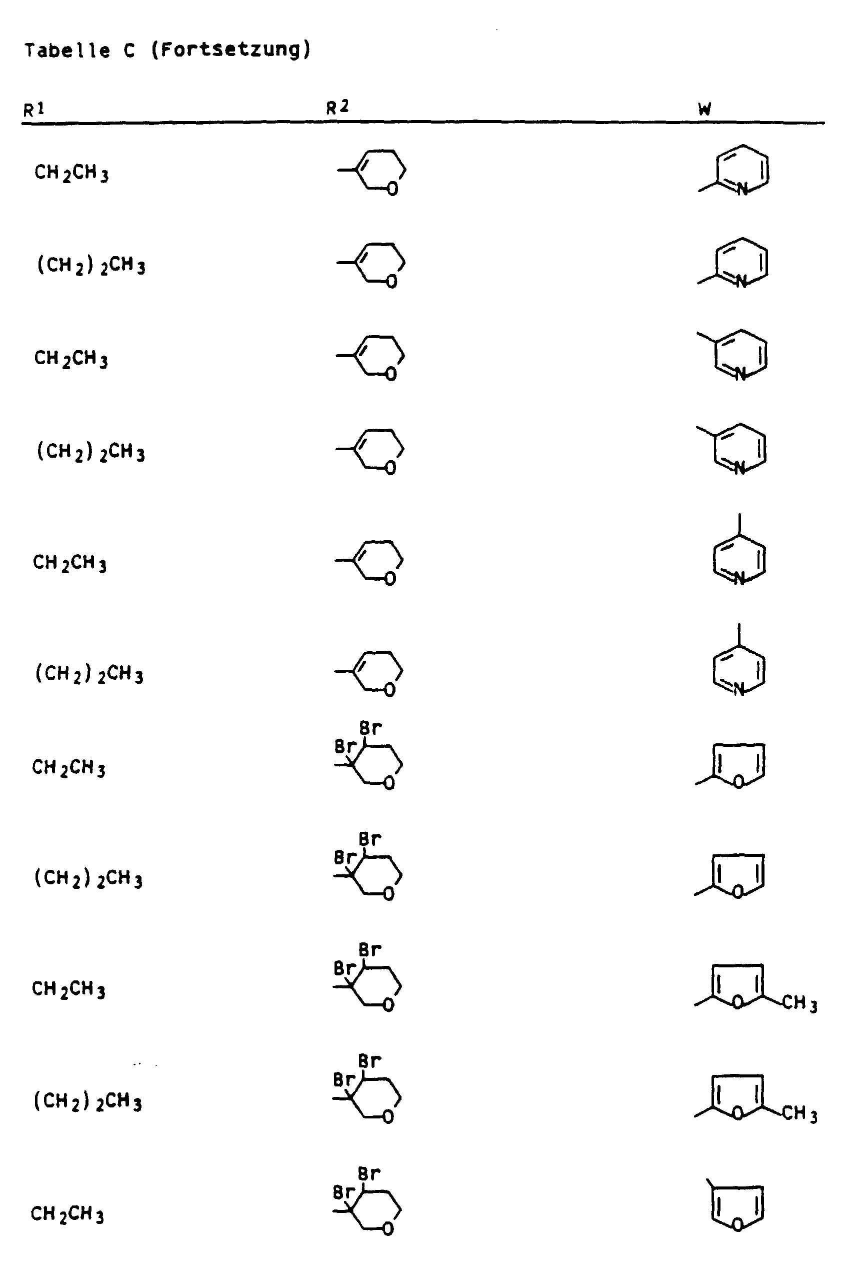

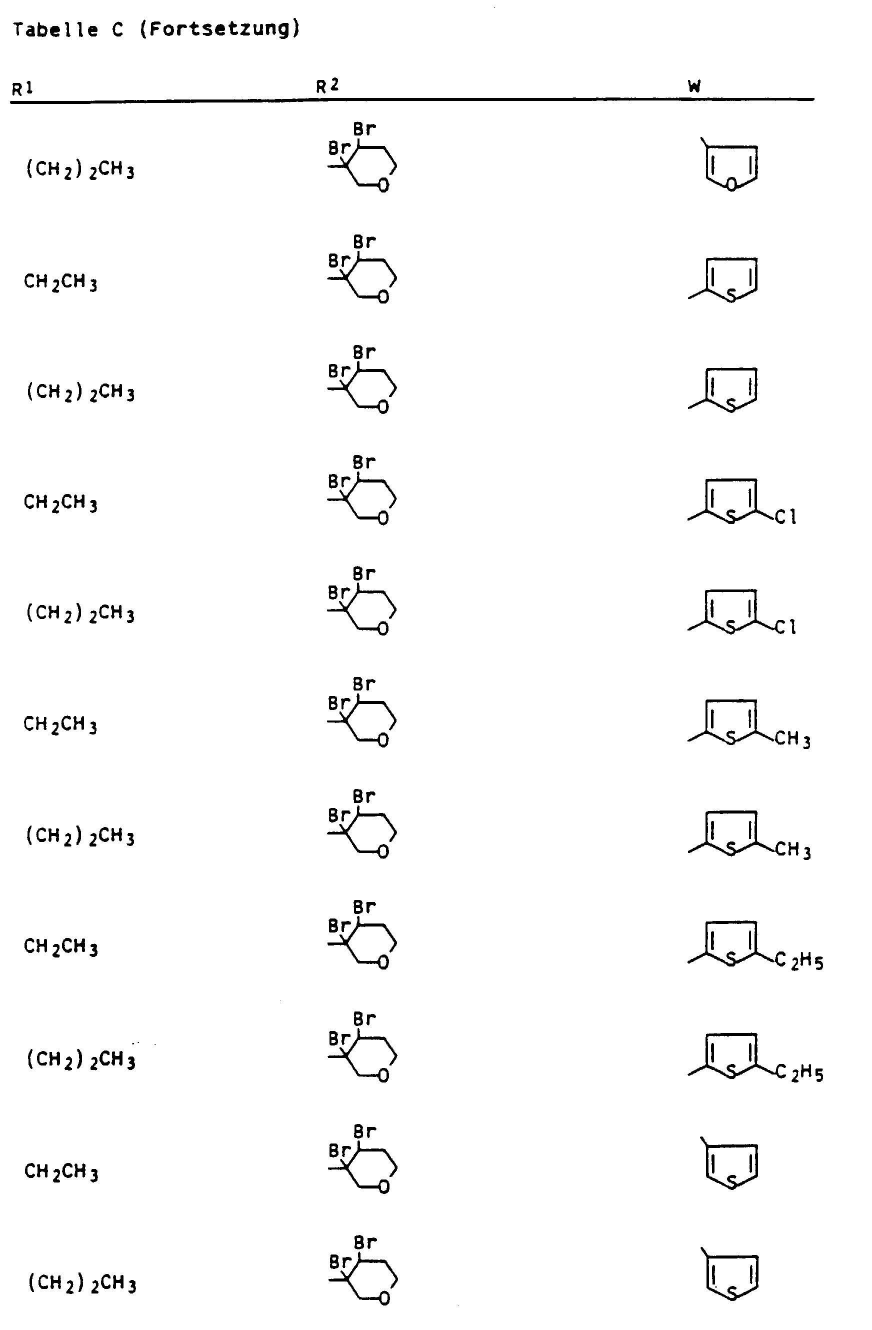

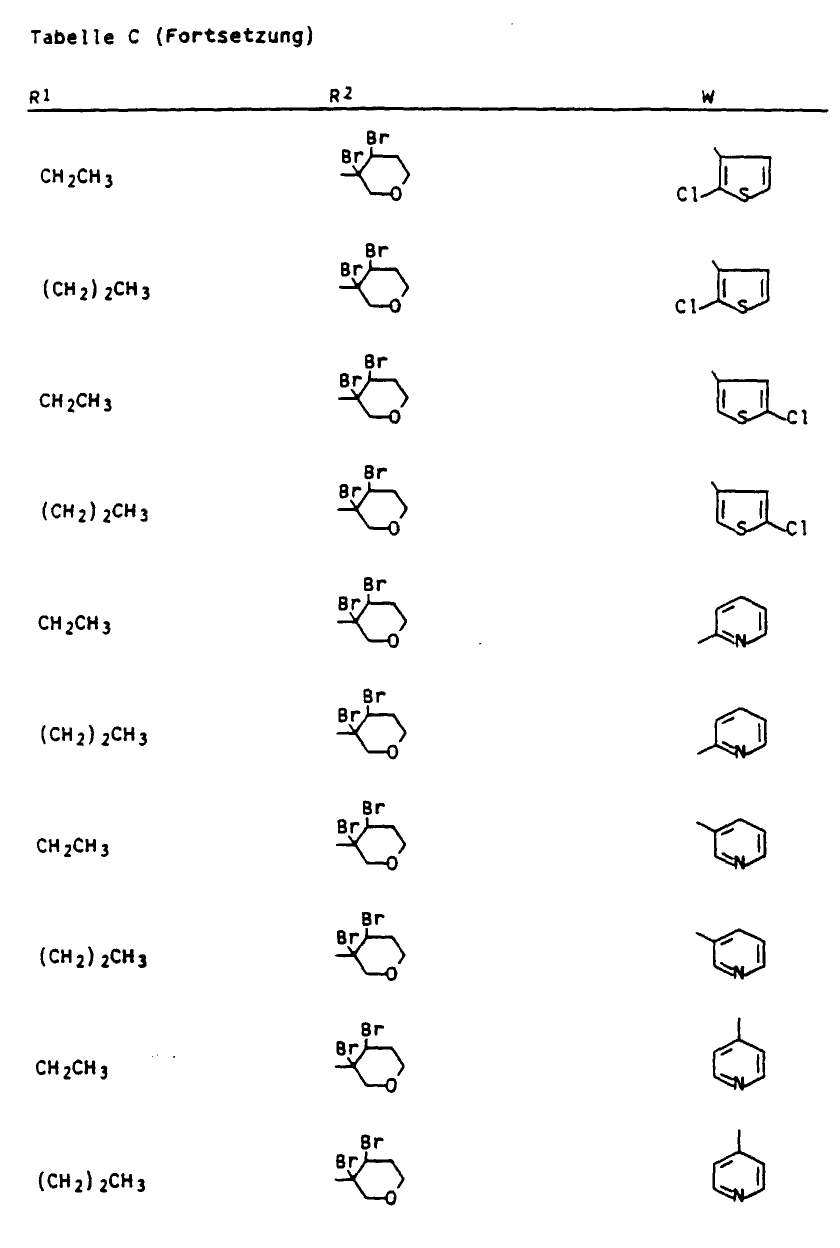

Die in Tabelle C aufgeführten Reste R¹, R² und W sind in analoger weise für die Cyclohexenonoximether der folgenden Strukturtypen D und E 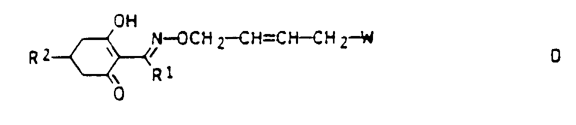 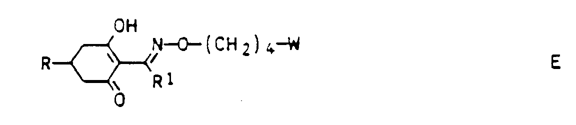 einzusetzen (W = Z-Xₙ).

Die entsprechenden Hydroxylamine III sind ebenfalls bevorzugt.

Die Cyclohexenonoximether I eignen sich als Herbizide, insbesondere zur Bekämpfung von Pflanzen aus der Familie der Gramineen (Gräser).

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Oispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen alkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nupschalenmehl, Cellulosepulver oder andere feste Trägerstoffe.

Die Formulierungen enthalten zwischen 0,02 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 8.01 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 8.02 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 8.03 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 8.04 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 8.05 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr. 8.06 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 8.07 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 8.08 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Mittel kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 3 kg/ha, vorzugsweise 0,01 bis 2,0 kg/ha.

In Anbetracht des erfaßbaren Wirkungsspektrums zur Unkrautbekämpfung, der Verträglichkeit für Kulturpflanzen oder der erwünschten Beeinflussung des wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| HumuluS lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| PrunuS avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (v. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Sulfonylharnstoffderivate, Cyclohexenone, (Hetero)-aryloxypropionsäuren, deren Salze, Ester und Amide und andere in Betracht.

Außerdem kann es von Nutzen sein, die Cyclohexenonderivate der Formel I bzw. sie enthaltende herbizide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die im nachstehenden Synthesebeispiel wiedergegebene Vorschrift wurde unter entsprechender Abwandlung der Ausgangsverbindung zur Gewinnung weiterer Hydroxylamine der Formel III und Cyclohexenonoximether der Formel I benutzt; die erhaltenen Verbindungen sind in den nachfolgenden Tabellen mit physikalischen Angaben aufgeführt.

### Synthesebeispiel zur Herstellung der Hydroxylamine III

### (E)-4-Brom-1-(2-thienyl)-1-buten

Bei 5-100C tropfte man innerhalb 1 h 225 g (1.46 mol) Cyclopropyl-2-thienylcarbinol zu 972 ml 48%iger Bromwasserstoffsäure. Nach 2 h bei Raumtemperatur wurde die organische Phase abgetrennt und die wäßrige Lösung dreimal mit je 300 ml Dichlormethan extrahiert. Die vereinigten organischen Phasen wurden mit verd. Natronlauge und Wasser neutral gewaschen, über Magnesiumsulfat getrocknet und im Vakuum eingeengt. 322 g (94% korrigiert) rohes Bromid (GC: 92%). ¹H-NMR (250 MHZ, CDCl₃): δ=2.65-2.80 (m, 2H), 3.46 (t,2H), 5.90-6.10 (m, 1H), 6.61 (d, 1H), 6.80-7.00 (m, 2H), 7.14 (d, 1H).

### N-[(E)-4-(2-Thienyl)-3-butenyloxy]phthalimid

Bei 20-25°C tropfte man innerhalb 2,5 h 190 ml (1.37 mol) Triethylamin zu einer Mischung aus 283 g (1.30 mol) des oben hergestellten Bromids, 1300 ml N-Methyl-2-pyrrolidinon, 10 g Kaliumiodid und 212 9 (1.30 mol) N-Hydroxyphthalimid. Nach 4 h bei 20-25°C goß man in 4000 ml Eiswasser und ergänzte portionsweise 500 ml 10%ige Natronlauge. Man extrahierte darauf viermal mit je 500 ml Essigester. Die vereinigten Essigester-Phasen wurden mit verd. Natronlauge und Wasser neutral gewaschen, über Magnesiumsulfat getrocknet und im Vakuum eingeengt. Das Rohprodukt wurde chromatographisch an 1000 g Kieselgel/Säule 30x15 cm, (Laufmittel: n-Hexan/Dichlormethan 7:3) gereinigt. 113 g (29%) Phthalimidether, Fp 69-71°C (Isopropanol). ¹H-NMR (250 MHZ, d₆-DMSO): δ=2.55-2.70 (m, 2H), 4.28 (t, 2H), 6.00-6.20 (m, 1H), 6.77 (d, 1H), 7.00 (m, 2H), 7.35 (m, 1H), 7.87 (s, 4H).

### O-[(E)-4-(2-Thienyl)-3-butenyl]hydroxylamin

Eine Mischung aus 90.2 g (0.30 mol) des oben hergestellten Phthalimidethers und 136 ml Ethanolamin wurden 3 h bei 60°C gerührt. Die kalte Reaktionsmischung goß man in 200 ml Eiswasser. Man ergänzte 200 ml ges. Kochsalz-Lösung und extrahierte das Hydrolysat dreimal mit je 300 ml Dichlormethan. Die vereinigten organischen Phasen wurden darauf dreimal mit je 100 ml ges. Kochsalz-Lösung gewaschen, über Magnesiumsulfat getrocknet und im Vakuum eingeengt. 45 g (89%) Hydroxylamin. ¹H-NMR(250 MHz, CDCl₃): δ=2.40-2.55 (m, 2H), 3.78 (t, 2H), 5.40 (bs, 2H), 5.95-6.20 (m, 1H), 6.57 (d, 1H), 6.80-7.15 (m, 3H).

### Synthesebeispiel zur Herstellung der Cyclohexenonoximether-Derivate I

### 2-[1-[[(E)-4-(2-Thienyl)-3-butenyloxy]imino]-butyl]-3-hydroxy-5-(2H-tetrahydropyran-4-yl)-2-cyclohexen-1-on

Eine Mischung aus 35 g (0.13 mol) 2-Butyryl-3-hydroxy-5-(2H-tetrahydropyran-4-yl)-2-cyclohexen-1-on und 24 g (0.14 mol) O-[(E)-4-(2-Thienyl)-3-butenyl]hydroxylamin in 300 ml Methanol wurde 16 h gerührt. Man engte im Vakuum ein und nahm in 1000 ml 10%iger Natronlauge auf. Man extrahierte dreimal mit je 200 ml Methylenchlorid und stellte die wäßrige Phase unter Eiskühlung mit konz. Salzsäure auf pH 1 ein. Die wäßrige Phase wurde anschließend dreimal mit je 200 ml Ether extrahiert, über Magnesiumsulfat getrocknet und im Vakuum eingeengt. Das Rohprodukt wurde chromatographisch an 1000 g Kieselgel/Säule 30x15 cm, (Laufmittel: Essigester) gereinigt. 46 g (85%) Cyclohexenonoximether. ¹H-NMR(200 MHz, CDCl₃): δ=0.95 (t, 3H), 1.17-1.96 (m, 9H), 2.13 (m, 1H), 2.36 (m, 1H), 2.43-2.70 (m, 3H), 2.88 (m, 2H), 3.36 (t, 2H), 4.02 (d, 2H), 4.15 (t, 2H), 6.00 (dt, 1H), 6.60 (d, 1H), 6.80-7.20 (m, 3H), 14.75 (s, 1H).

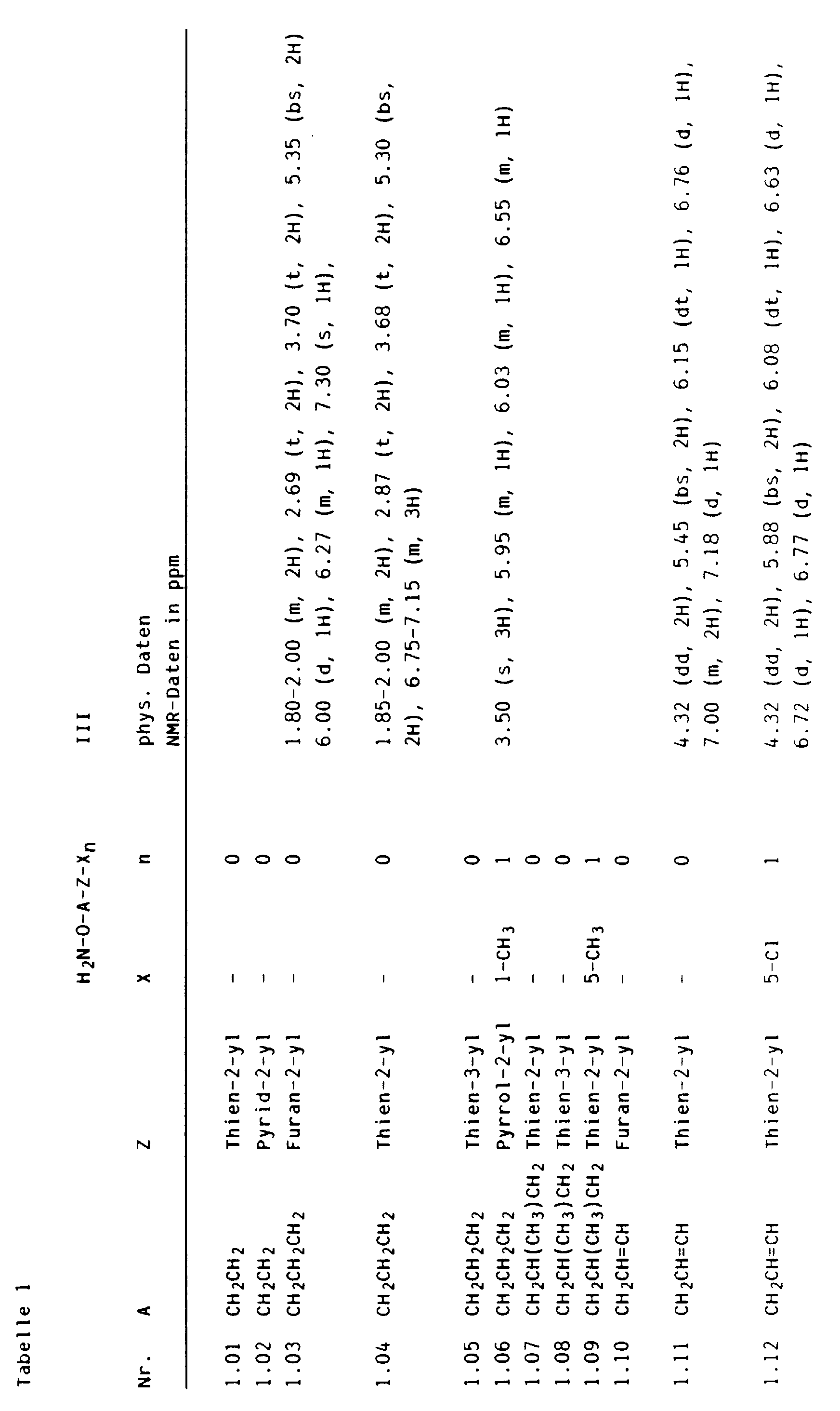

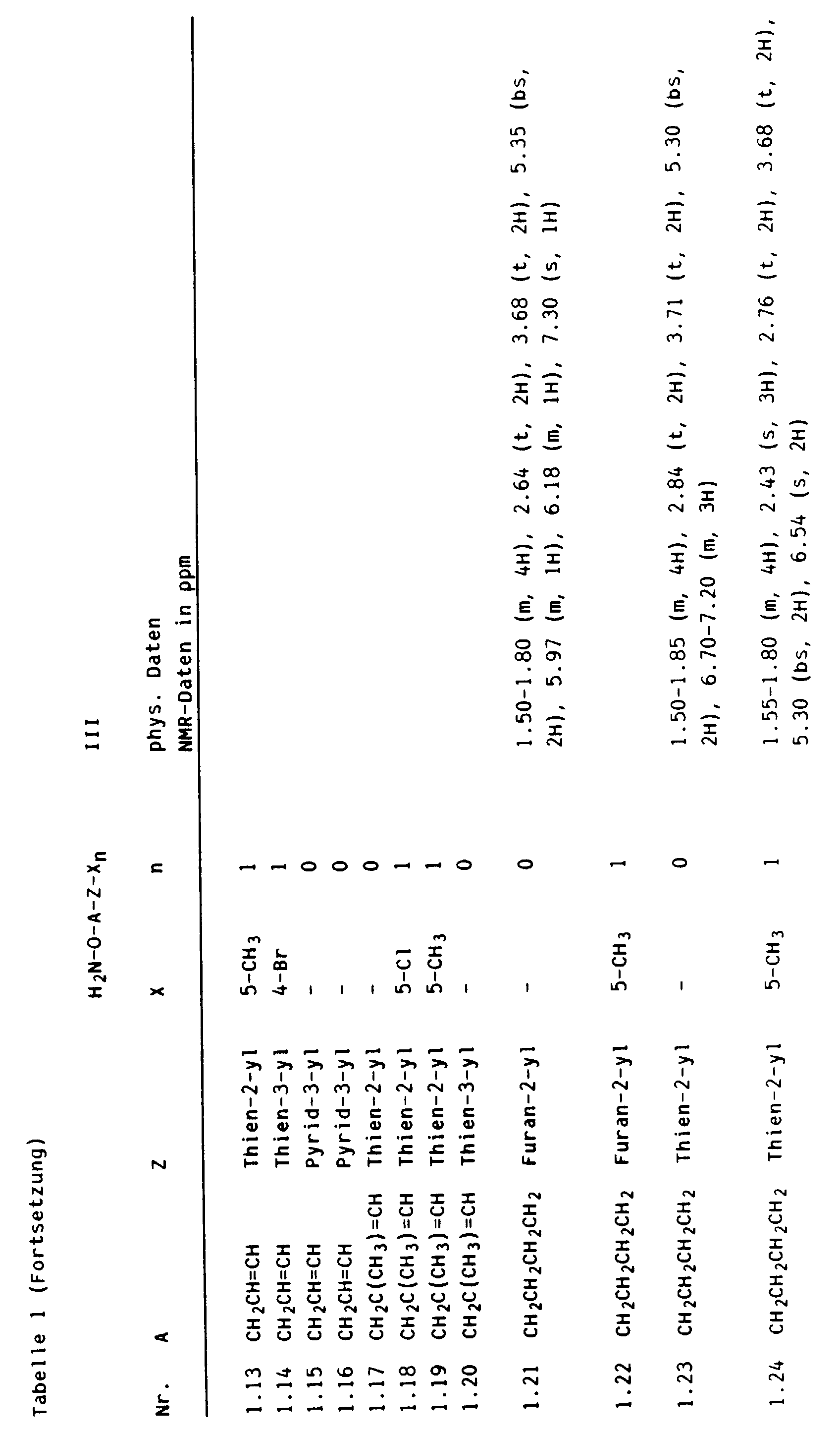

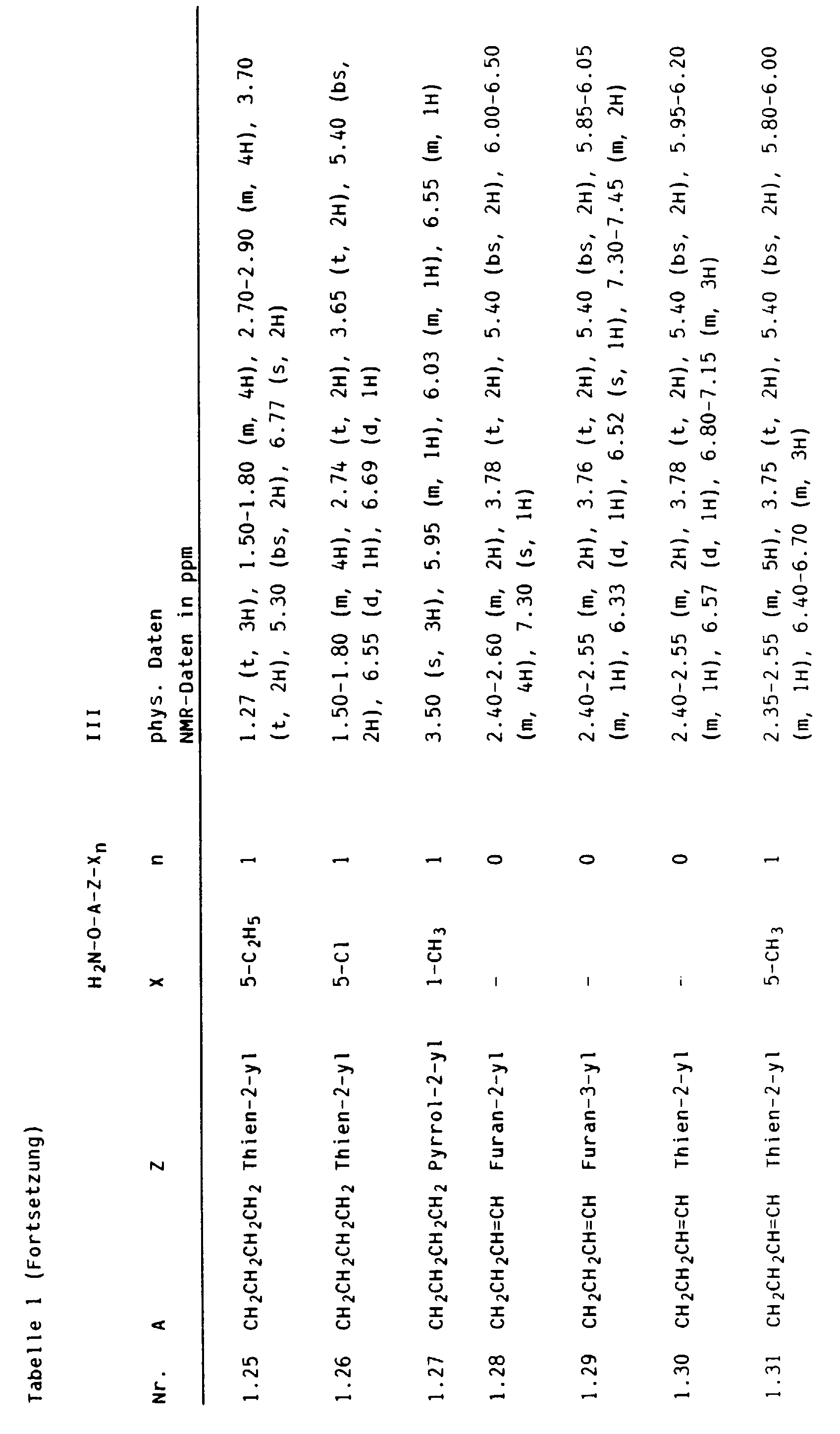

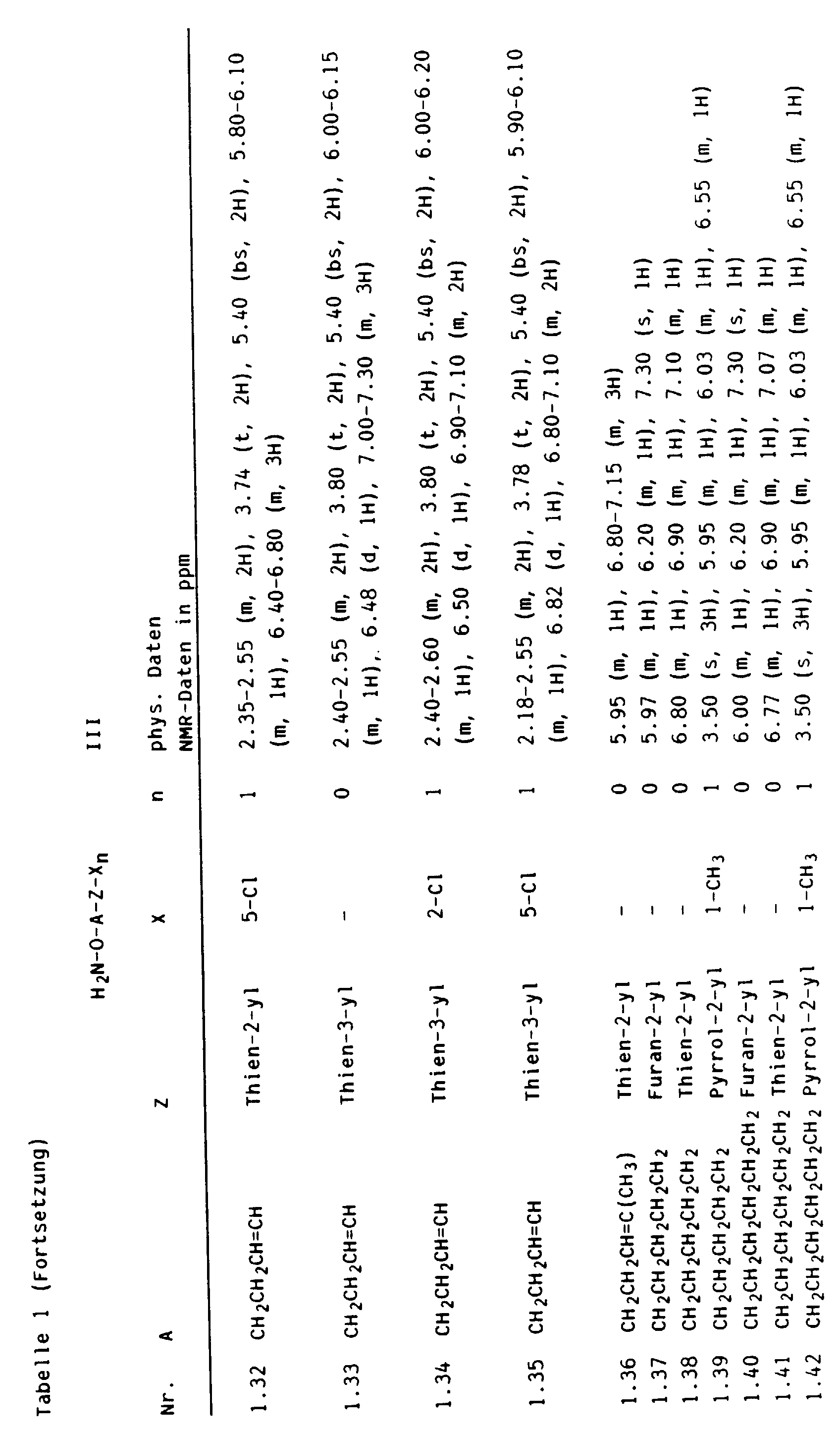

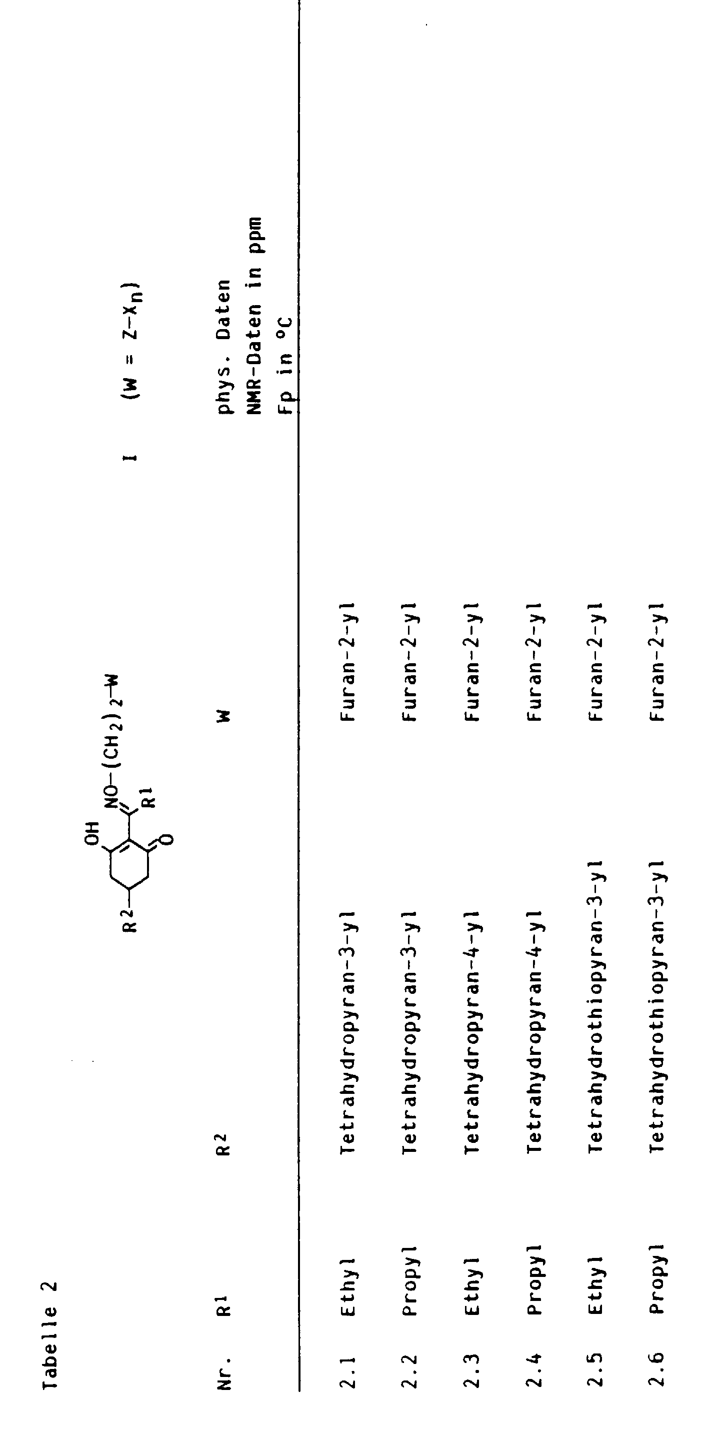

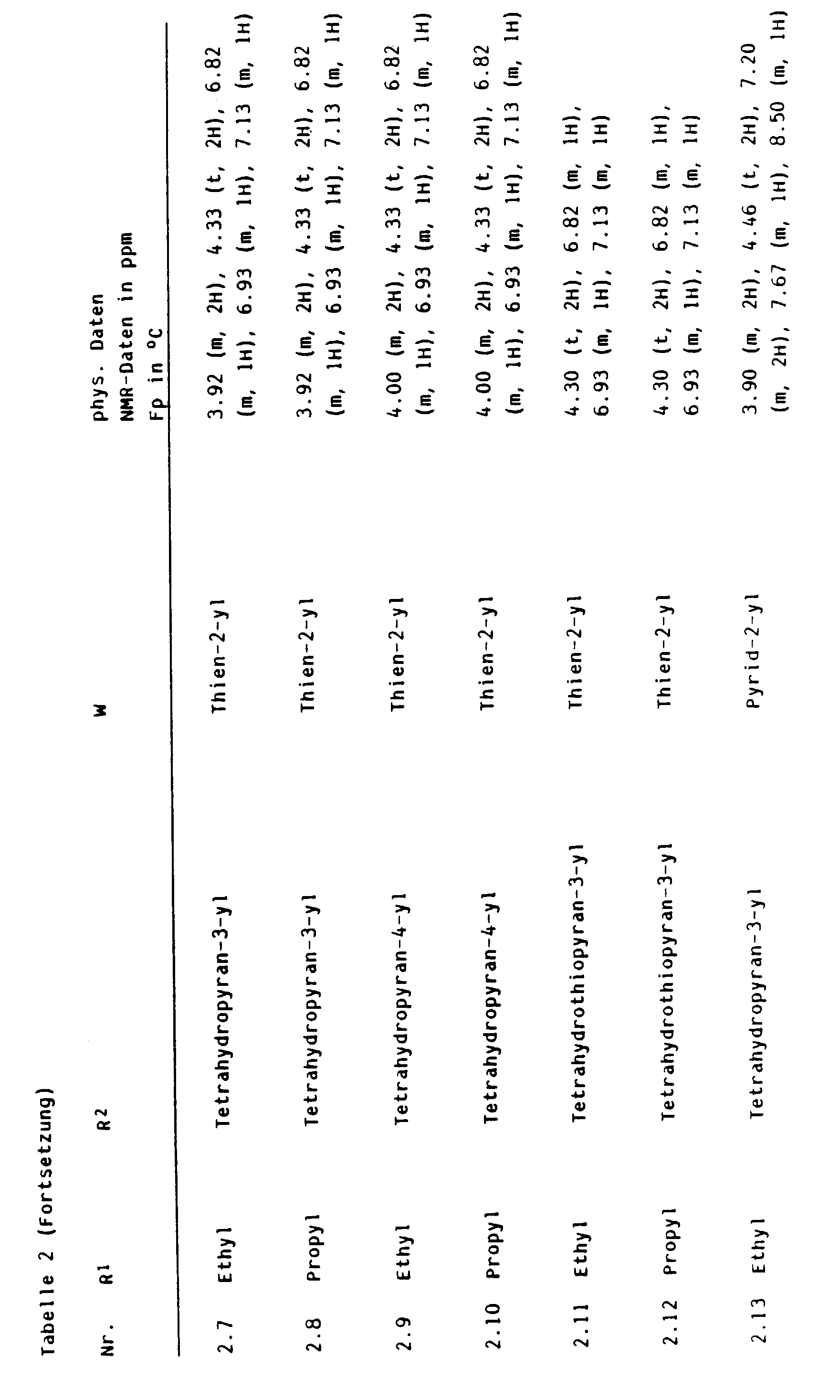

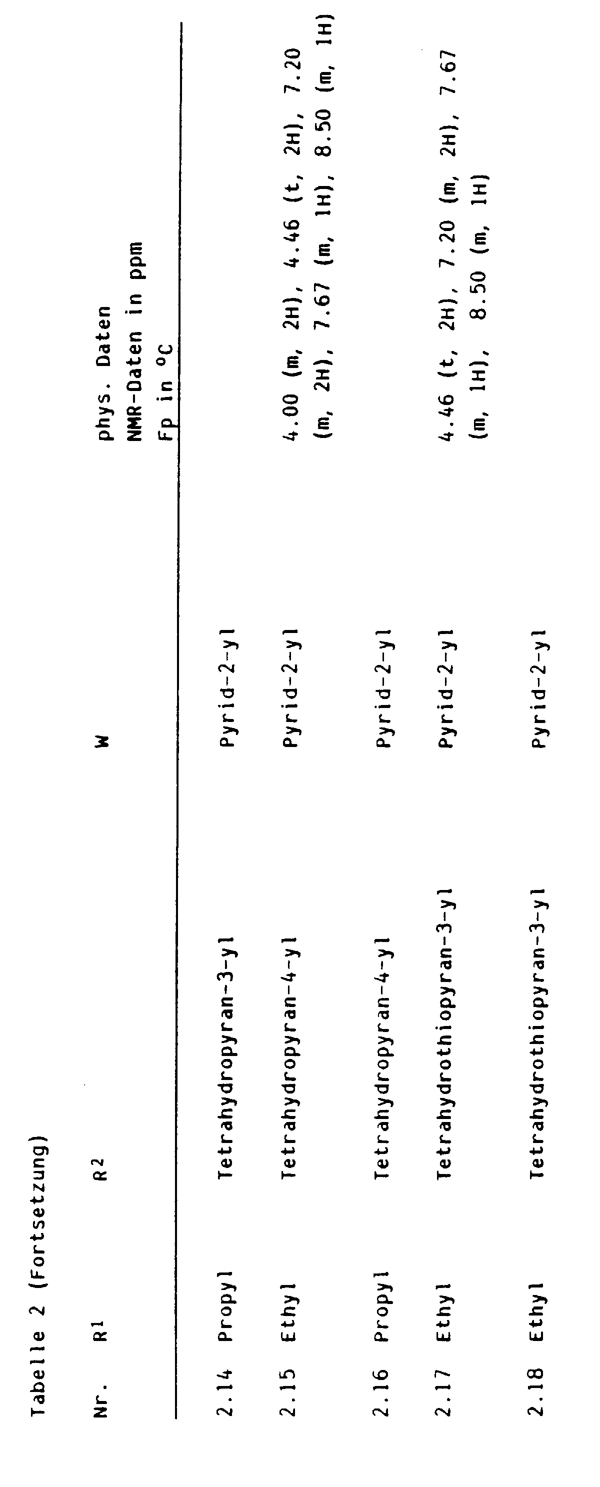

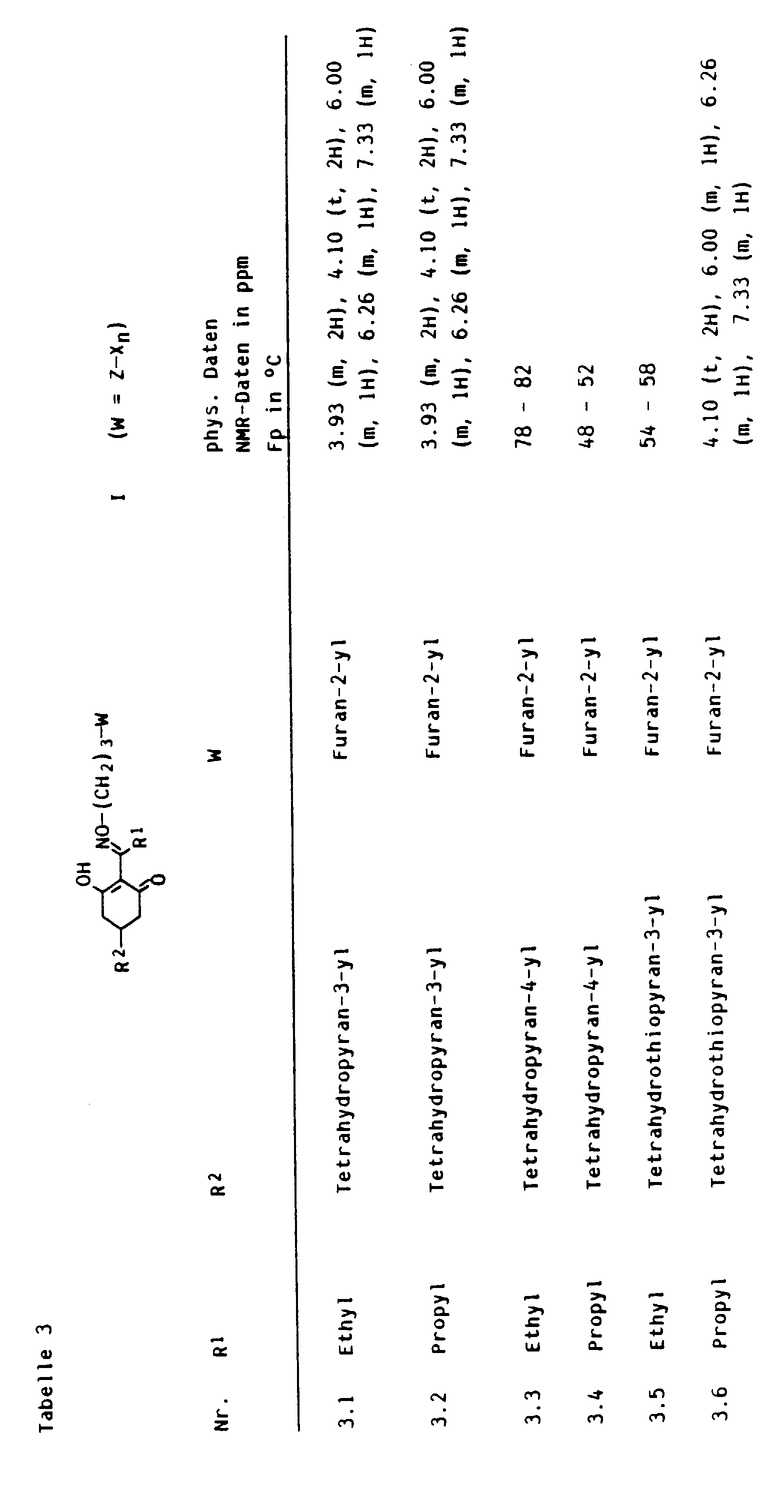

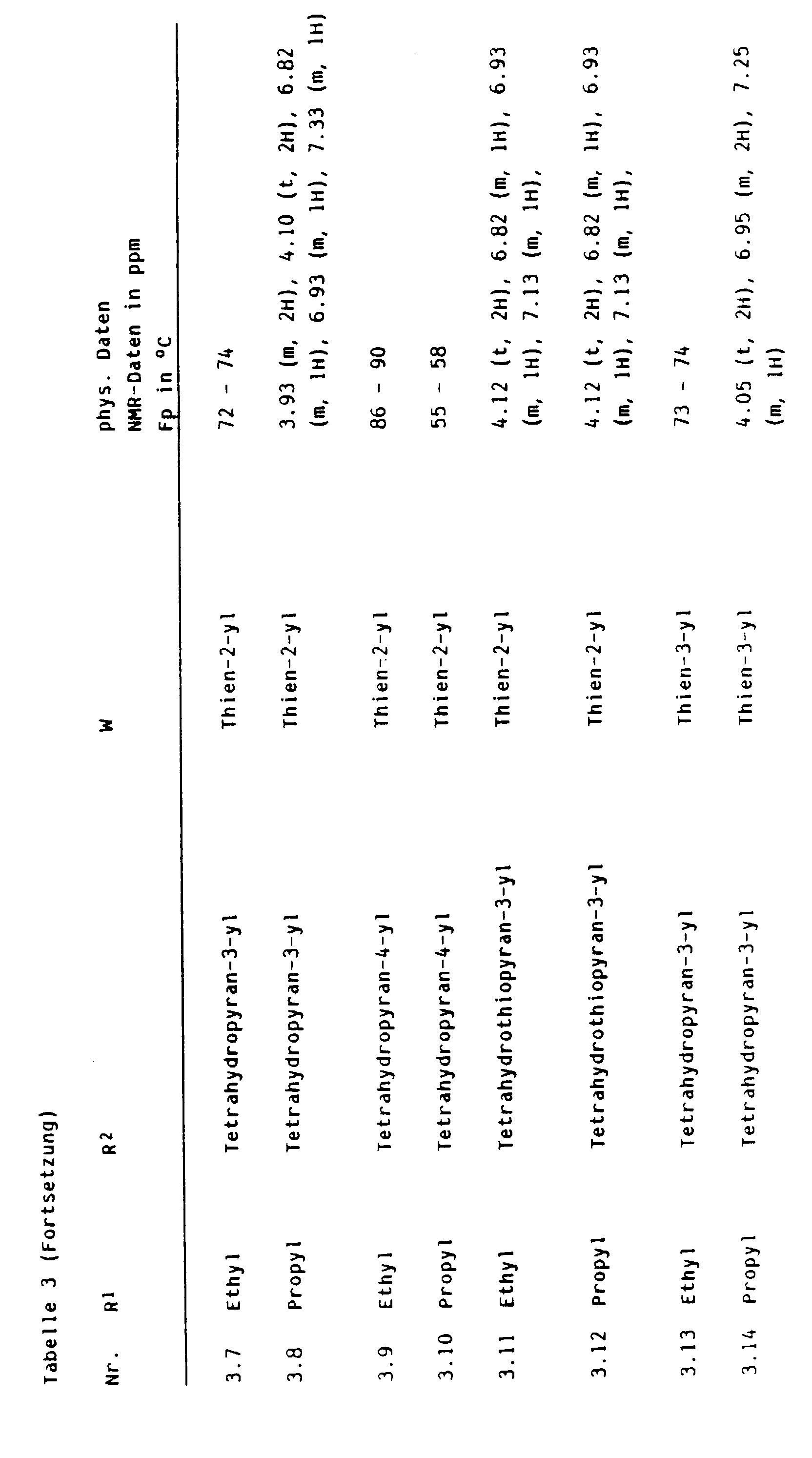

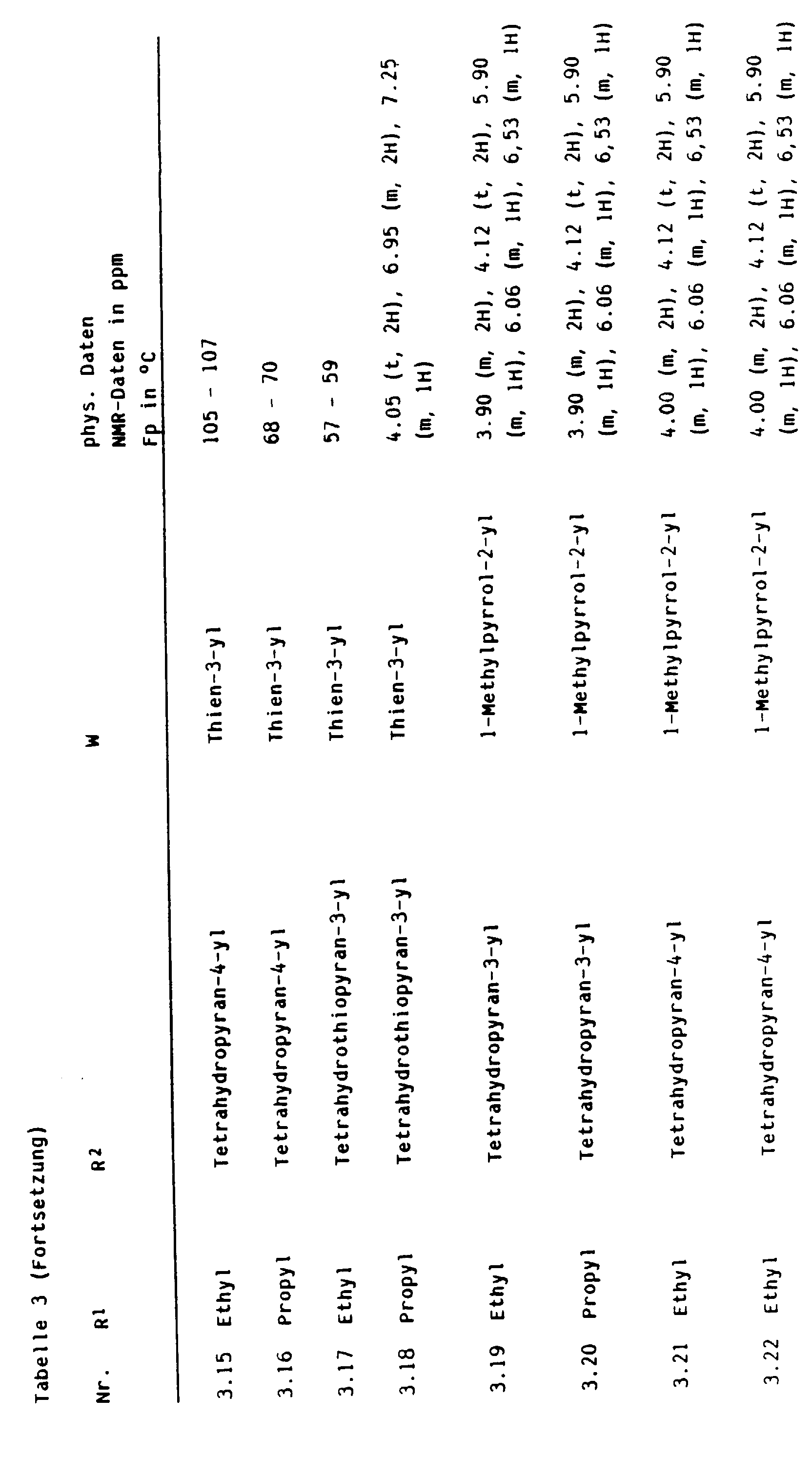

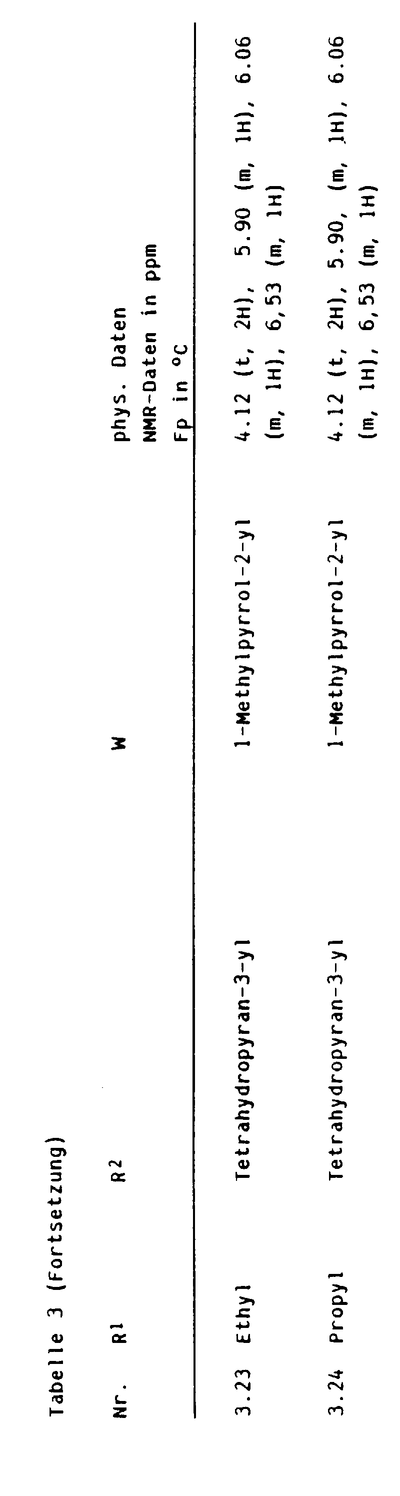

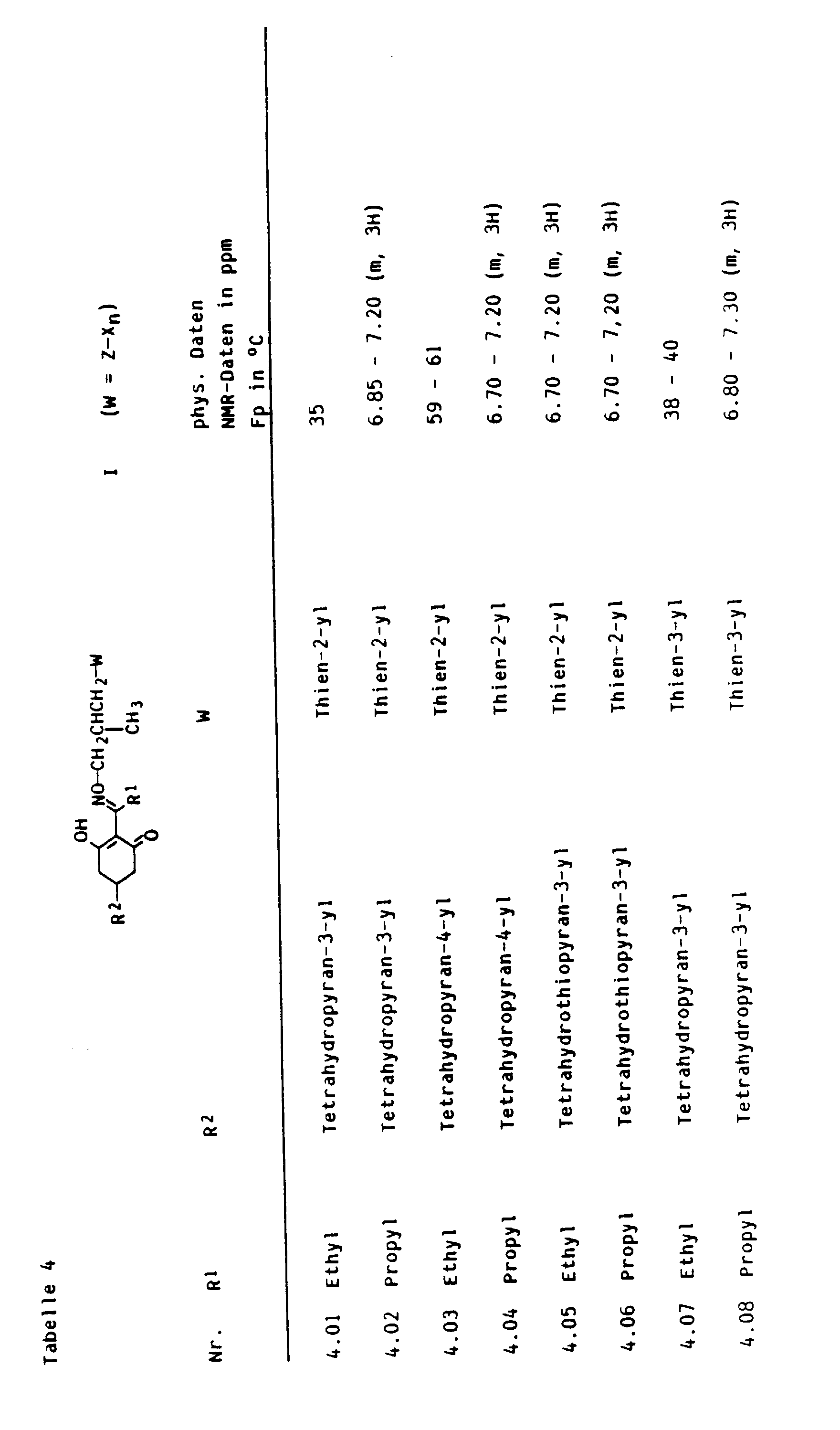

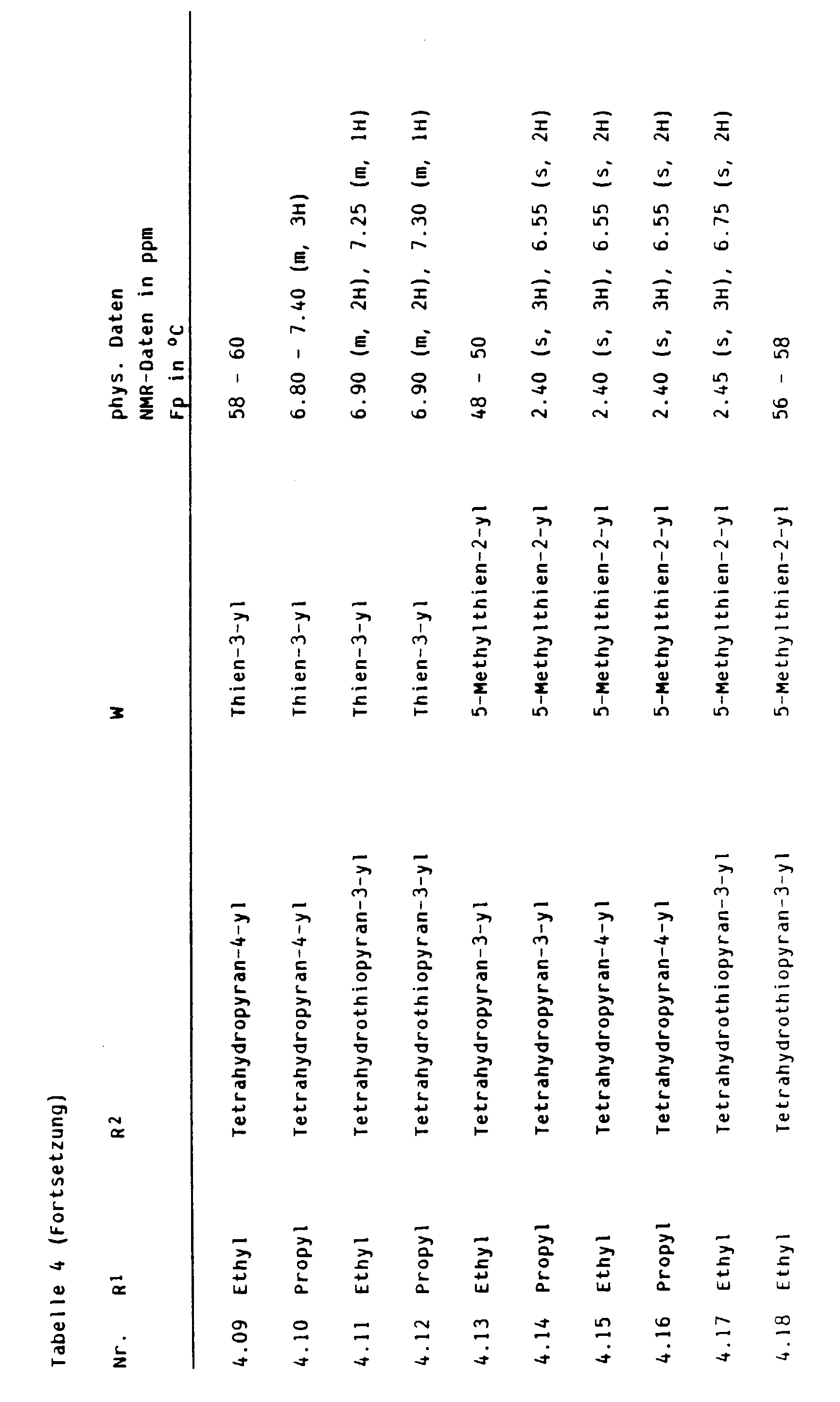

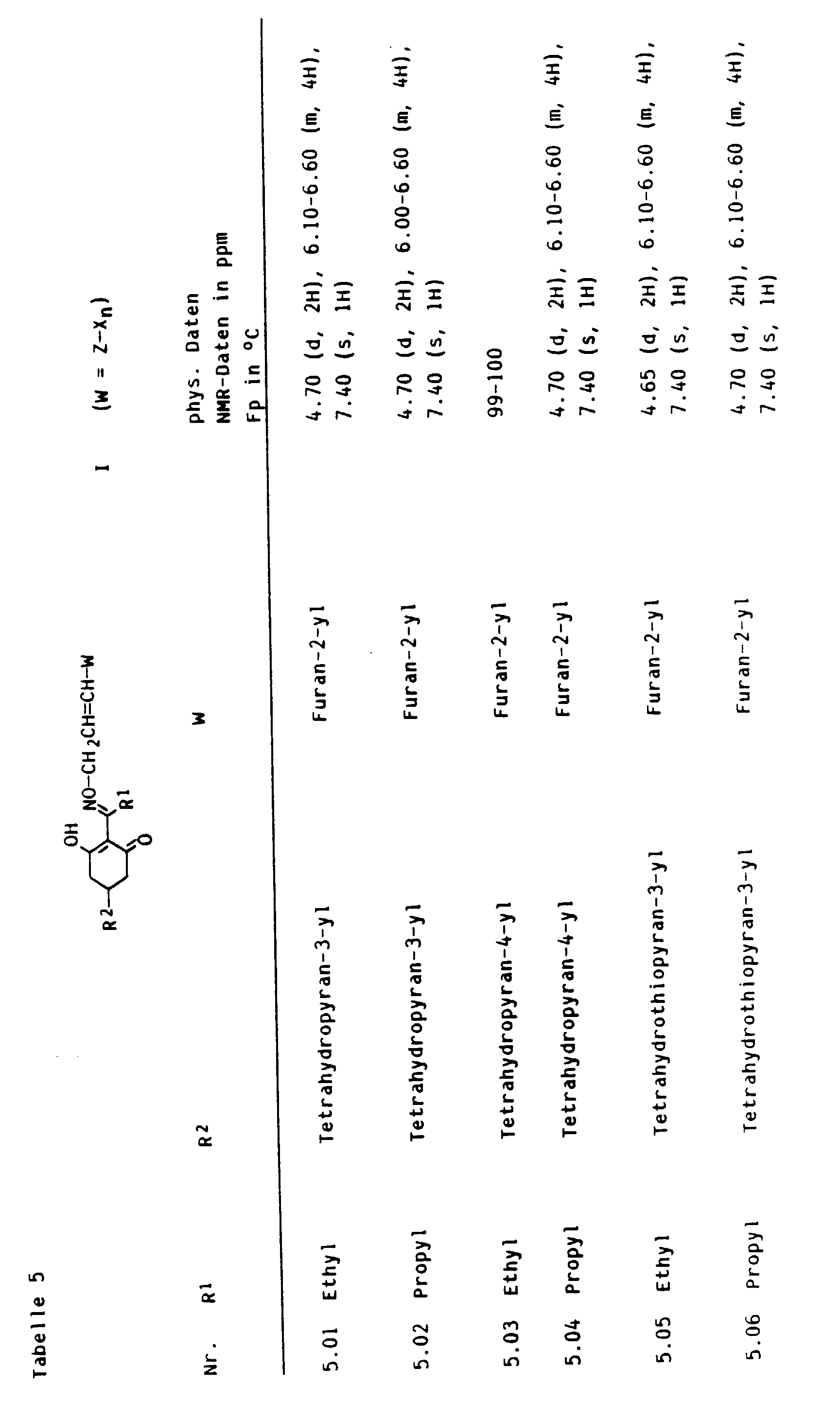

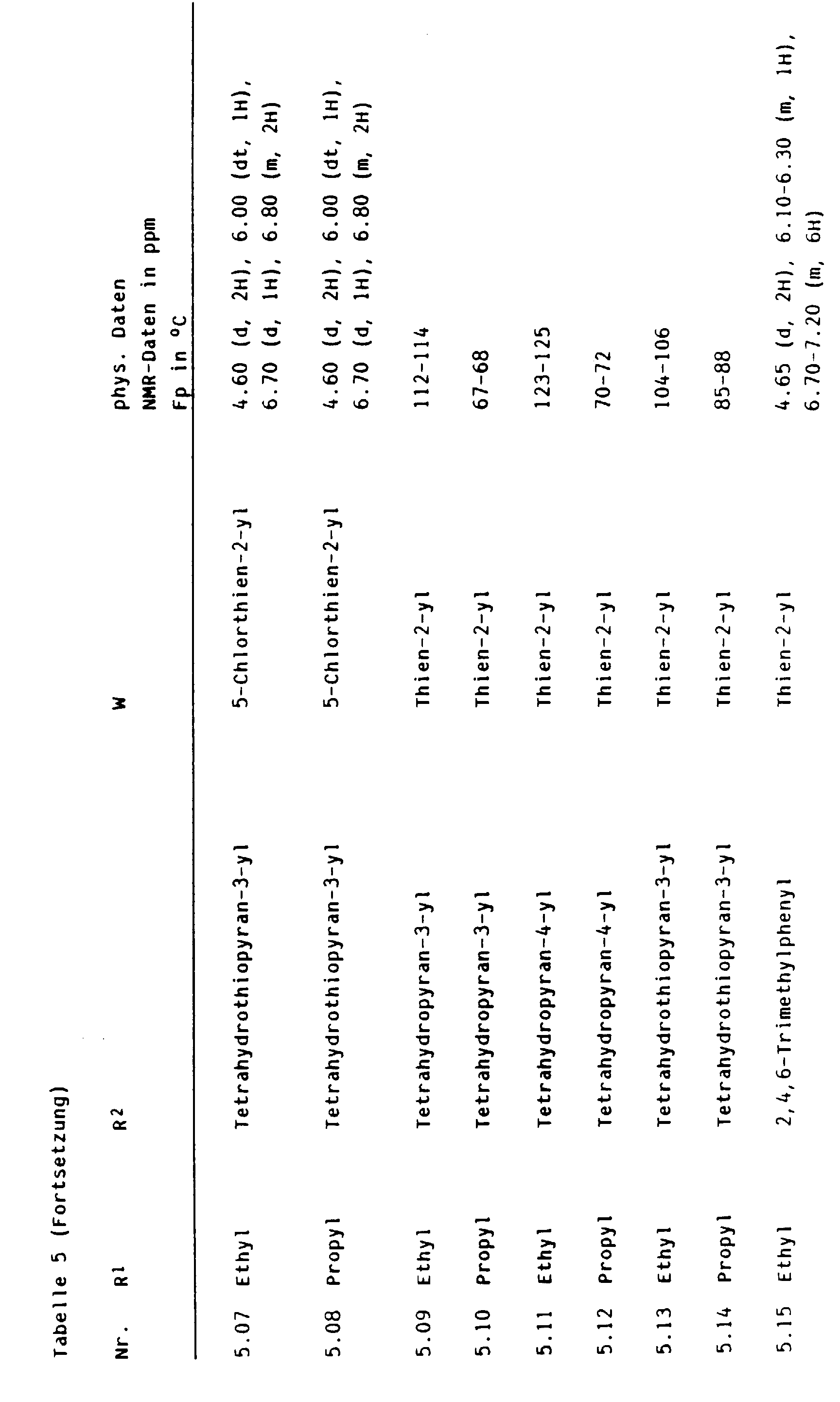

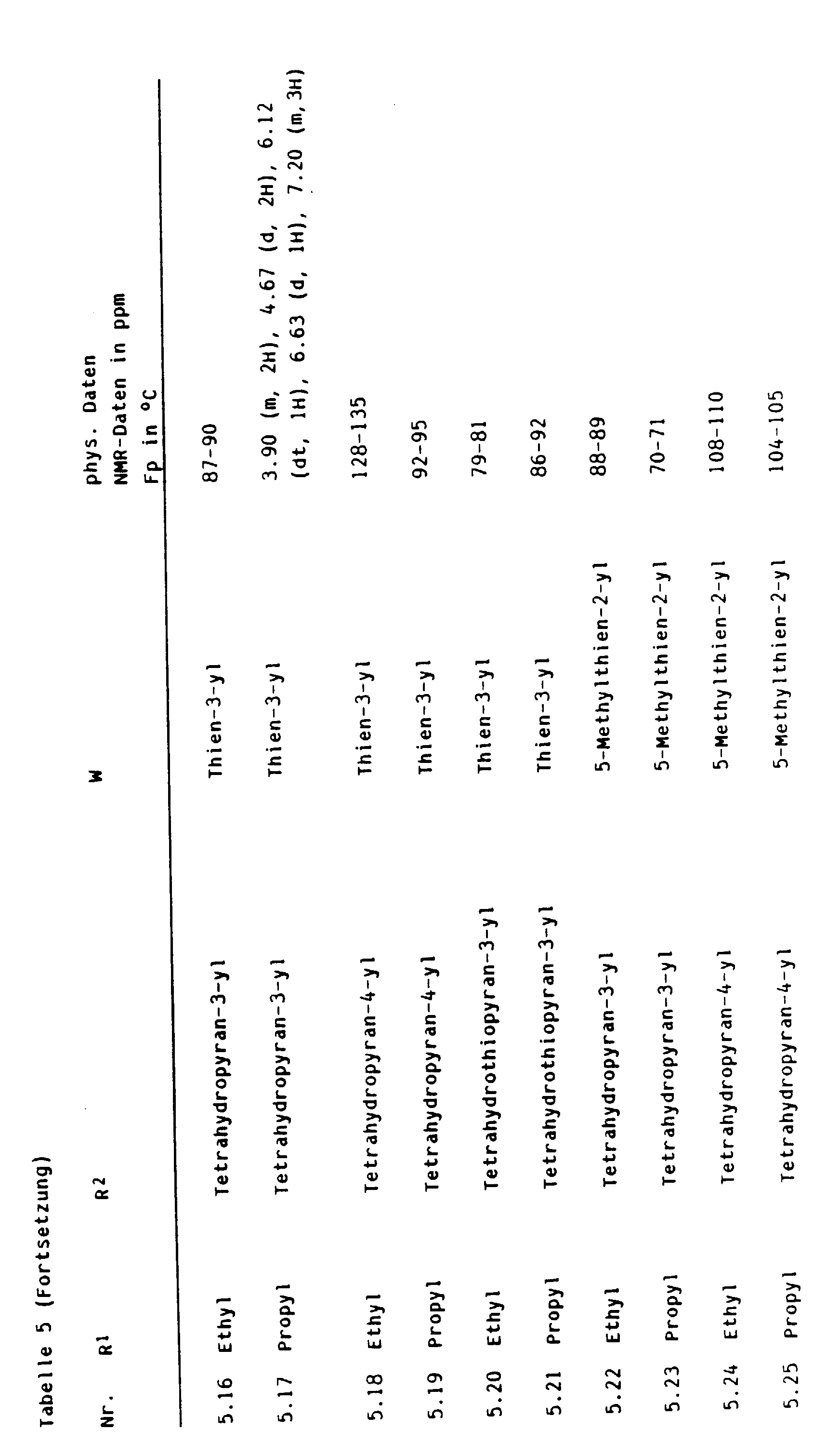

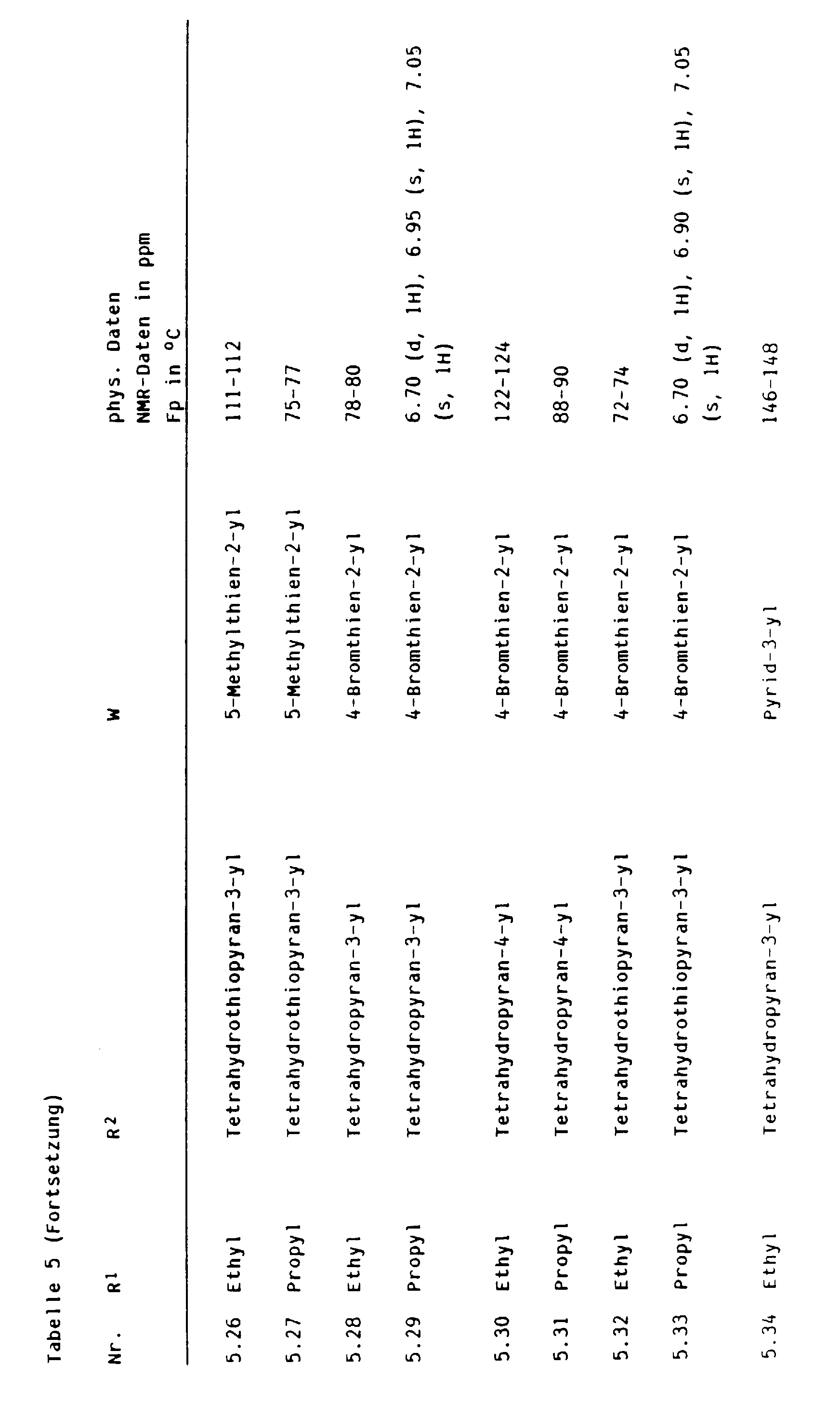

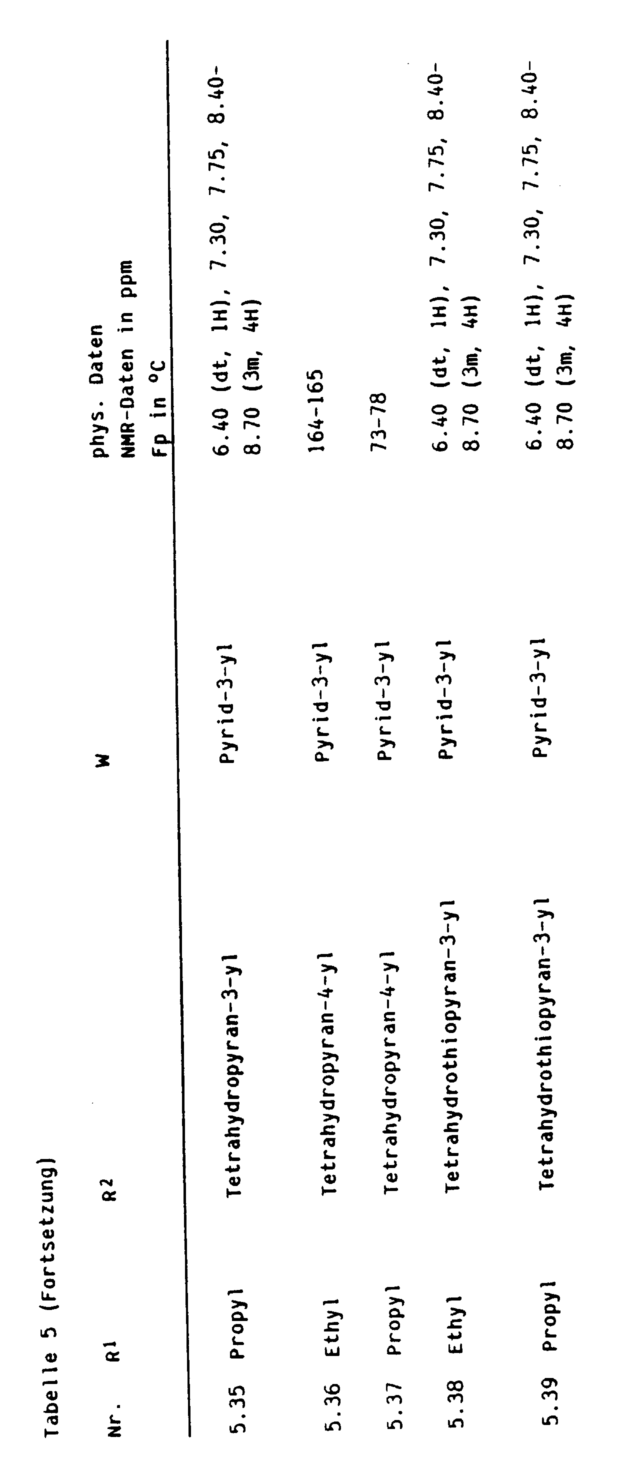

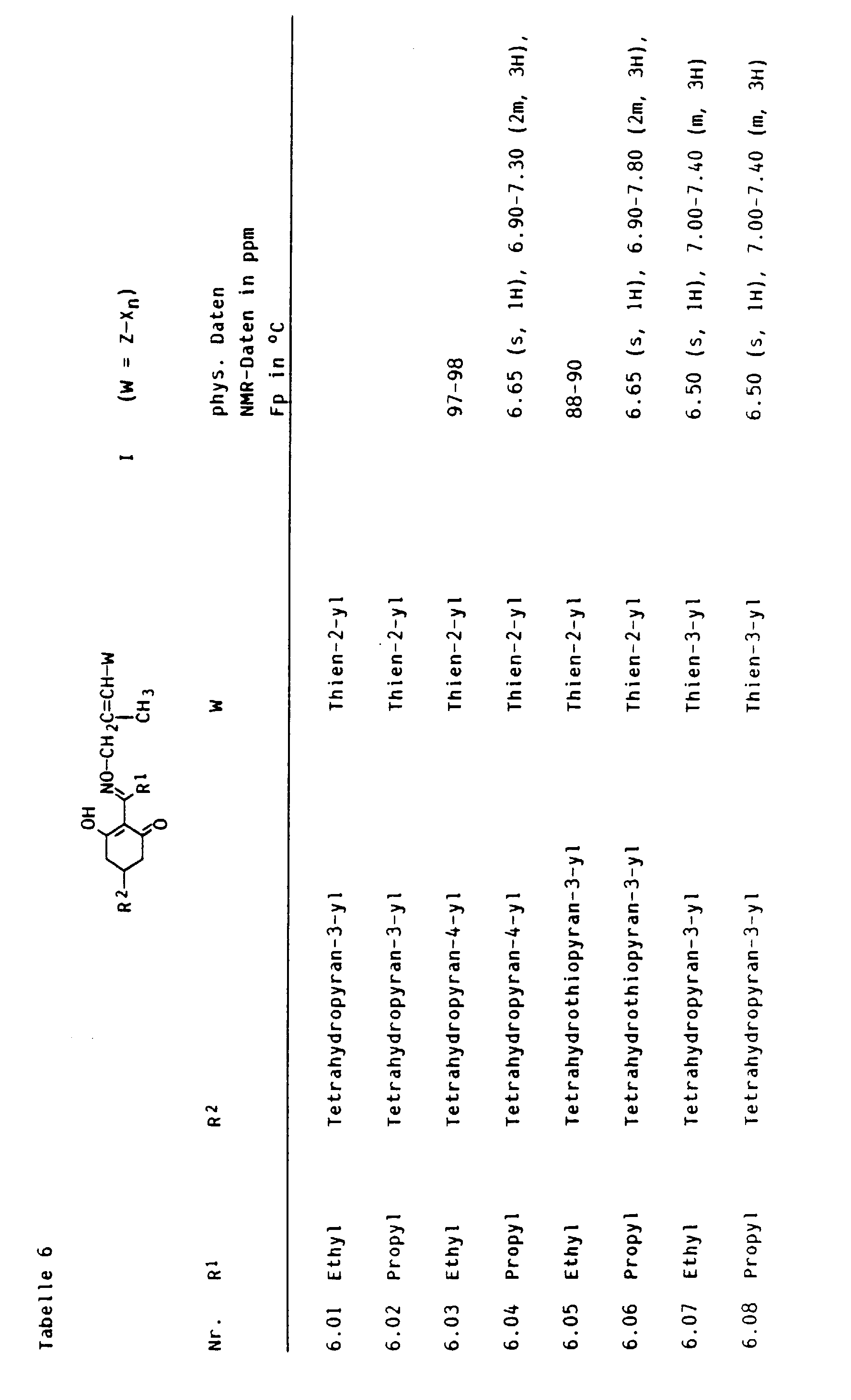

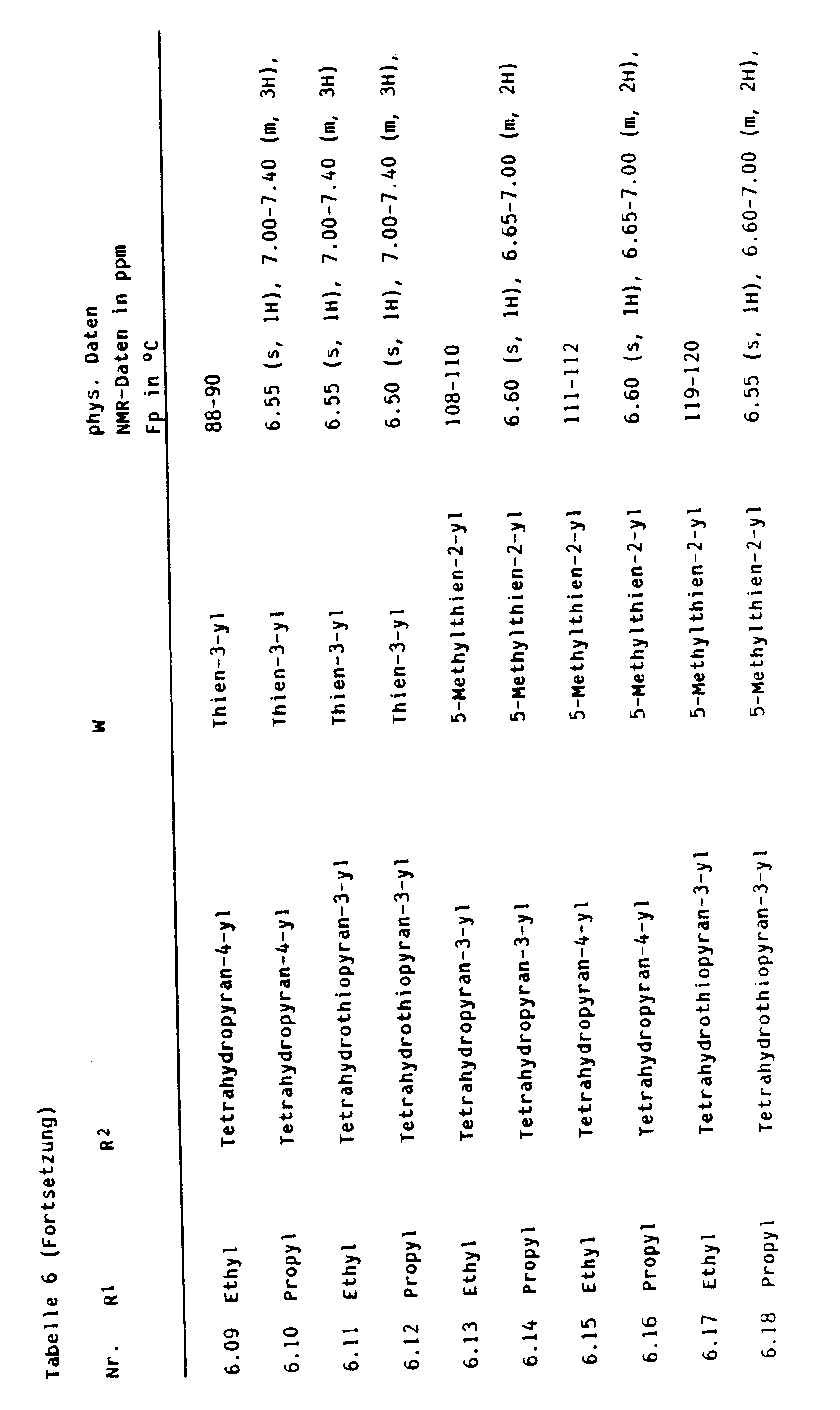

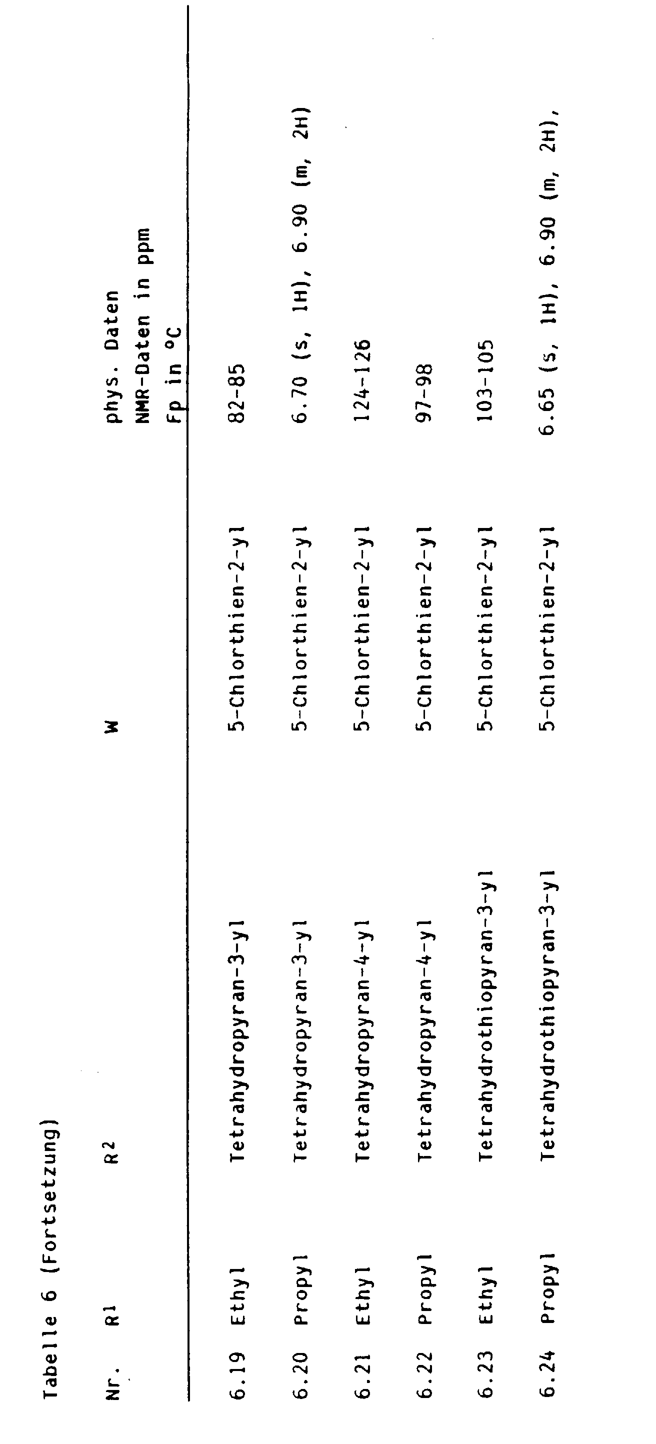

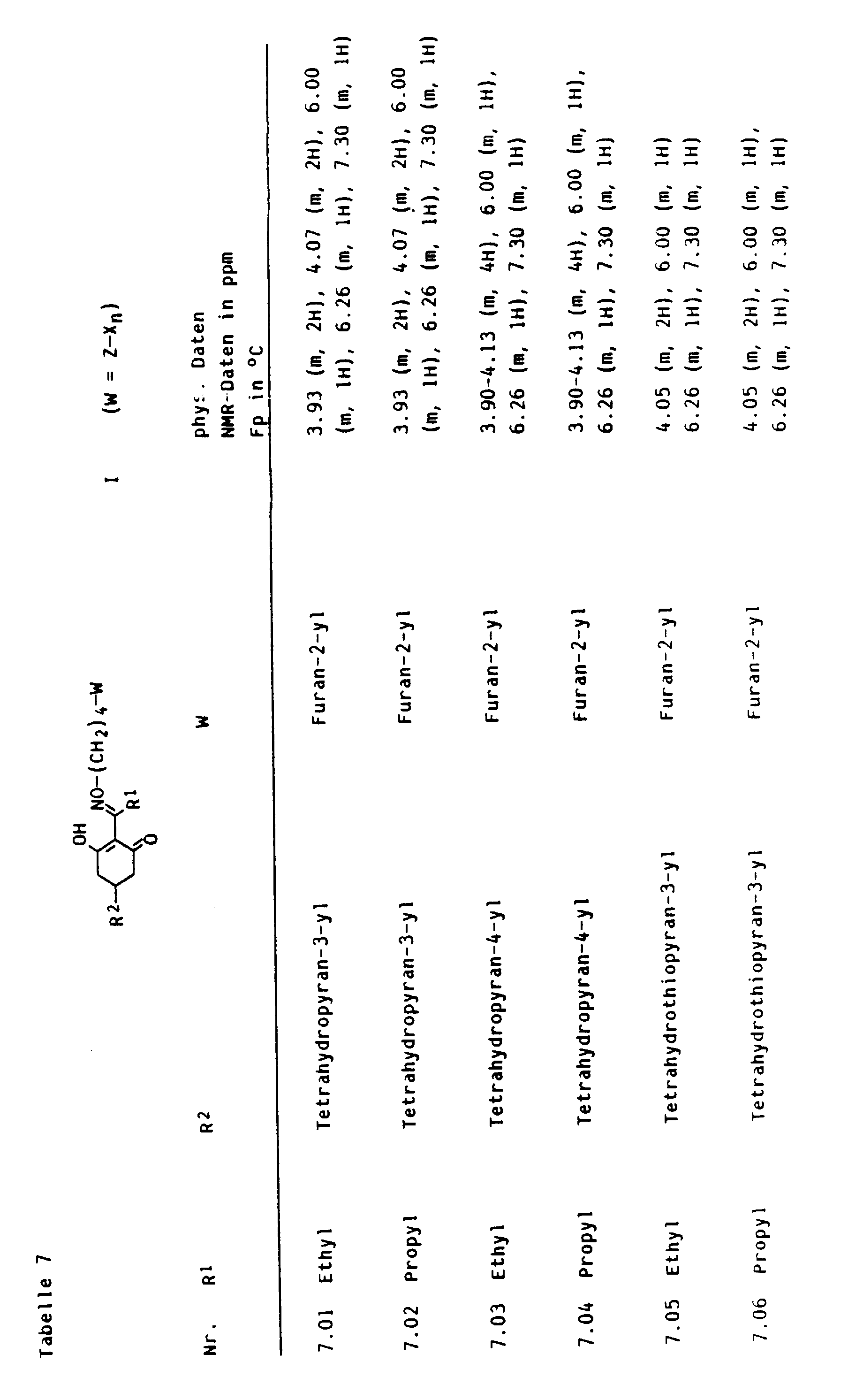

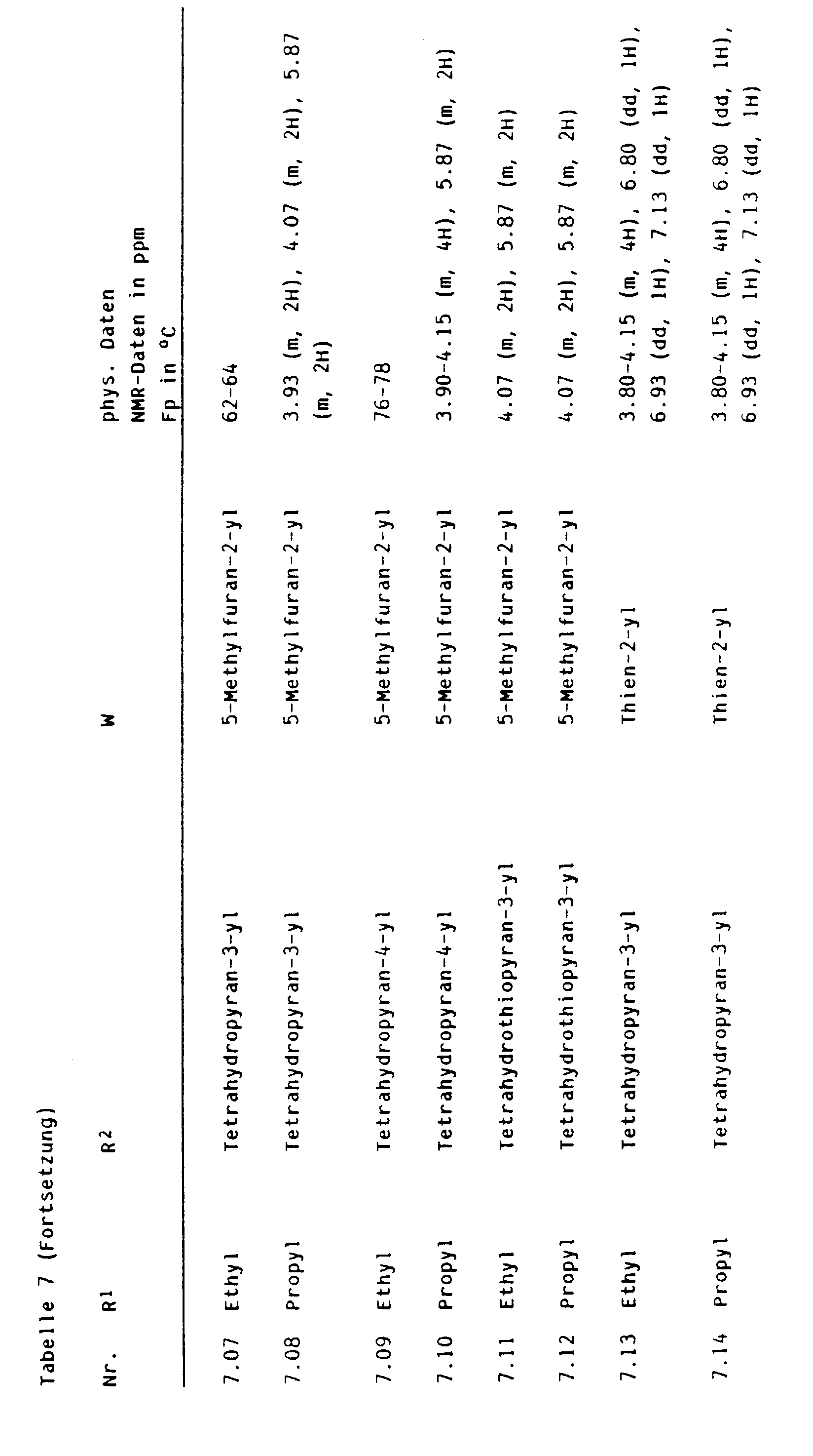

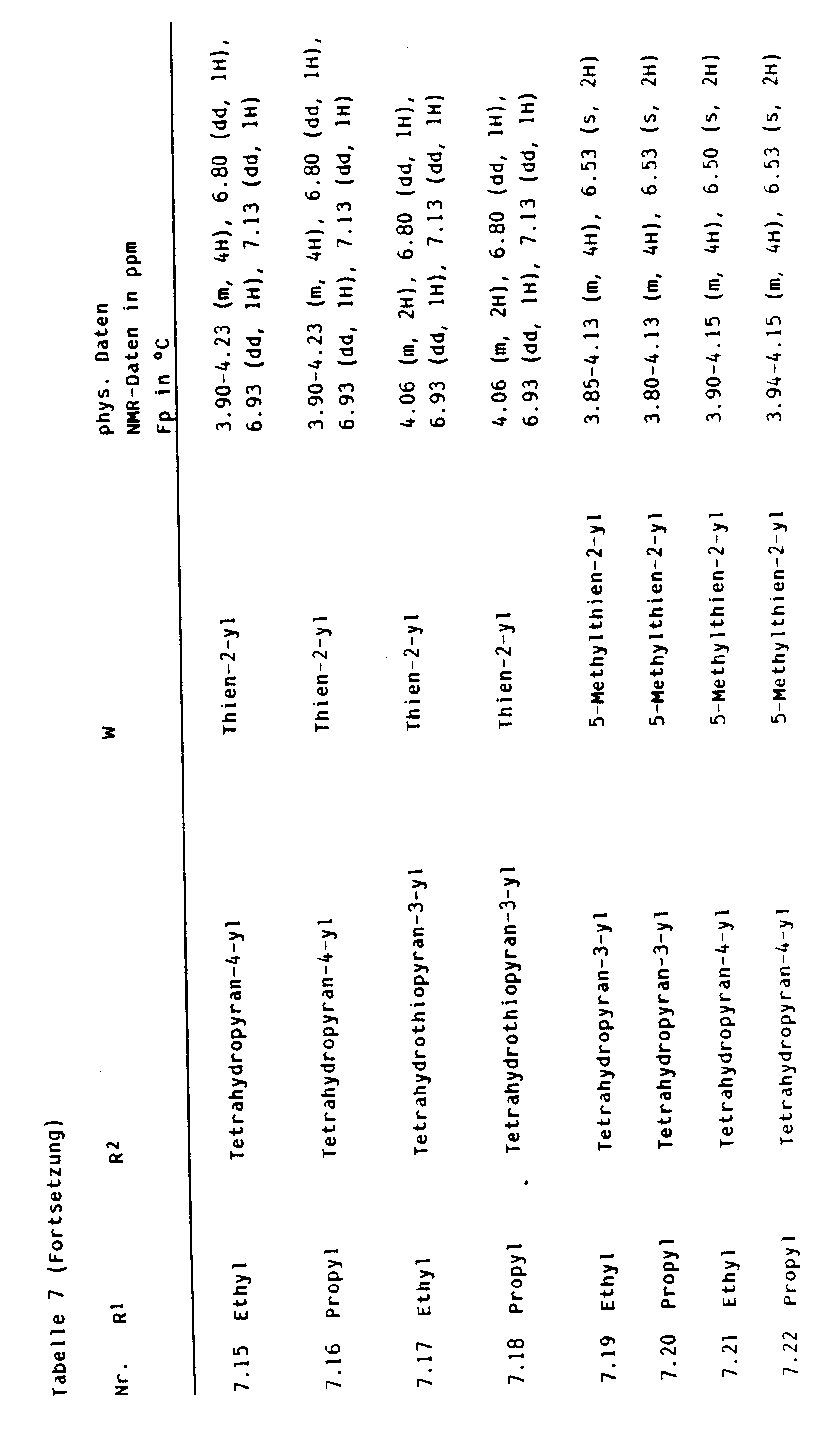

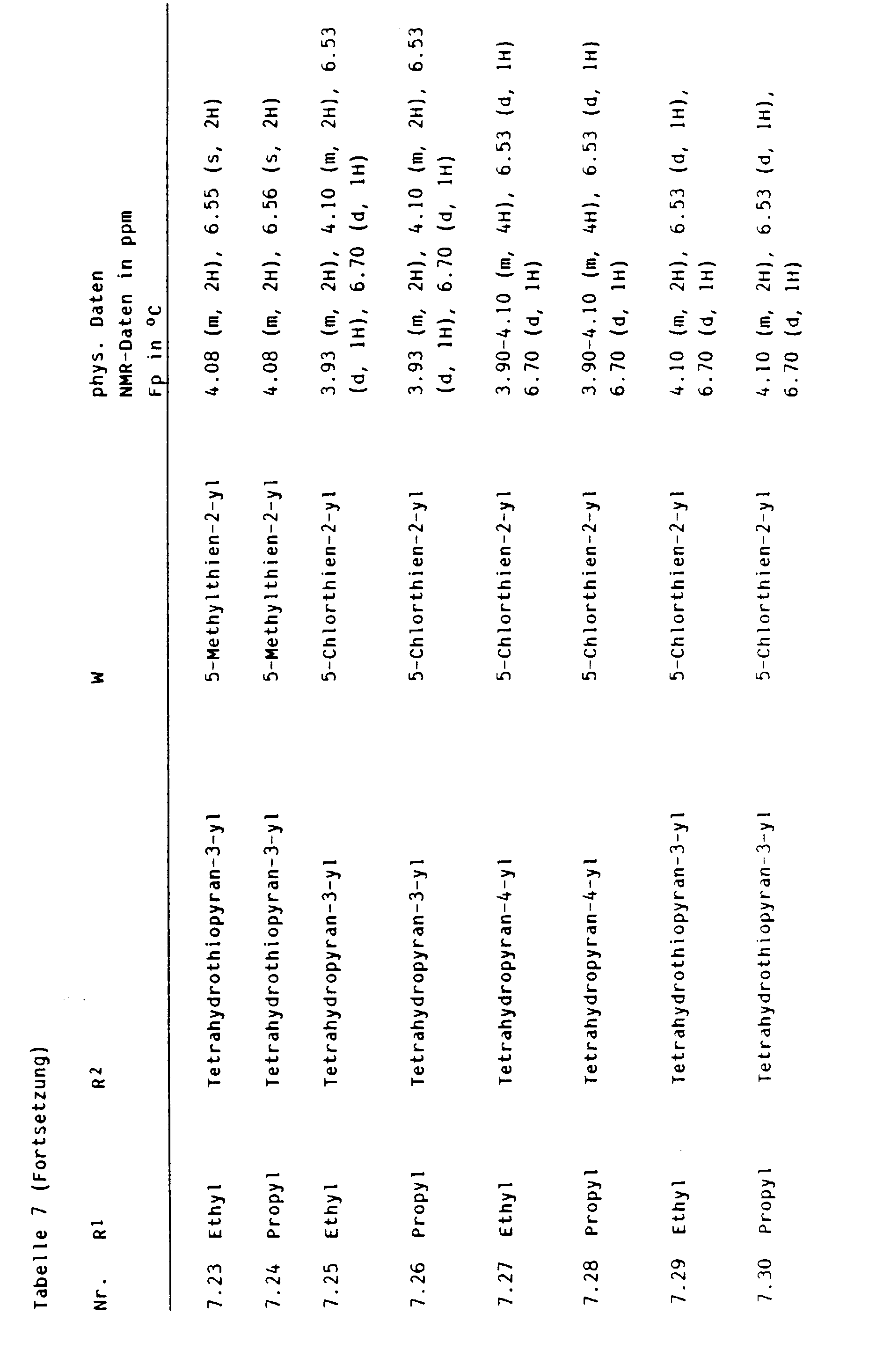

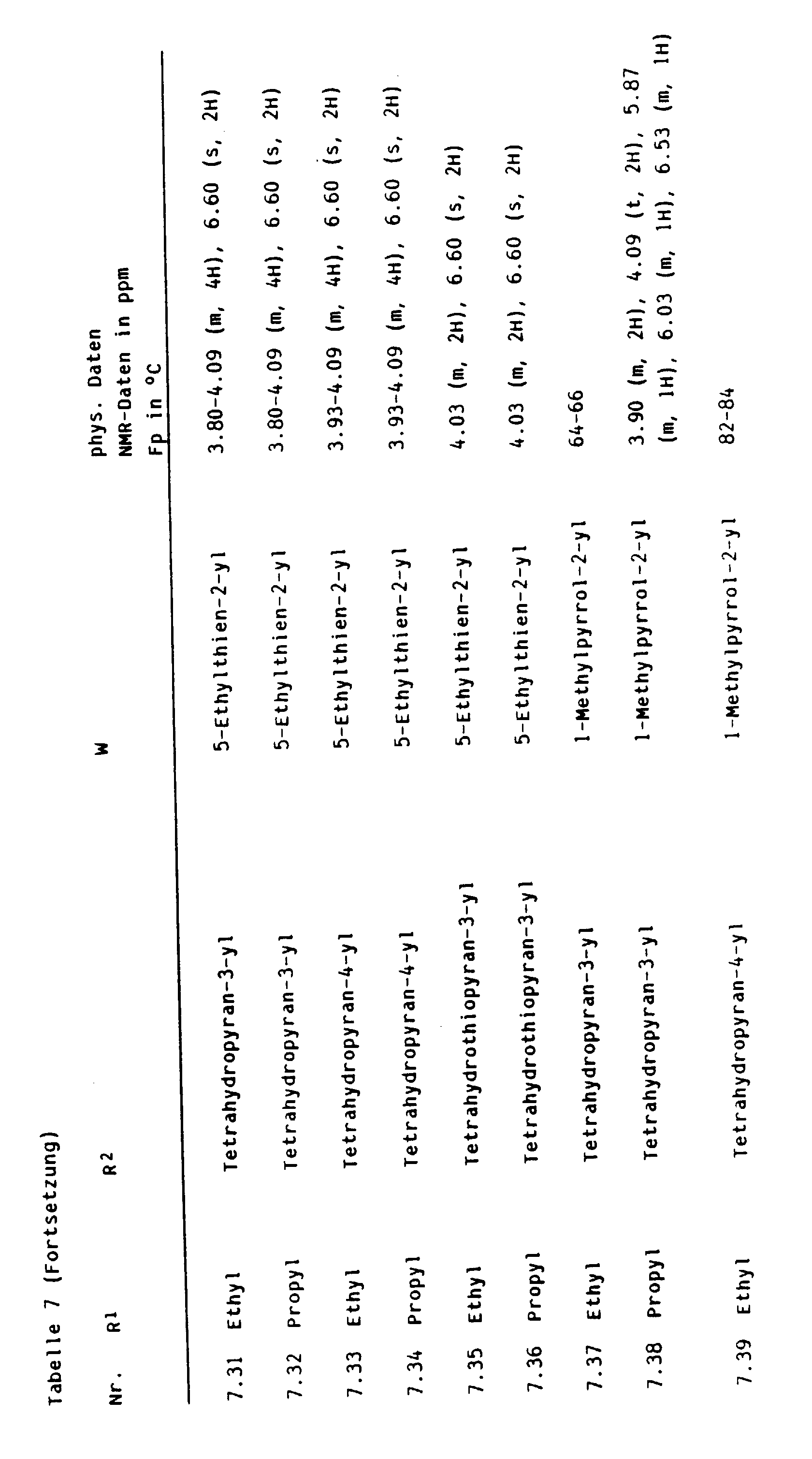

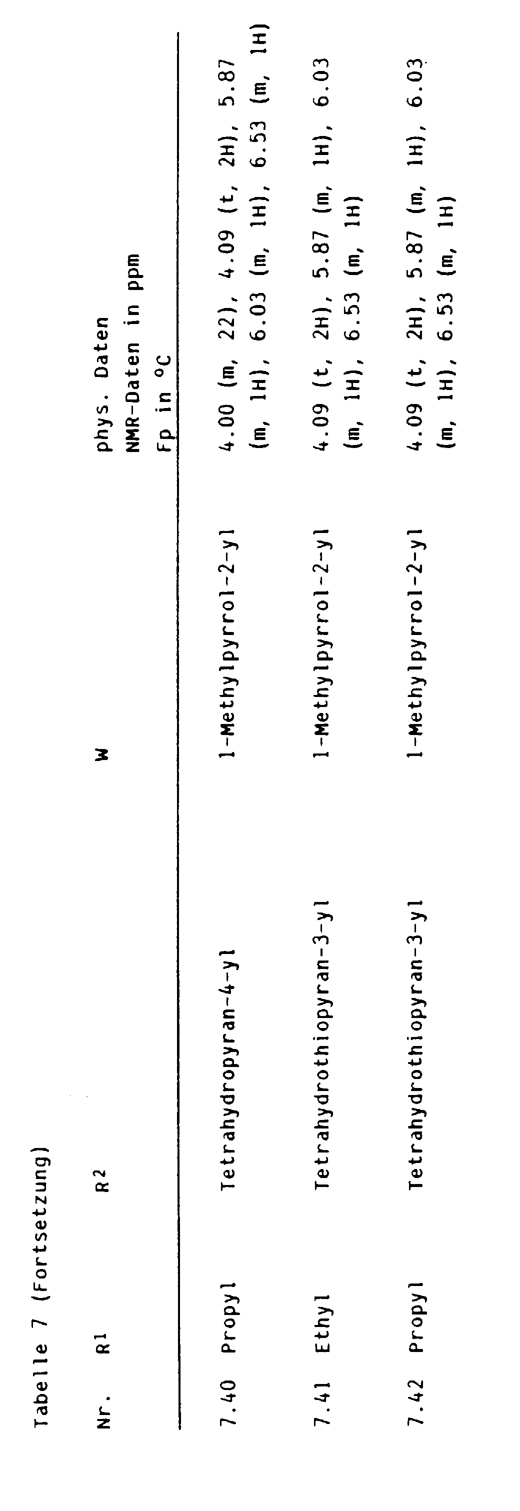

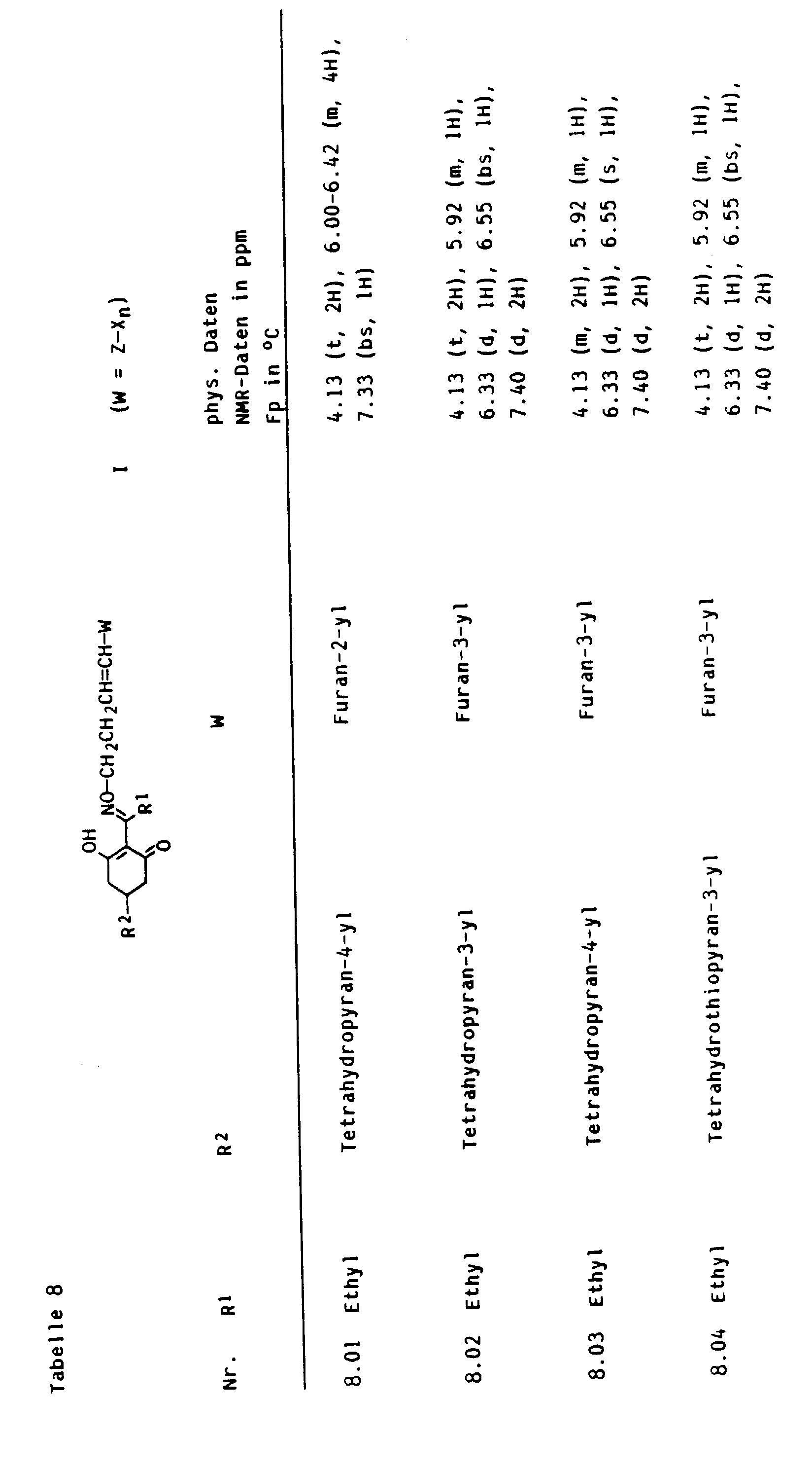

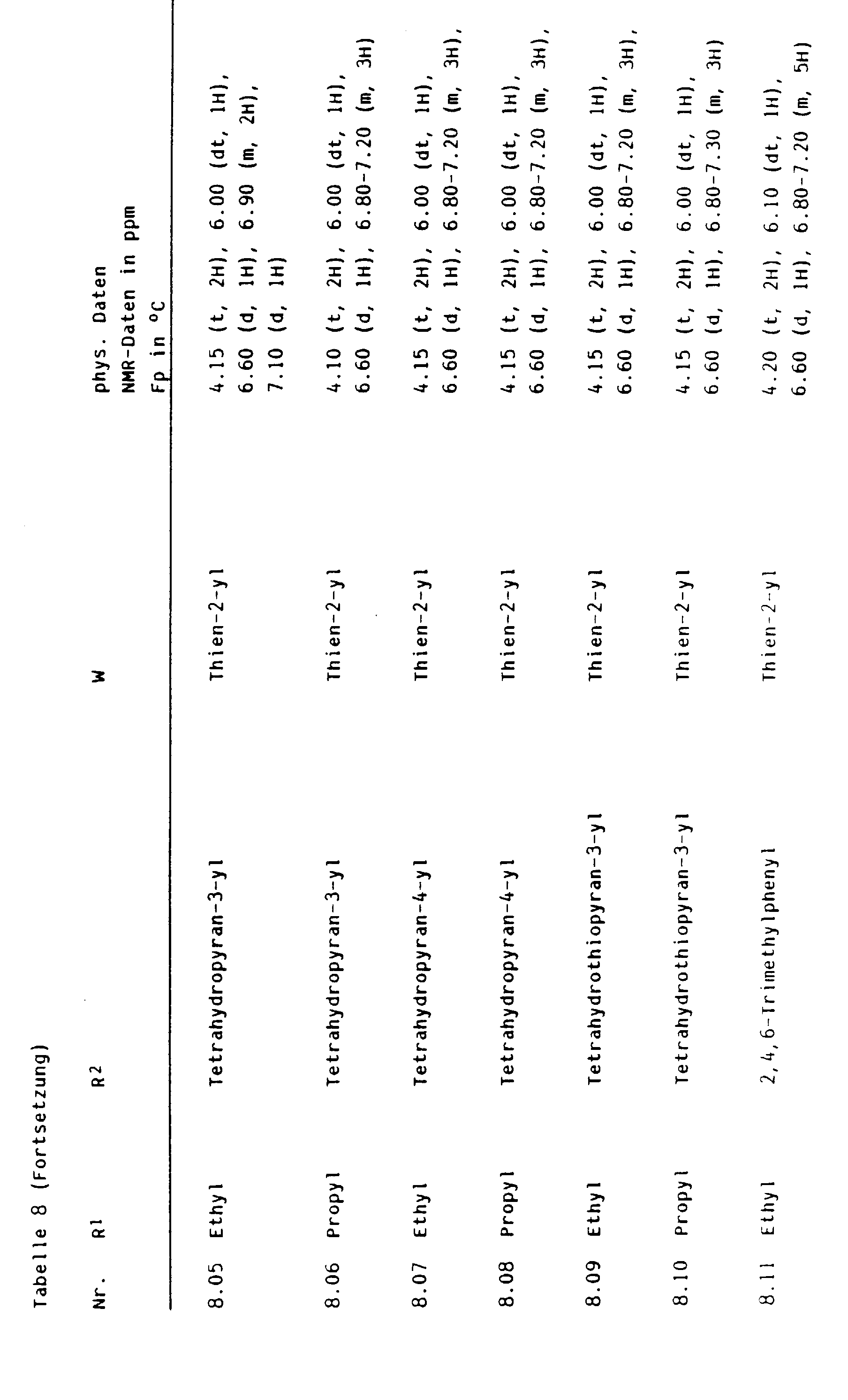

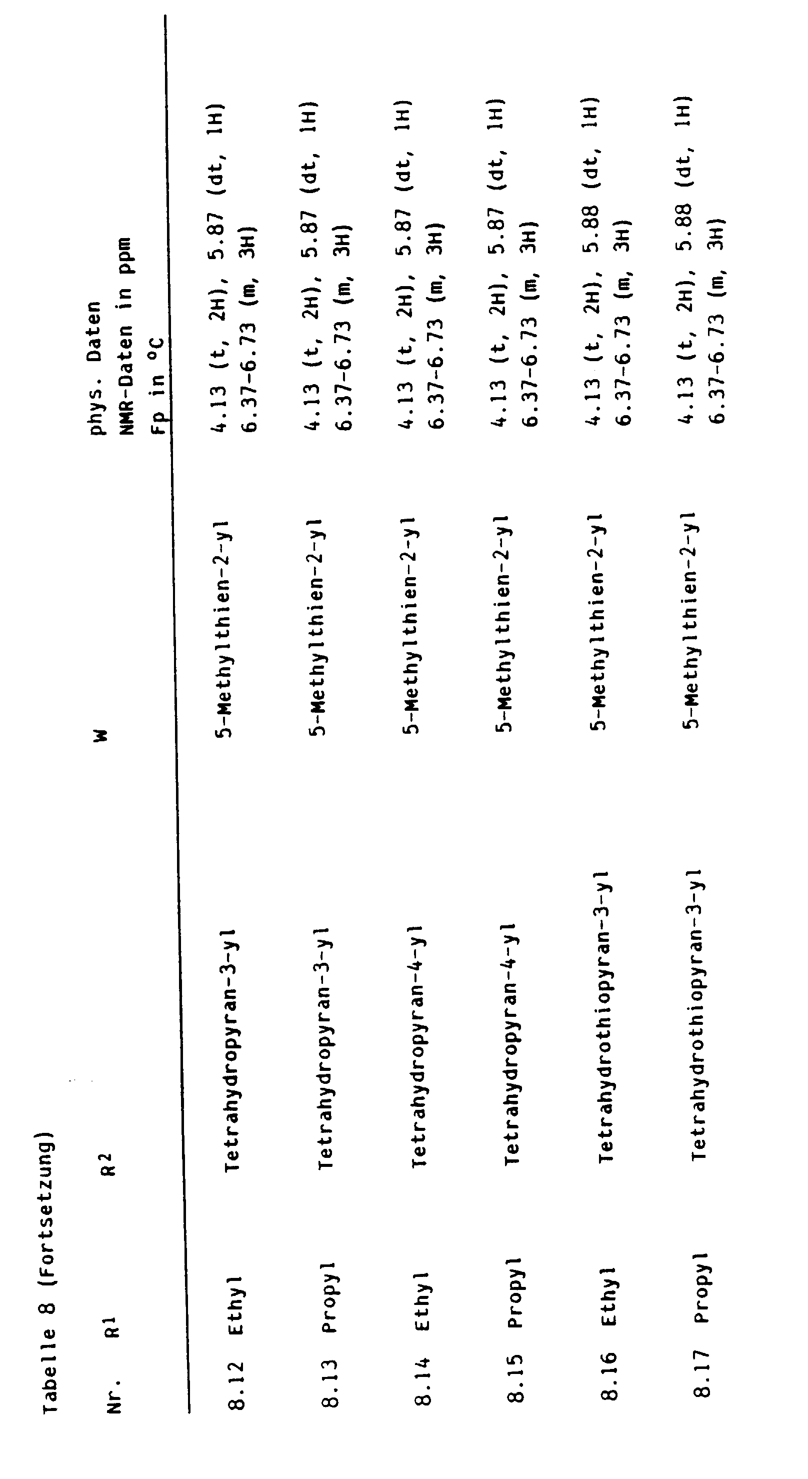

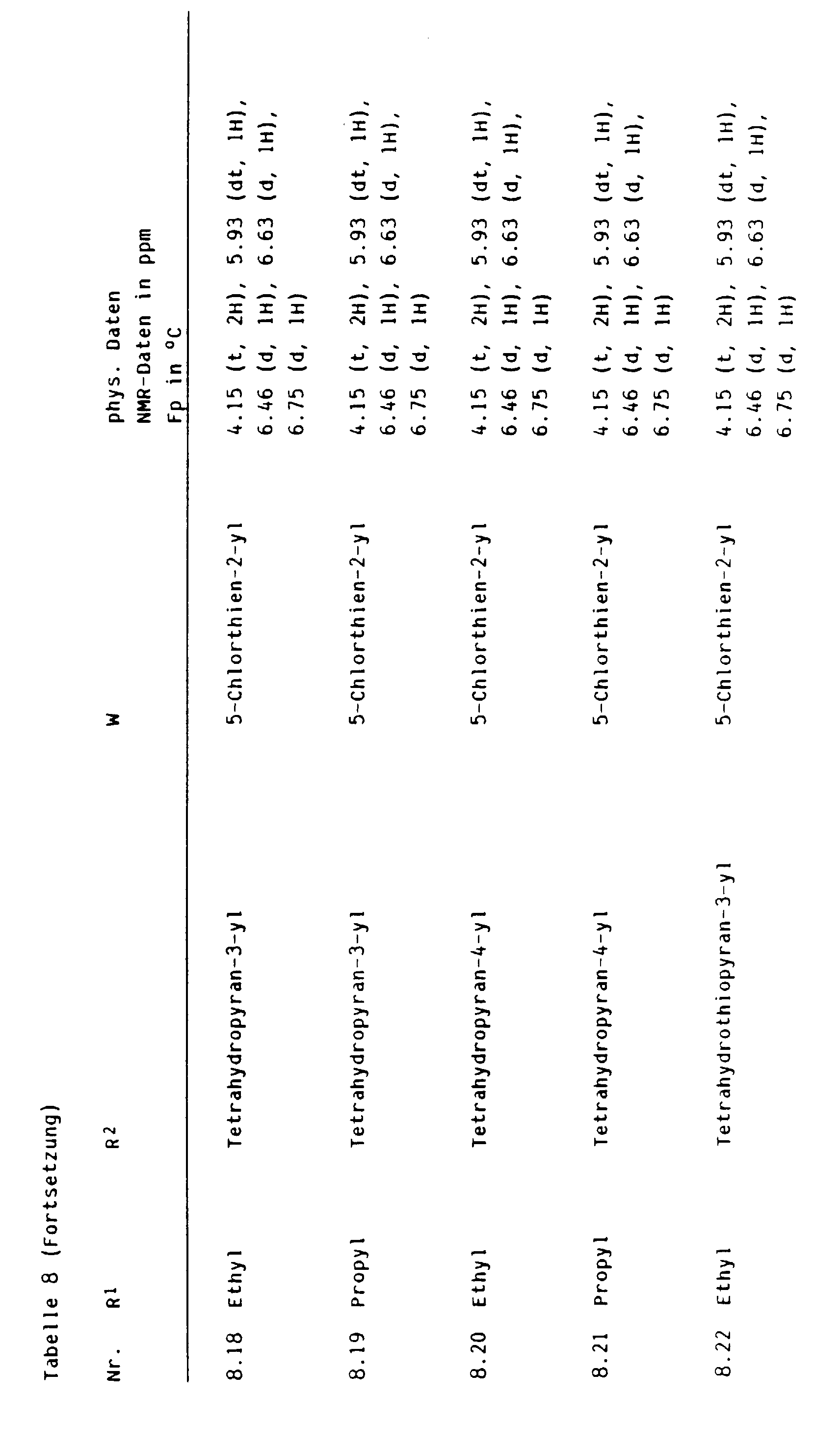

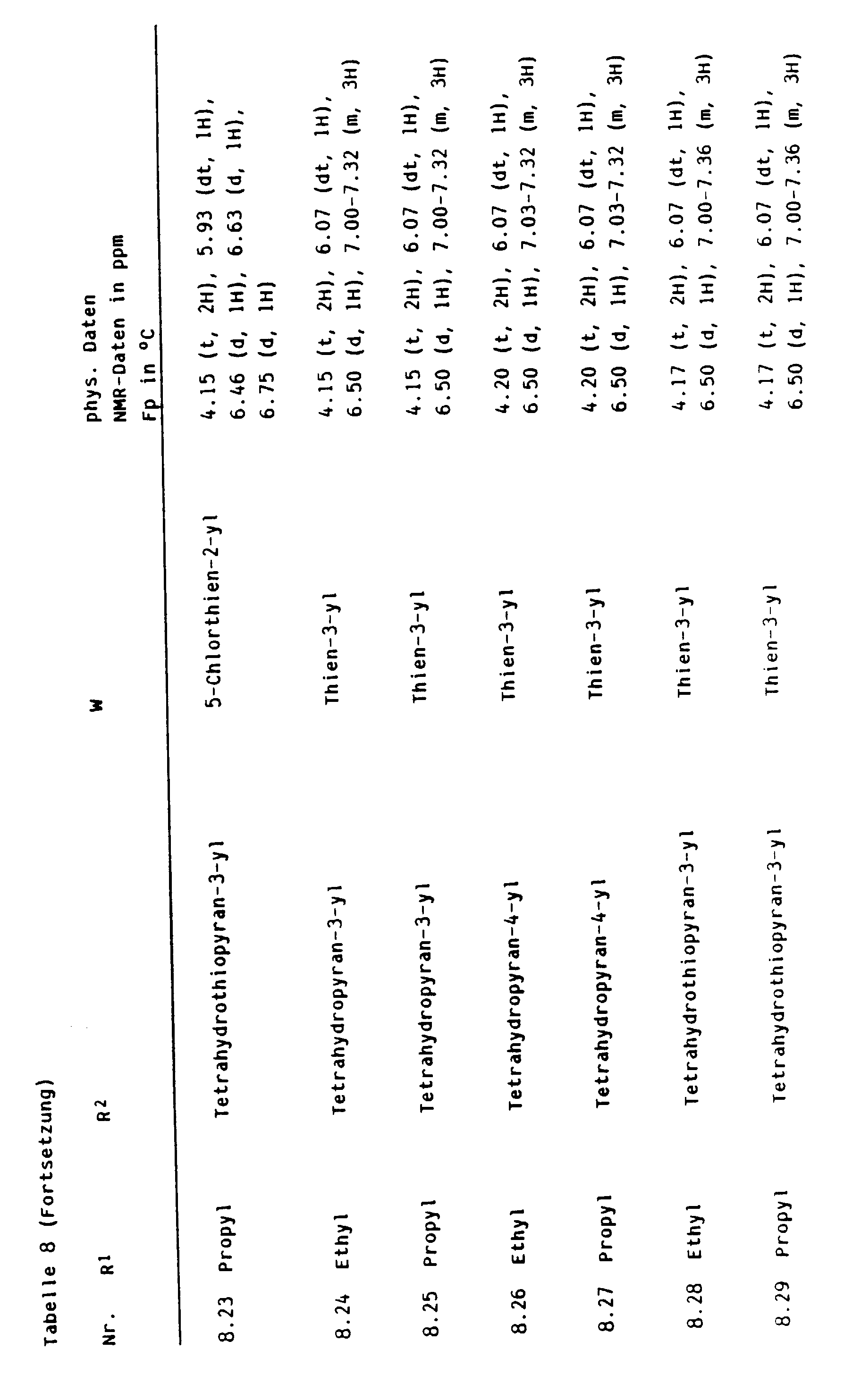

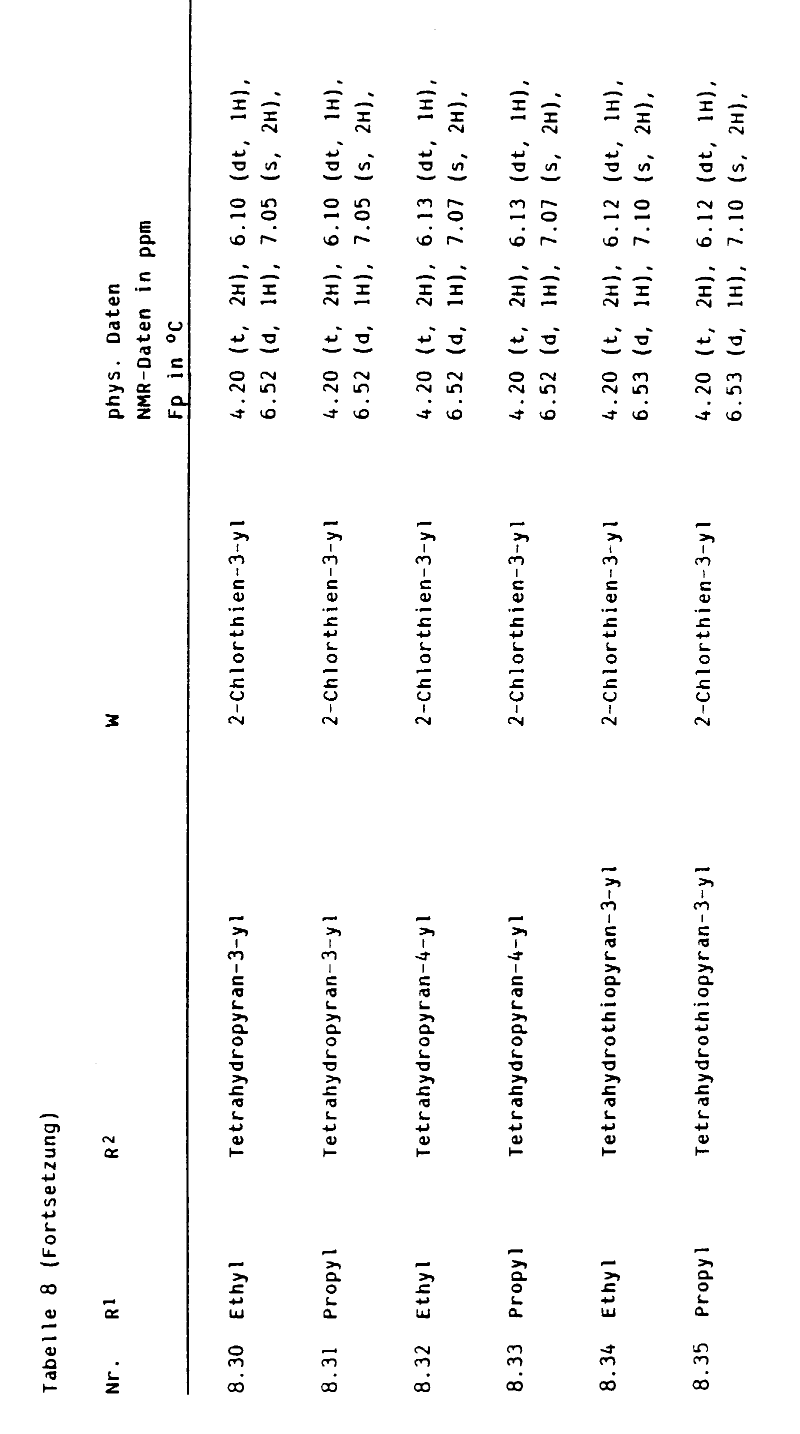

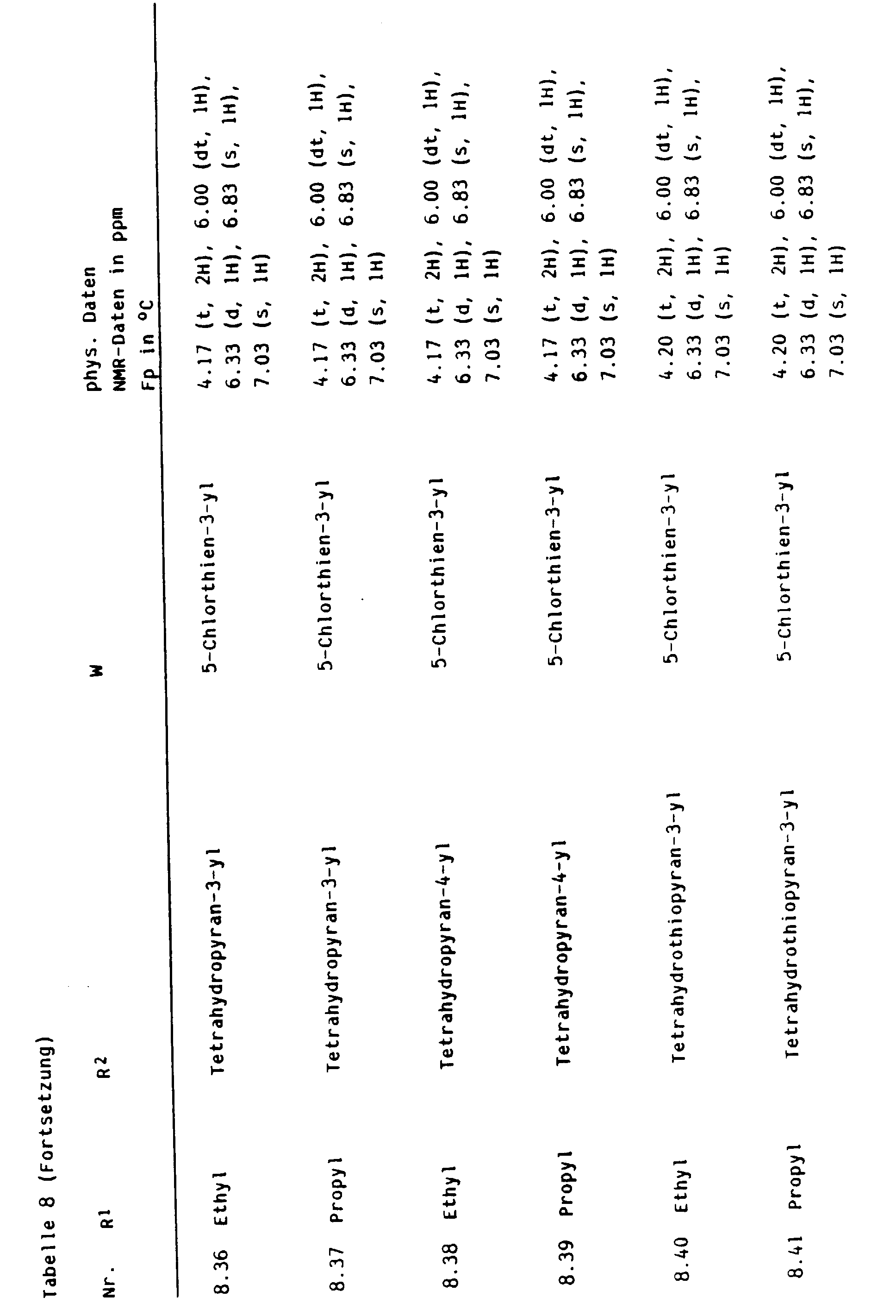

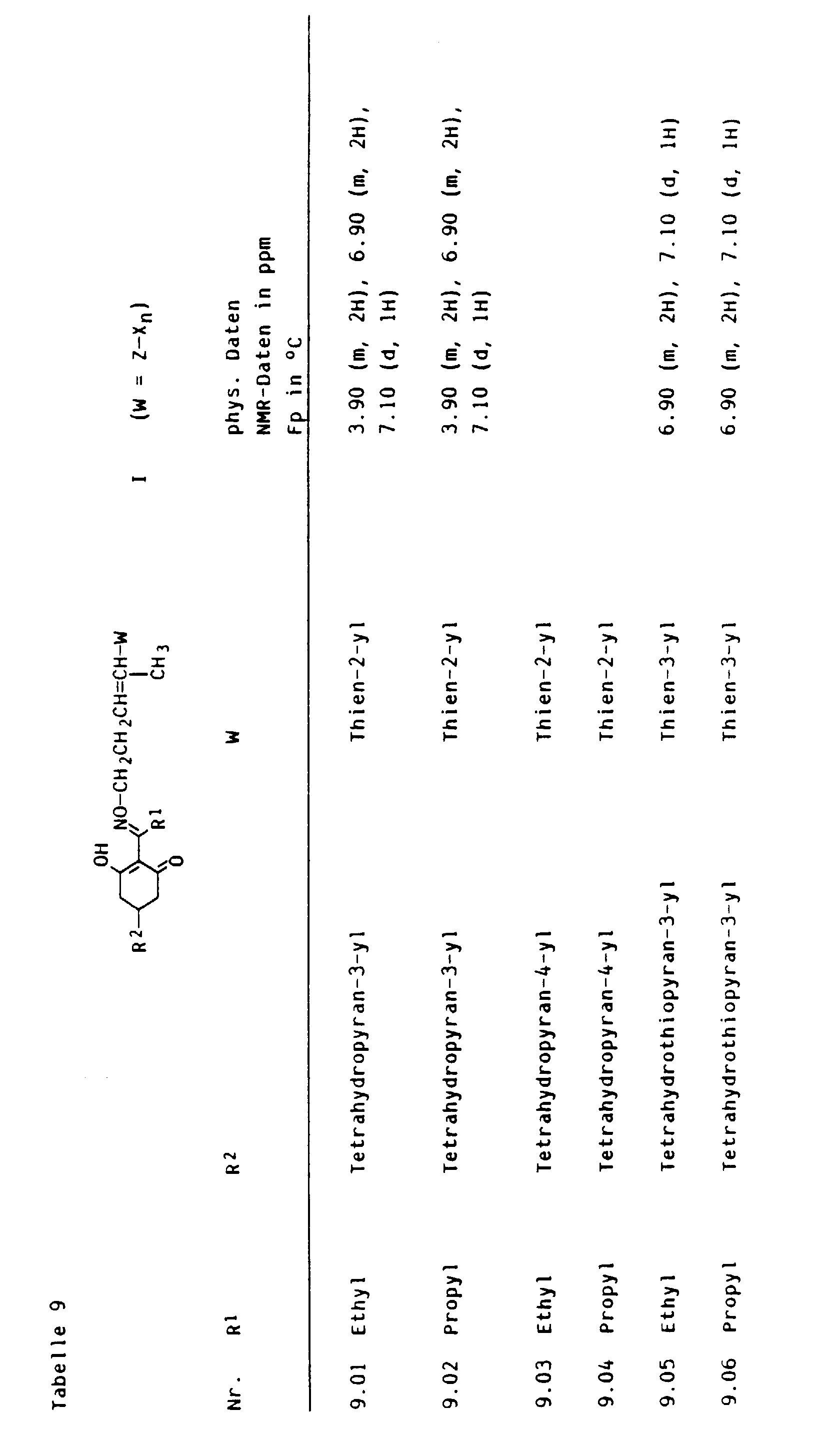

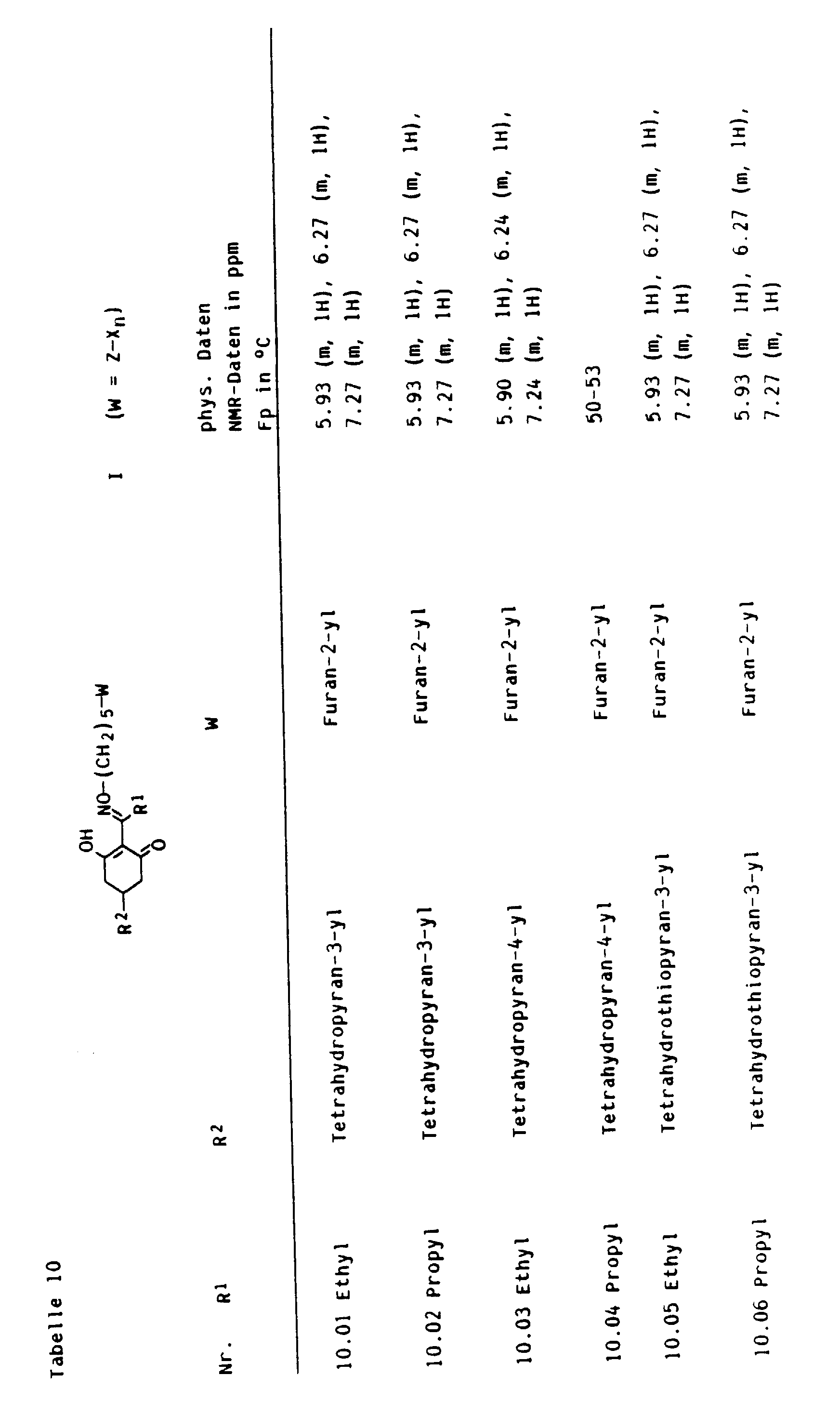

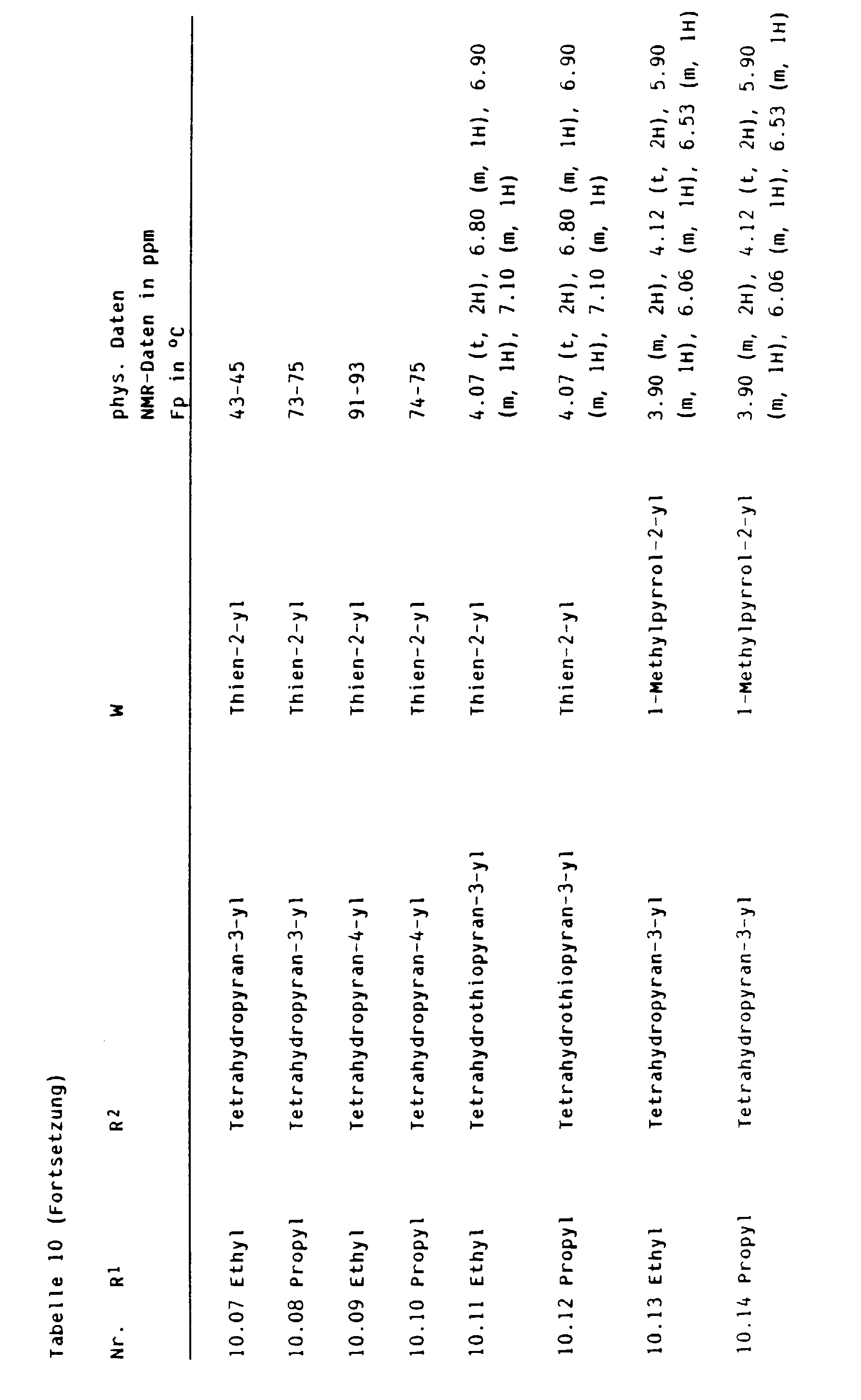

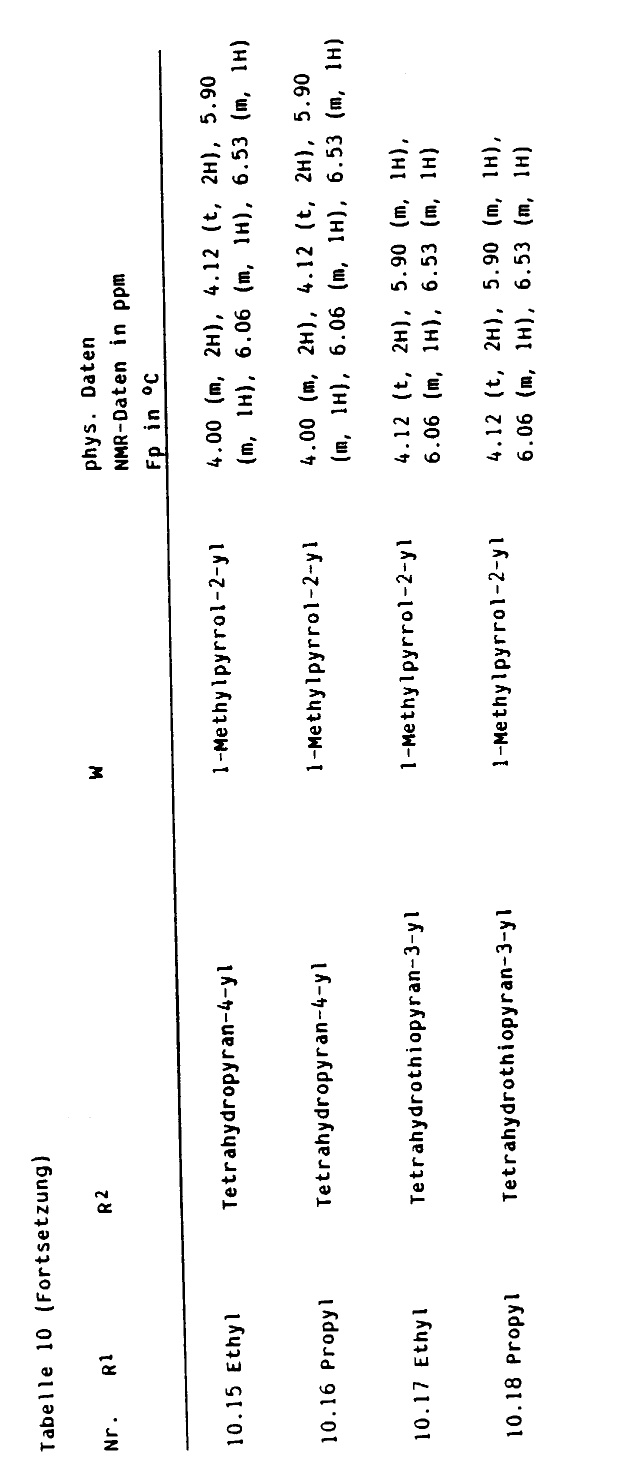

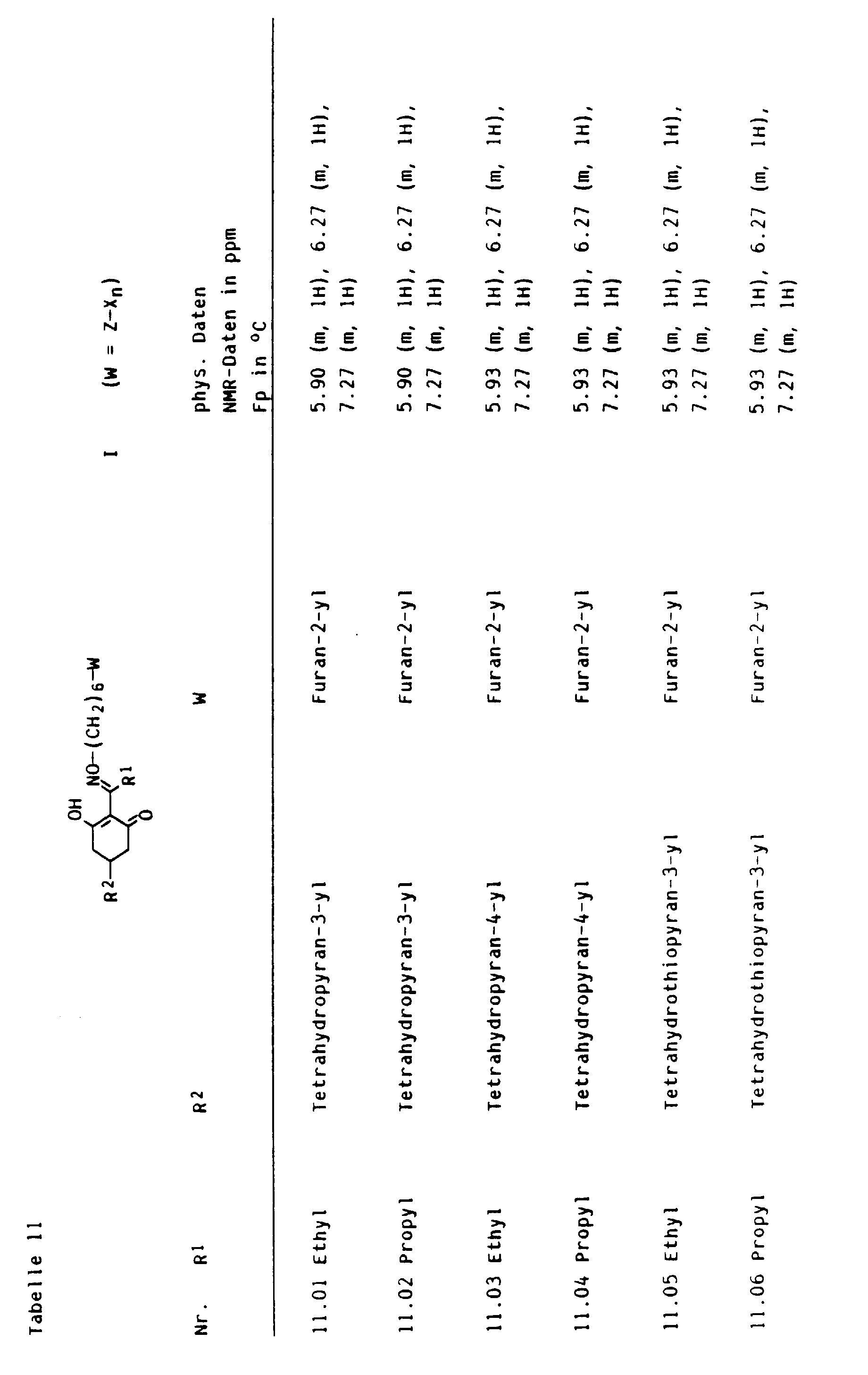

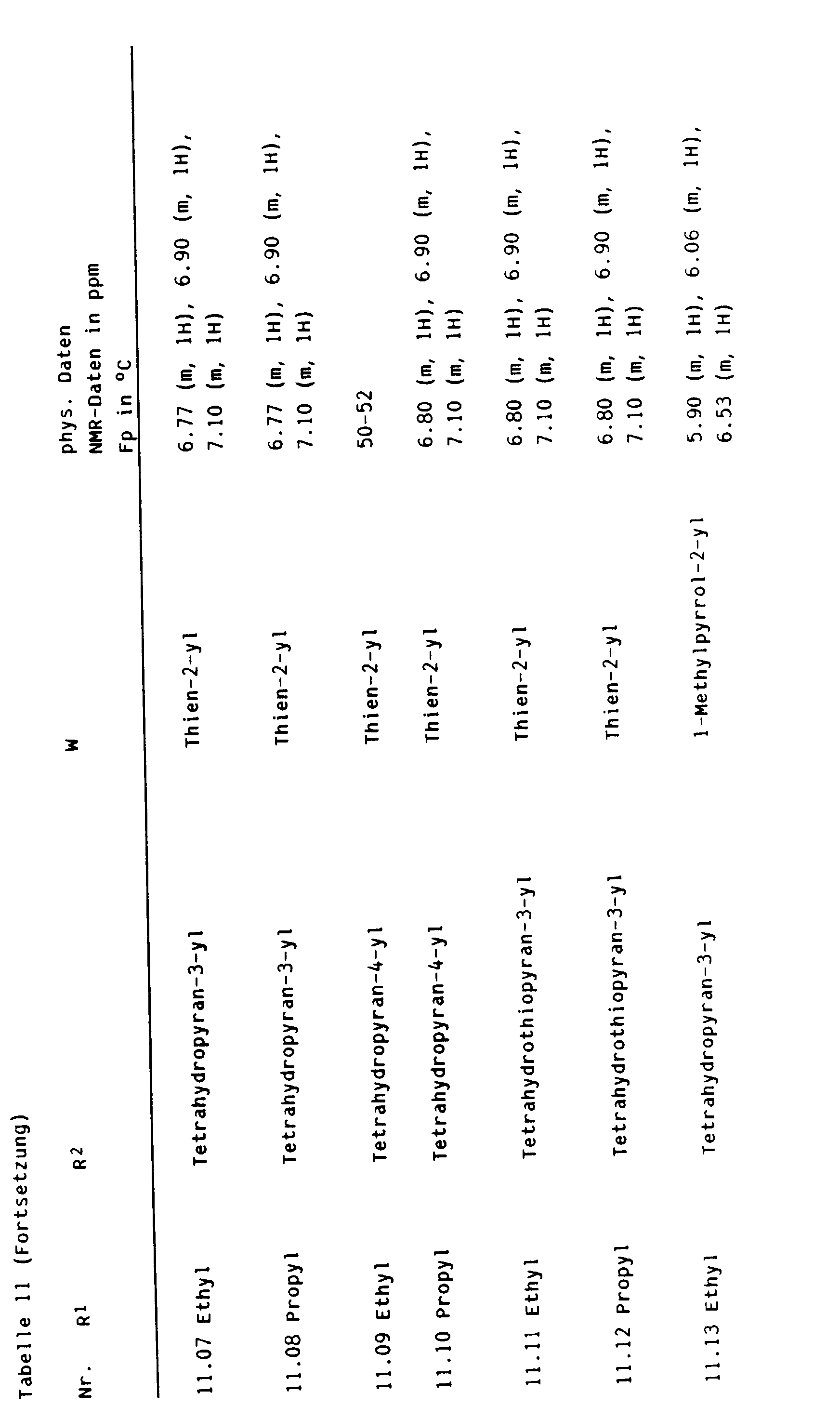

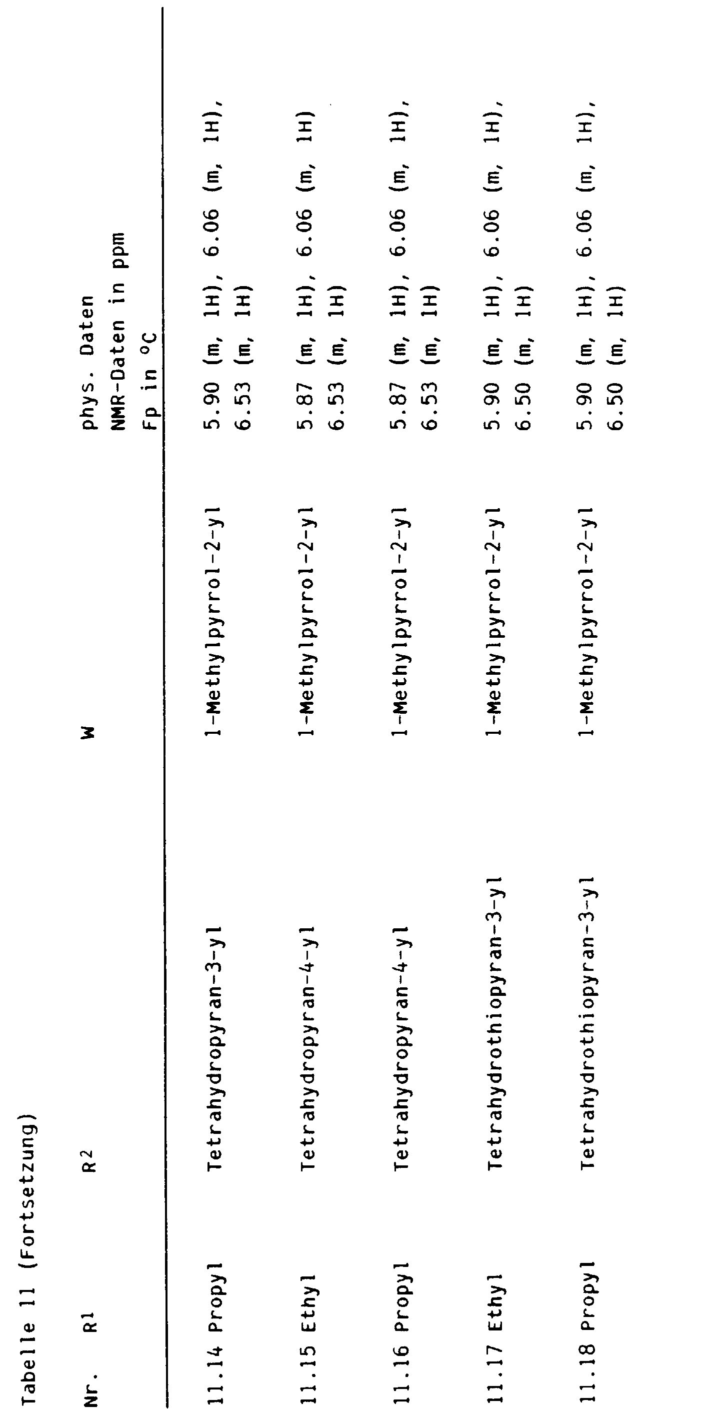

### Anwendungsbeispiele

Die Wirkung der Cyclohexenonderivate der Formel I auf das Pflanzenwachstum läßt sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Oryza sativa | Reis | rice |
| Setaria viridis | Grüne Borstenhirse | green foxtail |

Mit 0,25 kg/ha a.S. im Nachauflaufverfahren eingesetzt, lassen sich mit dem Beispiel 8.08 unerwünschte grasartige Pflanzen sehr gut bekämpfen, bei gleichzeitiger Verträglichkeit an der Beispielkultur Reis.

## Patentansprüche

1. Cyclohexenonoximether der allgemeinen Formel I 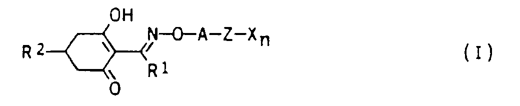 in der die Substituenten folgende Bedeutung haben:
R¹ eine C₁-C₆-Alkylgruppe;
A C₂-C₆-Alkylen oder C₃-C₆-Alkenylen, wobei diese Gruppen ein bis drei C₁-C₃-Alkylgruppen, Halogenatome tragen können;
Z ein 5-gliedriger Heteroaromat mit ein bis drei Heteroatomen, ausgewählt aus einer Gruppe bestehend aus drei Stickstoffatomen und einem Sauerstoff- oder Schwefelatom,
ein 6-gliedriger Heteroaromat mit ein bis vier Stickstoffatomen;
X eine Aminogruppe -NR^{a}R^{b}, worin
R^{a} Wasserstoff, eine C₁-C₄-Alkylgruppe, eine C₃-C₅-Alkenylgruppe oder eine C₃-C₆-Alkinylgruppe und
R^{b} Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei der folgenden Substituenten tragen kann: Nitro, Cyano, Halogen, C₁-C₄-Alkyl, C₁-C₄-Alkoxy, C₁-C₄-Alkylthio und C₁-C₄-Halogenalkyl bedeuten, oder
Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkoxy, Carboxyl, C₁-C₄-Alkoxycarbonyl, Benzyloxycarbonyl, Phenyl, wobei die aromatischen Reste noch einen bis drei der folgenden Substituenten tragen können: Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl, Benzyloxycarbonyl;
n 0 bis 3 oder 1 bis 4 für den Fall, dap Z einen halogensubstituierten Pyridylrest bedeutet;
R² eine C₁-C₄-Alkoxy-C₁-C₆-alkyl- oder C₁-C₄-Alkylthio-C₁-C₆-alkylgruppe;
eine C₃-C₇-Cycloalkylgruppe oder eine C₅-C₇-Cycloalkenylgruppe, wobei diese Gruppen noch einen bis drei Reste tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus, der ein oder zwei Heteroatome enthält, ausgewählt aus einer Gruppe bestehend aus Sauerstoff und Schwefel und wobei der Heterocyclus noch einen bis drei Reste tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein 6- oder 7-gliedriger gesättigter oder ein- oder zweifach ungesättigter Heterocyclus, enthaltend ein oder zwei Heteroatome, ausgewählt aus einer Gruppe bestehend aus Sauerstoff und Schwefel, wobei dieser Ring noch einen bis drei Reste tragen kann, ausgewählt aus einer Gruppe bestehend aus Hydroxy, Halogen, C₁-C₄-Alkyl, C₁-C₄-Alkoxy, C₁-C₄-Alkylthio und C₁-C₄-Halogenalkyl;
die Phenylgruppe oder die Pyridylgruppe, wobei diese Gruppen noch einen bis drei Reste tragen können, ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy, C₁-C₄-Alkoxy-C₁-C₄-alkyl und -NR^{a}R^{b}, worin R^{a} und R^{b} die oben genannte Bedeutung haben,
bedeuten, sowie ihre landwirtschaftlich nutzbaren Salze und die Ester von I mit C₁-C₁₀-Carbonsäuren oder anorganischen Säuren.

2. Verfahren zur Herstellung von Verbindungen der Formel I gemäß Anspruch 1, dadurch gekennzeichnet, daß man ein entsprechendes Cyclohexenon der Formel II 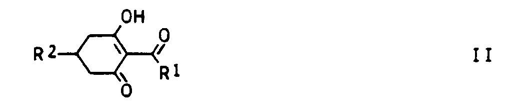 in an sich bekannter Weise in einem inerten organischen Lösungsmittel mit einem Hydroxylamin der Formel III
H₂N-O-A-Z-Xₙ III
oder einem Salz des entsprechenden Hydroxylamins umsetzt, und die Verfahrensprodukte I gewünschtenfalls in ihre landwirtschaftlich nutzbaren Salze oder in die Ester von C₁-C₁₀-Carbonsäuren oder organischen Säuren überführt.

3. Herbizid, enthaltend inerte Zusatzstoffe und eine herbizid wirksame Menge mindestens einer Verbindung der Formel I gemäß Anspruch 1.

4. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenon-derivates der Formel I gemäß Anspruch 1 behandelt.

5. Hydroxylamine der Formel III
H₂N-O-A-Z-Xₙ III,
in der A, Z und Xₙ die in Anspruch 1 genannte Bedeutung haben.

6. Verfahren zur Herstellung von Hydroxylaminen der Formel III, gemäß Anspruch 5, dadurch gekennzeichnet, daß man eine Verbindung der Formel IV
Y-A-Z-Xₙ IV,
in der A, Z und Xₙ die in Anspruch 1 genannte Bedeutung haben und Y für eine nukleophil verdrängbare Abgangsgruppe steht, mit einem cyclischen Hydroxyimid der Formel V 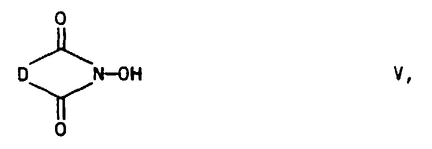 in der D für C₂- und C₃-Alkylen, C₂-Alkenylen oder einen Fünf- oder Sechsring steht, der 1 bis 3 Doppelbindungen und ein Stickstoffatom enthalten kann, in Gegenwart einer Base zu Verbindungen der Formel VI 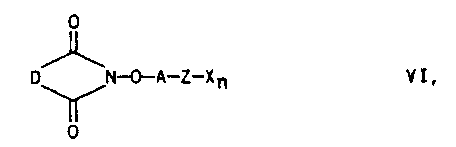 umsetzt und aus den Imidethern VI die Hydroxylamine III mittels einer Base oder Säure freisetzt.

7. Verfahren nach Anspruch 6, dadurch gekennzeichnet, daß man das Hydroxylamin III aus dem Imidether VI mittels Ethanolamin freisetzt.

## Claims

1. A cyclohexenone oxime ether of the formula I 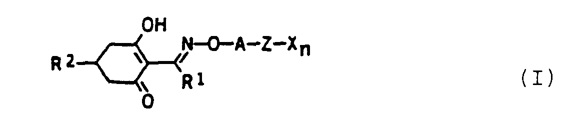 where
R¹ is C₁-C₆-alkyl;
A is C₂-C₆-alkylene or C₃-C₆-alkenylene, where these groups may carry from one to three C₁-C₃-alkyl groups or halogen atoms;
Z is a 5-membered heteroaromatic structure having from one to three hetero atoms selected from the group consisting of three nitrogen atoms and one oxygen or sulfur atom or a 6-membered heteroaromatic structure having from one to four nitrogen atoms;
X is an amino group -NR^{a}R^{b}, where
R^{a} is hydrogen, C₁-C₄-alkyl, C₃-C₆-alkenyl or C₃-C₆-alkynyl and
R^{b} is hydrogen, C₁-C₄-alkyl, C₃-C₆-alkenyl, C₃-C₆-alkynyl, C₁-C₆-acyl or benzoyl, where the aromatic ring may also carry from one to three of the following substituents: nitro, cyano, halogen, C₁-C₄-alkyl, C₁-C₄-alkoxy, C₁-C₄-alkylthio and C₁-C₄-haloalkyl,
nitro, cyano, halogen, C₁-C₄-alkyl, C₁-C₄-alkoxy, C₁-C₄-alkylthio, C₁-C₄-haloalkyl, C₁-C₄-haloalkoxy, carboxyl, C₁-C₄-alkoxycarbonyl, benzyloxycarbonyl or phenyl, where the aromatic radicals may also carry from one to three of the following substituents: nitro, cyano, halogen, C₁-C₄-alkyl, C₁-C₄-alkoxy, C₁-C₄-alkylthio, C₁-C₄-haloalkyl, C₁-C₄-haloalkoxy, carboxyl, C₁-C₄-alkoxycarbonyl and benzyloxycarbonyl,
n is from 0 to 3, or from 1 to 4 where Z is halogen-substituted pyridyl, and
R² is C₁-C₄-alkoxy-C₁-C₆-alkyl or C₁-C₄-alkylthio-C₁-C₆-alkyl;
C₃-C₇-cycloalkyl or C₅-C₇-cycloalkenyl, where these groups may also carry from one to three radicals selected from the group consisting of C₁-C₄-alkyl, C₁-C₄-alkoxy, C₁-C₄-alkylthio, C₁-C₄-haloalkyl, hydroxyl and halogen,
a 5-membered saturated heterocyclic structure which contains one or two hetero atoms selected from the group consisting of oxygen and sulfur and where the heterocyclic structure may also carry from one to three radicals selected from the group consisting of C₁-C₄-alkyl, C₁-C₄-alkoxy, C₁-C₄-alkylthio and C₁-C₄-haloalkyl,
a 6-membered or 7-membered saturated or mono- or diunsaturated heterocyclic structure containing one or two hetero atoms selected from the group consisting of oxygen and sulfur, where this ring may also carry from one to three radicals selected from the group consisting of hydroxyl, halogen, C₁-C₄-alkyl, C₁-C₄-alkoxy, C₁-C₄-alkylthio and C₁-C₄-haloalkyl, or phenyl or pyridyl, where these groups may also carry from one to three radicals selected from the group consisting of halogen, nitro, cyano, C₁-C₄-alkyl, C₁-C₄-alkoxy, C₁-C₄-alkylthio, C₁-C₄-haloalkyl, C₃-C₆-alkenyloxy, C₃-C₆-alkynyloxy, C₁-C₄-alkoxy-C₁-C₄-alkyl and -NR^{a}R^{b}, where R^{a} and R^{b} have the abovementioned meanings,
and its agriculturally useful salts and the esters of I with C₁-C₁₀-carboxylic acids or inorganic acids.

2. A process for the preparation of a compound of the formula I as claimed in claim 1, wherein a corresponding cyclohexenone of the formula II 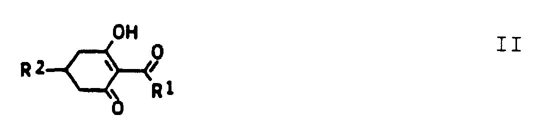 is reacted in a conventional manner, in an inert organic solvent, with a hydroxylamine of the formula III
H₂N-O-A-Z-Xₙ III
or a salt of the corresponding hydroxylamine, and, if desired, the products I are converted into their agriculturally useful salts or into the esters of C₁-C₁₀-carboxylic acids or organic acids.

3. A herbicide containing inert additives and a herbicidal amount of at least one compound of the formula I as claimed in claim 1.

4. A method for controlling undesirable plant growth, wherein the undesirable plants and/or their habitat is or are treated with a herbicidal amount of a cyclohexenone derivative of the formula I as claimed in claim 1.

5. A hydroxylamine of the formula III
H₂N-O-A-Z-Xₙ III
where A, Z and Xₙ have the meanings stated in claim 1.

6. A process for the preparation of a hydroxylamine of the formula III as claimed in claim 5, wherein a compound of the formula IV
Y-A-Z-Xₙ IV
where A, Z and Xₙ have the meanings stated in claim 1 and Y is a nucleophilically displaceable leaving group, is reacted with a cyclic hydroximide of the formula V 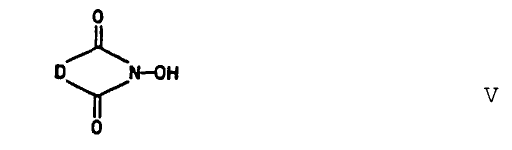 where D is C₂- or C₃-alkylene, C₂-alkenylene or a five-membered or six-membered ring which may contain from 1 to 3 double bonds and one nitrogen atom, in the presence of a base to give a compound of the formula VI 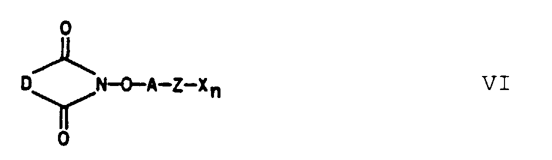 and the hydroxylamine III is liberated from the imido ether VI by means of a base or acid.

7. A process as claimed in claim 6, wherein the hydroxylamine III is liberated from the imido ether VI by means of ethanolamine.

## Revendications

1. Ethers de cyclohexénonoximes de formule générale I 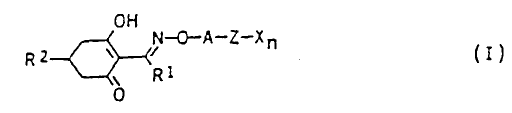 dans laquelle les symboles ont les significations suivantes :
R¹ : un groupe alkyle en C1-C6 ;
A : un groupe alkylène en C2-C6 ou alcénylène en C3-C6 qui peut porter un à trois groupes alkyle en C1-C3 ou atomes d'halogènes ;
Z : un radical hétéroaromatique à cinq chaînons contenant un à trois hétéroatomes choisis dans un groupe consistant en trois atomes de soufre et un atome d'oxygène ou de soufre,
un radical hétéroaromatique à six chaînons contenant un à quatre atomes d'azote ;
X : un groupe amino-NR^{a}R^{b} dans lequel
R^{a} représente l'hydrogène, un groupe alkyle en C1-C4, alcényle en C3-C6 ou alcynyle en C3-C6 et
R^{b} représente l'hydrogène, un groupe alkyle en C1-C4, alcényle en C3-C6, alcynyle en C3-C6, acyle en C1-C6 ou benzoyle dont le noyau aromatique peut encore porter un à trois des substituants suivants : nitro, cyano, halogéno, alkyle en C1-C4, alcoxy en C1-C4, alkylthio en C1-C4 ou halogénoalkyle en C1-C4, ou bien
un groupe nitro, cyano, un halogène, un groupe alkyle en C1-C4, alcoxy en C1-C4, alkylthio en C1-C4, halogénoalkyle en C1-C4, halogénoalcoxy en C1-C4, carboxyle, (alcoxy en C1-C4)carbonyle, benzyloxycarbonyle, phényle, les radicaux aromatiques pouvant encore porter un à trois des substituants suivants : nitro, cyano, halogéno, alkyle en C1-C4, alcoxy en C1-C4, alkylthio en C1-C4, halogénoalkyle en C1-C4, halogénoalcoxy en C1-C4, carboxyle, (alcoxy en C1-C4)carbonyle, benzyloxycarbonyle ;
n : 0 à 3, ou 1 à 4 lorsque Z représente un radical pyridyle à substituant halogéno ;
R² : un groupe (alcoxy en C1-C4)alkyle en C1-C6 ou (alkylthio en C1-C4)alkyle en C1-C6 ;
un groupe cycloalkyle en C3-C7 ou cycloalcényle en C5-C7 qui peut encore porter un à trois substituants choisis parmi les groupes alkyle en C1-C4, alcoxy en C1-C4, alkylthio en C1-C4, halogénoalkyle en C1-C4, hydroxy et les halogènes ;
un hétérocycle saturé à cinq chaînons contenant un ou deux hétéroatomes choisis dans un groupe consistant en l'oxygène et le soufre, l'hétérocycle pouvant encore porter un à trois substituants choisis parmi les groupes alkyle en C1-C4, alcoxy en C1-C4, alkylthio en C1-C4 et halogénoalkyle en C1-C4 ;
un hétérocycle saturé ou mono- ou di-insaturé de six ou sept chaînons contenant un ou deux hétéroatomes choisis parmi un groupe consistant en l'oxygène et le soufre, ce cycle pouvant encore porter un à trois substituants choisis parmi les groupes hydroxy, les halogènes, les groupes alkyle en C1-C4, alcoxy en C1-C4, alkylthio en C1-C4 et halogénoalkyle en C1-C4 ;
le groupe phényle ou le groupe pyridyle, qui peuvent encore porter un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6, (alcoxy en C1-C4)alkyle en C1-C4 et -NR^{a}R^{b} dans lequel R^{a} et R^{b} ont les significations indiquées ci-dessus,
et leurs sels utilisables en agriculture et les esters de I et d'acides carboxyliques en C1-C10 ou d'acides minéraux.

2. Procédé de préparation des composés de formule I de la revendication 1, caractérisé par le fait que l'on fait réagir une cyclohexénone correspondante de formule II 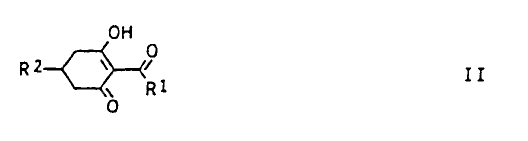 de manière connue en soi, dans un solvant inerte, avec une hydroxylamine de formule III
H₂N-O-A-Z-Xₙ III
ou l'un de ses sels et, si on le désire, on convertit les produits I ainsi obtenus en leurs sels utilisables dans l'agriculture ou en les esters d'acides carboxyliques en C1-C10 ou d'acides minéraux.

3. Produit herbicide contenant des additifs inertes et une quantité herbicide efficace d'au moins un composé de formule I de la revendication 1.

4. Procédé pour combattre les croissances de végétaux indésirables, caractérisé par le fait que l'on traite les végétaux indésirables et/ou leur habitat par une quantité herbicide efficace d'un dérivé de cyclohexénone de formule I de la revendication 1.

5. Hydroxylamines de formule III
H₂N-O-A-Z-Xₙ III
dans laquelle A, Z et Xₙ ont les significations indiquées dans la revendication 1.

6. Procédé pour la préparation des hydroxylamines de formule III de la revendication 5, caractérisé par le fait que l'on fait réagir un-composé de formule IV
Y-A-Z-Xₙ IV
dans laquelle A, Z et Xₙ ont les significations indiquées dans la revendication 1 et Y représente un groupe nucléophile éliminable, avec un hydroxyimide cyclique de formule V 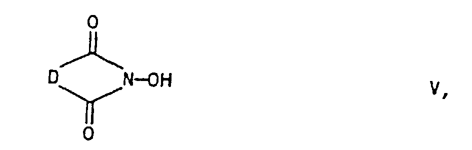 dans laquelle D représente un groupe alkylène en C2-C3, alcénylidène en C2 ou un cycle à cinq chaînons qui peut contenir une à trois doubles liaisons et un atome d'azote, en présence d'une base, ce qui donne des composés de formule VI 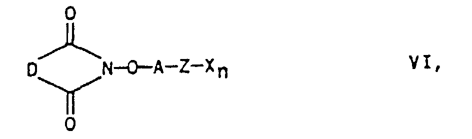 c'est-à-dire des éthers d'imides VI, à partir desquels on libère, à l'aide d'une base ou d'un acide, les hydroxylamines III.

7. Procédé selon la revendication 6, caractérisé par le fait que l'on libère l'hydroxylamine III à partir de l'éther d'imide VI à l'aide de l'éthanolamine.
